(19) Europäisches Patentamt
European Patent Office
Office européen des brevets

(11) Publication number: **0 242 167 B1**

# (12) EUROPEAN PATENT SPECIFICATION

(45) Date of publication of patent specification: **27.11.91**

(51) Int. Cl.5: **C07D 209/22**, C07D 403/06, C07D 413/06, C07D 231/56, C07D 209/12, A61K 31/405

(21) Application number: **87303221.3**

(22) Date of filing: **13.04.87**

## (54) Heterocyclic carboxamides.

(30) Priority: **15.04.86 GB 8609175**
**15.10.86 GB 8624698**

(43) Date of publication of application:
**21.10.87 Bulletin 87/43**

(45) Publication of the grant of the patent:
**27.11.91 Bulletin 91/48**

(84) Designated Contracting States:
**AT BE CH DE ES FR GB GR IT LI LU NL SE**

(56) References cited:
**EP-A- 0 107 618**
**EP-A- 0 166 591**
**EP-A- 0 179 619**
**DE-A- 738 727**
**FR-M- 4 035**
**FR-M- 7 631**
**US-A- 3 271 416**
**US-A- 3 470 298**

(73) Proprietor: **ICI AMERICAS INC.**
**Concord Pike & New Murphy Road**
**Wilmington Delaware 19897(US)**

(72) Inventor: **Brown, Frederick Jeffrey**
**112 Great Circle Road**
**Newark, DE 19711(US)**
Inventor: **Yee, Ying Kwong**
**98 Quail Run**
**Kennett Square, PA 19348(US)**

(74) Representative: **Smith, Stephen Collyer et al**
**Imperial Chemical Industries PLC Legal Department: Patents P.O. Box 6 Bessemer Road**
**Welwyn Garden City Herts, AL7 1HD(GB)**

**EP 0 242 167 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

SUMMARY AND BACKGROUND OF THE INVENTION

This invention concerns novel heterocyclic carboxamide derivatives and, more particularly, novel benozic acids (and related tetrazoles and acylsulphonamides), derived from indolecarboxamides, indazolecarboxamides and indolinecarboxamides which antagonise the pharmacological actions of one or more of the arachidonic acid metabolites known as leukotrienes (hereinafter referred to as "leukotriene antagonist properties"). The novel derivatives are useful whenever such antagonism is desired. Thus, such compounds may be of value in the treatment of those diseases in which leukotrienes are implicated, for example in the treatment of allergic disorders, such as, for example, asthma, or of inflammatory diseases, or of endotoxic or traumatic shock conditions. The invention also provides pharmaceutical compositions containing the novel derivatives for use in such treatments, and processes and intermediates for the manufacture of the novel derivatives.

In European Patent Application publication number 0 107 618 A1 are disclosed N-substituted-2-(1-imidazoylyl)-indoles which possess thromboxane synthetase inhibitor properties. We have now discovered a series of indole, indazole and indoline derivatives which have a carboxamidic substituent in the benzenoid ring and a particularly substituted alkyl group in the 3-position and which unexpectedly possess the property of antagonising one or more of the arachidonic acid metabolites known as leukotrienes and this is the basis for our invention.

DESCRIPTION OF THE INVENTION

According to the invention there is provided a compound of formula I

(Formula set out on pages following Examples) I wherein the group -Y-Z< is selected from a group consisting of:

(a) -C(Ra) = C<

(b) -N = C<

(c) -CH(Ra)-CH<

in which "<" indicates two separate bonds;

Ra is hydrogen or (1-4C)alkyl;

Rb is hydrogen;

$R^1$ is (2-10C)alkyl optionally containing 1 or more fluorine substituents; or $R^1$ is phenyl-(1-6C)-alkyl in which the (1-6C)alkyl moiety may optionally bear a fluoro or (1-4C)alkoxy substituent and in which the phenyl moiety may optionally bear a substituent selected from a group consisting of halogeno, (1-4C)alkyl, (1-4C)alkoxy and trifluoromethyl; or $R^1$ is (3-8C)cycloalkyl or (3-8C)cycloalkyl (1-6C)alkyl, the cyclic moiety of any of which optionally may contain one unsaturated linkage and may optionally bear 1 or 2 (1-4C)alkyl substituents;

L is a (1-10C)alkylene link, optionally containing one double or triple bond;

P is a polar group selected from a group consisting of cyano, 1H-tetrazol-5-yl, carbamoyl of formula $CONR^2R^3$, ureido of formula $NR^4CONR^2R^3$, carbamoyloxy of formula $OCONR^2R^3$, a carbamate of formula $NR^4COOR^5$, and an (optionally oxidized) thio group of formula $S(O)_nR^5$ in which

$R^2$ is selected from a group consisting of hydrogen, (1-6C)alkyl, and phenyl, the phenyl moiety of which may optionally bear 1 or 2 substituents selected from a group consisting of halogeno, (1-4C)alkyl, (1-4C)-alkoxy and trifluoromethyl, and

$R^3$ and $R^4$ are independently selected from a group consisting of hydrogen and (1-6C)alkyl;

or $R^2$ and $R^3$ together with the adjacent nitrogen form a pyrrolidine, piperidine, morpholine, piperazine or N-(1-6C)alkylpiperazine ring, and $R^4$ is hydrogen or (1-6C)alkyl;

$R^5$ is chosen from a group consisting of (1-4C)alkyl and phenyl, the phenyl moiety of which may optionally bear 1 or 2 substituents selected from a group consisting of halogeno, (1-4C)alkyl, (1-4C)alkoxy and trifluoromethyl; and

n is the integer 0, 1 or 2;

Rc is selected from a group consisting of hydrogen and (1-4C)alkoxy; and

M is an acidic group selected from a group consisting of carboxy, an acylsulphonamide residue of formula $-CO.NH.SO_2R^6$ and 1H-tetrazol-5-yl in which

$R^6$ is selected from a group consisting of (1-6C)alkyl, (3-8C)cycloalkyl, (6-12C)aryl, heteroaryl selected from furyl, thienyl and pyridyl, and (6-12C)aryl-(1-4C)alkyl, in which any of the aromatic or heteroaromatic moieties may bear 1 or 2 substituents selected from a group consisting of halogeno, (1-4C)alkyl, (1-4C)-

alkoxy, and trifluoromethyl;

or a pharmaceutically acceptable salt thereof.

It will be appreciated that certain of the compounds of formula I, for example those wherein $R^1$ contains an asymmetrically substituted carbon atom, may exist in, and be isolated in, optically-active and racemic forms. In addition, it will be appreciated that certain compounds of formula I, for example, those wherein L contains a double bond, may exist in, and be isolated in, separate stereoisomeric forms ('E' and 'Z') about that group. Some compounds may exist in more than one tautomeric form. Some compounds may exhibit polymorphism. It is to be understood that the present invention encompasses any racemic, optically-active, tautomeric, polymorphic or stereoisomeric form, or mixtures thereof, which form possesses leukotriene antagonist properties, it being well known in the art how to prepare optically-active forms (for example, by resolution of the racemic form or by synthesis from optically-active starting materials) and individual 'E' and 'Z' stereoisomers (for example, by chromatographic separation of a mixture thereof) and how to determine the leukotriene antagonist properties by the standard tests described hereinafter.

In this specification L, P, M, $R^1$, Ra, et cetera stand for generic radicals and have no other significance. It is to be understood that the generic term "(1-6C)alkyl" includes both straight and branched chain alkyl radicals but references to individual alkyl radicals such as "propyl" embrace only the straight chain ('normal") radical, branched chain isomers such as "isopropyl" being referred to specifically. A similar convention applies to other generic groups, for example, "alkylene" and "alkenylene" et cetera. Halogeno is fluoro, chloro, bromo or iodo.

Included in the ranges and values for the generic radicals are those wherein:

Ra is hydrogen;

Rb is hydrogen;

$R^1$ is (3-7C)alkyl optionally containing 1 or more fluorine substituents; or $R^1$ is phenyl-(1-4C)alkyl in which the (1-4C)alkyl moiety may optionally bear a fluoro or (1-4C)alkoxy substituent and in which the phenyl moiety may optionally bear a substituent selected from a group consisting of halogeno, (1-4C)alkyl, (1-4C)alkoxy and trifluoromethyl; or $R^1$ is (3-6C)cycloalkyl or (3-6C)cycloalkyl-(1-4C)alkyl, the cyclic moiety of any of which optionally may contain one unsaturated linkage and may optionally bear 1 or 2 (1-4C)alkyl substituents;

L is a (1-5C)alkylene link, optionally containing one double or triple bond;

P is a polar group selected from a group consisting of cyano, 1H-tetrazol-5-yl, carbamoyl of formula $CNR^2R^3$, carbamoyloxy of formula $OCONR^2R^3$, and an (optionally oxidized) thio group of formula $S(O)_nR^5$ in which

$R^2$ is selected from a group consisting of hydrogen, (1-6C)alkyl, and phenyl, the phenyl moiety of which may optionally bear 1 or 2 substituents selected from a group consisting of halogeno, (1-4C)alkyl, (1-4C)-alkoxy and trifluoromethyl, and

$R^3$ is hydrogen or (1-6C)alkyl;

or $R^2$ and $R^3$ together with the adjacent nitrogen form a pyrrolidine, piperidine, morpholine, piperazine or N-(1-6C)alkyl-piperazine ring;

$R^5$ is chosen from a group consisting of (1-4C)alkyl and phenyl, the phenyl moiety of which may optionally bear 1 or 2 substituents selected from a group consisting of halogeno, (1-4C)alkyl, (1-4C)alkoxy and trifluoromethyl; and

n is the integer 1 or 2;

Rc is selected from a group consisting of hydrogen and (1-4C)alkoxy; and

M is an acidic group selected from a group consisting of carboxy, an acylsulphonamide residue of formula $-CO.NH.SO_2R^6$ and 1H-tetrazol-5-yl in which

$R_6$ is selected from a group consisting of (1-4C)alkyl, (3-6C)cycloalkyl, (6-12C)aryl, heteroaryl selected from furyl, thienyl and pyridyl, and (6-12C)aryl-(1-4C)alkyl, in which any of the aromatic or heteroaromatic moieties may bear 1 or 2 substituents selected from a group consisting of halogeno, (1-4C)alkyl, (1-4C)-alkoxy, and trifluoromethyl.

Particular values for the ranges of generic radicals described above under L, P, M, $R^1$, Ra et cetera are as follows:

A particular value for $R^1$ when it is (2-10C)alkyl is, for example, ethyl, propyl, isopropyl, butyl, isobutyl, sec-butyl, t-butyl, pentyl, 1-ethylpropyl, hexyl, heptyl, 1-ethylpentyl or nonyl; and when it contains 1 or more fluorine substituents a particular value is, for example 2,2,2-trifluoroethyl.

Particular values for $R^1$ when it is phenyl(1-6C)alkyl include, for example, benzyl, 1-phenylethyl, 2-phenylethyl, 1-phenylpropyl, 2-phenylpropyl, 3-phenylpropyl, 1-methyl-1-phenylethyl, 1-phenylbutyl and 1-phenylpentyl; and a particular value for an optional (1-4C)alkoxy substituent on the (1-6C)alkyl moiety is, for example, methoxy and ethoxy.

Particular values for certain optional substituents which may be present on a phenyl moiety of $R^1$, or as a part thereof, as defined above, include, for example:

for halogeno: a member selected from a group consisting of fluoro, chloro and bromo;

for (1-4C)alkyl: a member selected from a group consisting of methyl and ethyl; and

for (1-4C)alkoxy: a member selected from a group consisting of methoxy and ethoxy.

A particular value for $R^1$ when it is (3-8C)cycloalkyl is, for example, cyclopropyl, cyclobutyl, cyclopentyl, cyclohexyl or cycloheptyl; when $R^1$ is (3-8C)cycloalkyl-(1-6C)alkyl a particular value is, for example, cyclobutylmethyl, cyclopentylmethyl, cyclohexylmethyl, 1-cyclopentylethyl, 2-cyclopentylethyl, 1-cyclopentylpropyl, 1-cyclohexylpropyl, 1-cyclopentylbutyl, 1-cyclohexylbutyl; and a particular value for $R^1$ when it is a radical containing an unsaturated linkage in the cycloalkyl ring is, for example, cyclopentenyl, cyclohexenyl, cyclopentenyl(1-6C)alkyl (such as cyclopentenylmethyl) or cyclohexenyl(1-6C)alkyl (such as 1-cyclohexen-4-ylmethyl or 1-(cyclohexenyl)butyl); and a particular value for an optional (1-4C)alkyl subsituent on the cyclic moiety of such a radical is, for example, methyl, ethyl or isopropyl.

A particular value for L when it is (1-10C)alkylene is, for example, methylene, ethylene, ethylidene, trimethylene, tetramethylene, 1,1-dimethylethylene, 2,2-dimethylethylene, pentamethylene, or hexamethylene. When L contains one double bond, a particular value is, for example, vinylene, 1-propenylene, 2-propenylene, 1-butenylene, 2-butenylene, 1,2-dimethylvinylene, 1,1-dimethyl-2-propenylene or 3,3-dimethyl-1-propenylene, and when it contains one triple bond is, for example, ethynylene, 1-propynylene, 2-propynylene, 2-butynylene, 1,1-dimethyl-2-propynylene or 3,3-dimethyl-1-propynylene;

A particular value for $R^2$, $R^3$, $R^4$, or the N-substituent of a piperazine when it is (1-6C)alkyl is, for example, methyl, ethyl, propyl, isopropyl, butyl, t-butyl or pentyl;

A particular value for $R^5$ or Ra when it is (1-4C)alkyl is, for example, methyl, ethyl, propyl or isopropyl;

Particular values for optional substituents which may be present on a phenyl moiety of $R^2$ or $R^5$ include those defined above in connection with a phenyl moiety in $R^1$.

A particular value for Rc when it is (1-4C)-alkoxy is, for example, methoxy or ethoxy;

A particular value for $R^6$ when it is (1-6C)alkyl is, for example, methyl, ethyl, propyl, isopropyl or butyl; when $R^6$ is (3-8C)cycloalkyl a particular value is, for example, cyclopentyl or cyclohexyl; when $R^6$ is (6-12C)aryl a particular value is, for example, phenyl, 1-naphthyl or 2-naphthyl; and when $R^6$ is (6-12C)aryl-(1-4C)alkyl a particular value is, for example, benzyl, 1-naphthylmethyl or 2-naphthylmethyl.

Particular values for optional substitutents which may be present on an aromatic or heteroaromatic moiety of $R^6$ include those defined above in connection with a phenyl moiety in $R^1$.

Thus, particular values for the radicals include for $R^1$: cyclopentylmethyl; for Ra and Rb: hydrogen; for Rc: methoxy; for L: methylene, ethylene, 2-methylethylene, and vinylene; for M: carboxy and an acylsulphonamide residue of formula $-CO.NH.SO_2R^6$ in which $R^6$ is phenyl or 2-methylphenyl;
and for P: cyano, carbamoyl of formula $CONR^2R^3$ and carbomoyloxy of formula $OCONR^2R^3$ wherein $R^2$ and $R^3$ are independently hydrogen or methyl, or $R^2$ and $R^3$ together with the adjacent nitrogen form a pyrrolidine or morpholine ring.

More particular values for the groups listed above include by way of example those selected from the groups consisting of:

for $R^1$: ethyl, propyl, isopropyl, butyl, isobutyl, sec-butyl, t-butyl, pentyl, 1-ethyl-propyl, hexyl, heptyl, 1-ethylpentyl, nonyl, benzyl, 4-chlorobenzyl, 4-trifluoromethylbenzyl, 4-methylbenzyl, 1-phenylethyl, 2-phenylethyl, 1-methyl-1-phenylethyl, 1-phenylpropyl, 1-phenylpentyl, cyclopentyl, cyclohexyl, cyclobutylmethyl, cyclopentylmethyl, cyclohexylmethyl, 2-cyclopentylethyl, 1-cyclopentylbutyl, 1-cyclohexylpropyl, 1-cyclohexylbutyl, cyclopentenylmethyl, and 1-cyclohexen-4-ylmethyl;

for $R^2$ : hydrogen, methyl, ethyl, propyl, isopropyl, t-butyl, phenyl, 2-methylphenyl and 4-chlorophenyl;

for $R^3$ and $R^4$ (independently selected): hydrogen, methyl and ethyl;

for $R^2$ and $R^3$ together with the adjacent nitrogen: piperidine, morpholine, and N-methylpiperazine;

for $R^5$: methyl, ethyl, propyl, isopropyl, phenyl, 2-methylphenyl and 4-chlorophenyl;

for $R^6$: methyl, isopropyl, butyl, cyclopentyl, phenyl, 4-chlorophenyl, 4-methylphenyl, 2-chlorophenyl, 2-methylphenyl, 2-methoxyphenyl, naphthyl, thien-2-yl and 6-chloropyrid-3-yl;

for Ra: hydrogen and methyl;

for Rb: hydrogen; and

for Rc: hydrogen and methoxy.

Examples of specific groups which are of special interest include those selected from groups consisting of:

for $R^1$: butyl, 1-ethylpentyl, 1-phenylpropyl, cyclopentyl, and cyclopentylmethyl;

for $R^6$: phenyl, 2-methylphenyl;

for Ra: hydrogen; and

for Rc: methoxy.

It will be appreciated that within the above definitions there are included a number of sub-groups of compounds, for example:

(i) indoles of formula Ia;

(Formula set out on pages following Examples) Ia

(ii) indazoles of Formula Ib;

(Formula set out on pages following Examples) Ib

and (iii) indolines of formula Ic;

(Formula set out on pages following Examples) Ic

together with the pharmaceutically acceptable salts thereof.

In the above sub-groups a preferred value for M is a radical of formula $-CO.NH.SO_2R^6$ wherein $R^6$ is phenyl, optionally substituted as defined above, for example, 2-methylphenyl. A preferred value for $R^1$ is (3-6C)cycloalkyl(1-4C)alkyl and, especially, cyclopentylmethyl.

Preferred groups of compounds of the invention comprise the indole derivatives of formula IIa:

(Formula set out on pages following Examples) IIa

and indazole derivatives of formula IIb:

(Formula set out on pages following Examples) IIb

wherein $R^1$, L, M and P have any of the meanings defined above, together with the pharmaceutically acceptable salts thereof.

Specific compounds of the invention are described in the accompanying examples.

Examples of suitable pharmaceutically acceptable salts are salts formed with bases which form a physiologically acceptable cation, such as alkali metal (especially sodium and potassium), alkaline earth metal (especially calcium and magnesium), aluminum and ammonium salts, as well as salts made with appropriate organic bases such as triethylamine, morpholine, piperidine and triethanolamine. For those compounds of formula I which are sufficiently basic, examples of suitable pharmaceutically acceptable salts include acid-addition salts such as those made with a strong acid, for example hydrochloric, sulphuric or phosphoric acid.

The compounds of formula I may be made by processes which include processes known in the chemical art for the production of structurally analogous heterocyclic compounds. Such processes for the manufacture of a compound of formula I as defined above are provided as further features of the invention and are illustrated by the following procedures in which the meanings of generic radicals are as defined above, T is defined as a radical selected from a group consisiting of COORh (wherein Rh has the values defined below), CN, and the values defined above for M; U is defined as a suitable leaving group, for example, halogeno (especially chloro, bromo, or iodo) or alkane- or arene-sulphonyloxy (especially methane-sulphonyloxy or p-toluenesulphonyloxy); and Hal is defined as chloro, bromo or iodo:

(A) For a compound of formula I wherein M is a carboxy group, decomposing a suitable ester of formula III:

(Formula set out on pages following Examples) III wherein Rh is a conveniently removed acid protecting group, for example, phenyl, benzyl, or (1-6C)alkyl optionally bearing an acetoxy, (1-4C)alkoxy or (1-4C)alkylthio substituent.

A particular value for Rh is, for example, methyl, ethyl, propyl, t-butyl, acetoxymethyl, methoxymethyl, 2-methoxyethyl, methylthiomethyl, phenyl, or benzyl.

Certain of the starting esters of formula III may be active in their own right as leukotriene antagonists (such as, for example, by in vivo conversion to the corresponding carboxylic acid), for example, those wherein Rh is (1-6C)alkyl, and they are included within the scope of the invention.

It will be appreciated that the decomposition can be performed using any one of a variety of procedures well known in the art of organic chemistry. Thus, it may be carried out, for example, by conventional hydrolysis under acid or base conditions, adjusted as necessary to minimize any hydrolytic removal of other functional groups in the molecule. Also, when Rh is methyl, the ester may be decomposed by nucleophilic demethylation with, for example, lithium thioethoxide in a solvent such as N,N'-dimethylpropyleneurea. Alternatively, it may in certain circumstances, for example, when Rh is t-butyl, be possible to carry out the decomposition by thermal means, for example, by heating the ester of formula III at a temperature of, for example, 100-150 °C, alone or in a suitable solvent or diluent such as diphenylether. In addition, when Rh is t-butyl the decomposition may be performed, for example, by using trimethylsilyl triflate and then water, in a conventional manner. Still further, in certain circumstances, for example, when Rh is benzyl, it may be possible to carry out the decomposition by reductive means, for example, by the use of hydrogen at about atmospheric pressure in the presence of a suitable catalyst, such as palladium or platinum, conveniently on charcoal as a support.

A preferred method for decomposing an ester of formula III comprises reacting the ester with a suitable base, for example, an alkali or alkaline earth metal hydroxide or carbonate (such as lithium hydroxide, potassium hydroxide, sodium hydroxide, calcium hydroxide or potassium carbonate) in a suitable aqueous solvent or diluent, for example, water, optionally together with a water-miscible alkanol, glycol, ketone or ether (such as methanol, ethanol, ethylene glycol, 2-methoxyethanol, acetone, methyl ethyl ketone, tetrahydrofuran or 1,2-dimethoxyethane), at a temperature of, for example, 15-100 °C and conveniently at or near ambient temperature. When such a method is employed, the resulting carboxylic acid of formula I, where M is a carboxy group, is initially obtained as the corresponding salt of the base used for the hydrolysis and may be isolated as such or converted to the free acid form by a conventional acidification procedure, for example, by reaction with a suitable strong acid such as hydrochloric or sulphuric acid.

(B) Acylating an amine of formula $R^1NHRb$ with a carboxylic acid (or a reactive derivative thereof) of formula IV:

(Formula set out on pages following Examples) IV but wherein T is chosen from the values defined for M. When M is a carboxy group, a preferred reactive derivative of the carboxy group shown in formula IV is a lower alkyl ester of the carboxy group shown in formula IV, for example, the methyl ester.

When an acid halide derivative of a compound of formula IV is used as the acylating agent, a suitable base such as triethylamine, N-methylmorpholine, pyridine, 2,6-lutidine or 4-(dimethylamino)-pyridine is conveniently also employed, preferably together with a suitable inert solvent or diluent, for example, dichloromethane, diethly ether, tetrahydrofuran or 1,2-dimethoxyethane.

Alternatively, a suitable condensing agent, for example, a carbodiimide (such as dicyclohexylcarbodiimide or 1-(3-dimethylaminopropyl)-3-ethylcarbodiimide, or a salt thereof) or 1,1′-carbonyldiimidazole, may be employed with an acid of formula IV, preferably together with a suitable inert solvent or diluent, for example, one of those mentioned above for use with an acid halide.

When a lower alkyl ester derivative of a compound of formula IV is used as an acylating agent, the reaction is preferably performed in the absence of any condensing agent or diluent and in the presence of an excess of the amine $R^1NHRb$.

In general, the acylations are carried out at a temperature in the range of, for example, -20 to 60 °C and, conveniently, at or near ambient temperature.

(C) For a compound of formula I wherein -Y-Z< has the value (a) defined hereinabove, reacting an indole of formula V:

(Formula set out on pages following Examples) V but wherein T is chosen from the values defined for M, with a reagent of formula U.L.P, in the presence of a suitable Lewis acid.

A particularly suitable Lewis acid is, for example, silver oxide, silver carbonate, silver fluoroborate, silver trifluoroacetate, silver trifluoromethanesulfonate, zinc chloride, ferric chloride or stannic chloride.

In general, the process is performed in a suitable solvent or diluent, for example, in acetone, dichloromethane, acetonitrile or an ether solvent such as 1,2-dimethoxyethane, dioxane or tetrahydrofuran, optionally together with a hydrocarbon diluent such as toluene or xylene, and at a temperature in the range of, for example, 15-100 °C and, more preferably, in the range of 40-80 °C.

Alternatively, in the absence of a Lewis acid catalyst, the process is generally performed in a suitable solvent or diluent, for example, in a polar solvent (such as N,N-dimethylformamide, N,N′-dimethylpropyleneurea of N-methylpyrrolidone) or in an ether solvent (such as dioxane or 1,2-dimethoxyethane), optionally together with a hydrocarbon diluent such as toluene or xylene; and the alkylation is generally performed at a temperature in the range of, for example 50-160 °C, and, preferably, in the range of 70-100 °C.

(D) Reacting an imino compound of formula VI:

(Formula set out on pages following Examples) VI with an alkylating agent of formula VII:

(Formula set out on pages following Examples) VII but wherein T is chosen from the values defined for M.

The reaction is preferably performed in the presence of a suitable base, for example, an alkali metal hydride such as sodium or potassium hydride in a suitable inert solvent or diluent, for example, tetrahydrofuran, 1,2-dimethoxyethane, N-methylpyrrolidone, or N,N-dimethylformamide. Alternatively, the compound of formula VI may be used in the form of its preformed anhydrous alkali metal salt, for example, by prior reaction with a suitable base such as sodium or potassium methoxide, t-butoxide or hydride, or butyl lithium; in which case a wider range of conventional solvents or diluents may be employed for the reaction with the alkylating agent.

In either case, the alkylation is generally performed at a temperature in the range of, for example, -10 to 40 °C and, conveniently, at or near ambient temperature.

(E) For a compound of formula I wherein M is a 1H-tetrazol-5-yl radical, reacting a cyano derivative of formula VIII:

(Formula set out on pages following examples) VIII with an azide.

A particularly suitable azide is, for example, an alkali metal azide such as sodium or potassium azide, preferably together with an ammonium halide, for example, ammonium chloride or ammonium bromide or, especially, with triethylammonium chloride. The reaction is preferably performed in a suitable polar solvent, for example, N,N-dimethylformamide or N-methylpyrrolidone, and conveniently at a temperature in the range of, for example, 50 to 160 ° C.

(F) For a compound of formula I where M is a group of formula $CO.NH.SO_2.R^6$, reacting a compound of formula I wherein M is carboxy (which compound is hereinafter referred to as "acid of formula I") with a sulphonamide derivative of formula $R^6.SO_2.NH_2$ in the presence of a dehydrating of an acid of formula I with a sulphonamide, or a salt thereof, of formula $R^6.SO_2.NH_2$.

Thus, for example, a free acid of formula I may be reacted with a suitable dehydrating agent, for example, with dicyclohexylcarbodiimide or 1-(3-dimethylaminopropyl)-3-ethylcarbodiimide, or with a hydrochloride or hydrobromide salt thereof, optionally together with an organic base, for example, 4-(dimethylamino)pyridine, and with a sulfonamide of formula $R^6.SO_2.NH_2$ in the presence of a suitable solvent or diluent, for example, dichloromethane, at a temperature in the range of, for example, 10 to 50 ° C, but preferably at or near ambient temperature.

Alternatively, a reactive derivative of an acid of formula I, for example, an acid halide (such as the acid chloride), acid anhydride or a mixed acid anhydride (such as that formed from N,N-diphenylcarbamic acid and the acid of formula I by reaction of the sodium salt of the latter acid with N,N-diphenylcarbamoylpyridinium chloride), may be reacted with an alkali metal salt (such as the lithium, sodium or potassium salt) of the appropriate sulphonamide of formula $R^6.SO_2.NH_2$, conveniently at or near ambient temperature and in a suitable solvent or diluent, for example, tetrahydrofuran, N,N-dimethylformamide or dichloromethane.

(G) For a compound of formula I wherein -Y-Z< has the value (c) defined above, catalytic hydrogenation of an indole of formula I wherein -Y-Z< has the value (a) defined above.

Particularly suitable catalytic hydrogenation conditions are those of catalytic transfer hydrogenation, for example, palladium-on-carbon (10% w/w) and formic acid (99%) at a temperature in the range of, for example, 15-100 ° C, more preferably in the range of 70-85 ° C.

(H) Reduction of the double bond of a compound of formula I in which L contains one double bond to provide the corresponding compound of formula I in which L contains no double bond.

Preferred reduction conditions include, for example, catalytic hydrogenation over palladium on carbon in a suitable solvent such as methanol, ethanol or tetrahydrofuran at ambient temperature and, optionally, the addition of an equivalent of a base, such as, for example, potassium hydroxide or triethyl amine.

(I) For a compound of formula I in which P is a carbamoyl group of the formula $CONR^2R^3$, acylation of an amine of the formula $HNR^2R^3$ with a corresponding acid (or a reactive derivative thereof, including suitable esters) of formula IX:

(Formula set out on pages following Examples) IX

but wherein T is chosen from the values defined for M.

When M is a carboxy group, it is preferred to use a lower alkyl ester derivative of the carboxy group shown in formula IX as the reactive derivative, for example, the methyl ester. The reaction may be performed using similar procedures to those described above in part (B).

(J) For a compound of formula I in which P is a 1H-tetrazol-5-yl group, reacting a nitrile of formula X:

(Formula set out on pages following Examples) X

but wherein T is chosen from the values defined for M, with an aside using similar procedures to those described above in part (E).

(K) For a compound of formula I in which P has the value $NR^4CONR^2R^3$, $OCONR^2R^3$, $NR^4COOR^5$, acylating a compound of formula XI:

(Formula set out on pages following Examples) XI

but wherein T is chosen from the values defined for M and wherein QH has the value $NR^4H$ or OH, with an appropriate acylating agent, for example, an isocyanate of the formula $R^2NCO$, a carbamoyl halide of formula $Hal.CONR^2R^3$, a haloformate of formula Hal. $COOR^5$ or a mixed carbonate such as (4-nitrophenoxy).$COOR^5$.

In general, the process is performed at a temperature in the range of, for example, 0-60 ° C and conveniently in a suitable inert diluent or solvent such as methylene chloride, diethyl ether, tetrahydrofuran or dioxane. When an acid halide is used as the acylating agent, a suitable base such as

triethylamine, N-methylmorpholine, piperidine or 4-(dimethylamino)pyridine is conveniently also employed.

(L) For a compound of formula I in which P has the value $S(O)_nR^5$ and n is 0, reacting a compound of formula XII:

(Formula set out on pages following Examples) XII

but wherein T is chosen from the values defined for M, with a mercaptan of formula $R^5SH$.

In general, the process is performed using an appropriate base, such as, for example, potassium carbonate, sodium hydroxide or sodium hydride, at a temperature in the range of, for example, 0 to 80 °C, and, optionally, in a suitable inert diluent or solvent such as, for example, acetone, tetrahydrofuran, dioxane, or N,N-dimethylformamide.

(M) For a compound of formula I in which P has the value $S(O)_nR^5$ and n is 1 or 2, oxidizing the corresponding compound I in which n is 0 or n is 1.

In general, the process is performed at a temperature in the range of, for example, -20 to 60 °C in a suitable inert diluent or solvent such as, for example, methylene chloride, tetrahydrofuran or diethyl ether or aqueous methanol and with a suitable oxidant such as, for example, potassium peroxymonosulfate, sodium periodate or a peroxy acid such as, for example, m-chloroperbenzoic acid.

(N) For a compound of formula I wherein L contains a double bond adjacent to Z, reacting an aldehyde or ketone of formula XIII:

(Formula set out on pages following Examples) XIII but wherein T is chosen from the values defined for M and wherein $R^7$ is selected from hydrogen and (1-4C)alkyl, with an appropriate zwitterionic or carbanionic reagent, such as, for example, that derived from a phosphonium salt or a phosphonate by treatment with a base.

The reaction is preferably performed with an excess of a zwitterionic reagent, such as, for example, cyanomethylenetriphenylphosphorane, or of a carbanionic reagent in a suitable inert solvent or diluent such as, for example, tetrahydrofuran, dioxane, 1,2-dimethoxyethane, N,N-dimethylformamide or dimethylsulfoxide at a temperature in the range of, of example, 20 to 100 °C.

It may be desired to optionally use a protecting group during all or portions of the above described processes (A)-(N); the protecting group then may be removed when the final compound is to be formed.

In general, when a compound of formula I wherein M is a carboxylic acid is required, it is preferred to carry out one of the procedures (B), (C), (D), (G), (H), (K), (M) and (N) mentioned above using an appropriate carboxylic ester and liberating the required acid as a final step using procedure (A) above.

Pharmaceutically acceptable salts may be obtained using standard procedures well known in the art, for example, by reacting a compound of formula I with a suitable base affording a physiologically acceptable cation or by reacting a sufficiently basic compound of formula I with a suitable acid affording a physiologically acceptable anion.

If not commercially available, the necessary starting materials for the above procedures may be made by procedures which are selected from standard techniques of heterocyclic chemistry, techniques which are analogous to the synthesis of known, structurally similar compounds, and techniques which are analogous to the above described procedures or the procedures described in the examples.

Thus, for example, for those intermediates in which -Y-Z< has the value (a) (-C(Ra)=C<), which are indoles, preparation may begin with the corresponding 2-substituted indole-6-carboxylic acid which may be converted to a corresponding amide of formula XIV:

(Formula set out on pages following Examples) XIV using a similar procedure to one of process (B). Substitution of an intermediate of formula XIV, at the N(1)- and C(3)-position may be carried out in a series of steps in any convenient order.

By using a similar procedure to that described in process (D) and using a compound of formula VII, a compound of formula XIV, wherein -Y-Z< has the value (a), may be converted into a corresponding indole of formula V. An indole of formula V may serve as an intermediate for other starting materials in which the value of -Y-Z< is (a). For example, using a similar method to method (C), a compound of formula V, but wherein T has the value COORh, may be converted into a compound of formula III, wherein -Y-Z< has the value (a). Similarly, a compound of formula V, but wherein T has the value CN, may be converted into a compound of formula VIII wherein -Y-Z< has the value (a). Also, by using a similar procedure to that of the process (C) and an appropriate alkylating agent (or protected version thereof, followed by deprotection), an indole of formula V may be converted into a corresponding intermediate of formula IX, X, XI, or XII, wherein -Y-Z< has the value (a).

Alternatively, an indole of formula XIV may be first alkylated at the C(3)-position using a similar procedure to that described in process (C) to afford a corresponding compound of formula VI wherein -Y-Z< has the value (a). By using a similar process to process (C) and an appropriate alkylating agent (or a

protected version thereof, followed by deprotection) and then using a similar process to process (D) and an appropriate compound of formula VII as an alkylating agent, an indole of formula XIV may be converted into a corresponding intermediate of formula III, VIII, IX, X, XI or XII, wherein -Y-Z< has the value (a).

In addition a compound of formula XIV may be acylated at the C(3)-position with, for example, the appropriate N,N-dimethylamide of formula $(CH_3)_2NCOR^7$ and phosphorous oxychloride and subsequently alkylated at the N(1)-position with a compound of formula VII employing a similar procedure to those described in part (D) to afford a compound of formula XIII, but wherein -Y-Z< has the value (a).

In general, an indoline intermediate in which -Y-Z< has the value (c) (-CH(Ra)-CH<) may be obtained by using a similar procedure to process (G) and from a corresponding indole in which -Y-Z< has the value (a).

Starting materials in which -Y-Z< has the value (b) (-N=C<) conveniently may be prepared from indazole-6-carboxylic acid, which may be (i) brominated to provide 3-bromoindazole-6-carboxylic acid, (ii) converted into an amide by using a similar procedure to that described in process (B), and (iii) alkylated at the N(1)-position using a similar procedure to that of process (D) and a compound of formula VII as an alkylating agent to provide an indazole of formula XV:

(Formulas set for on pages following Examples) XV in which T′ has the value COORh or CN.

An indazole of formula XV, but wherein T′ has the value COORh, may be converted into a starting material of formula III wherein -Y-Z< has the value (b) by a cross coupling reaction using a transition metal catalyst such as, for example, dichloro[1,1′-bis(diphenylphosphino)ferrocene]palladium(II) and a reagent such as, for example, BR.Zn.L.P, provided that the moiety -L.P does not interfere with the reaction or undergo reaction under the conditions, and an appropriate solvent or diluent such as, for example, ether or tetrahydrofuran at a temperature in the range of, for example, 0 to 25 °C. If the moiety -L.P does interfere with the reaction or undergo reaction under the conditions, a protected form may be used, followed by deprotection.

Similarly, a starting material of formula VIII wherein -Y-Z< has the value (b) may be obtained from a compound of formula XV, but wherein T′ has the value CN, and a reagent such as, for example, BR.Zn.L.P, or a protected derivative thereof.

By using analogous methodology and a compound of formula XV, a starting material of formula IX, X, XI or XII, but wherein -Y-Z< has the value (b), and wherein T has the value COORh or CN, may be obtained from a corresponding precursor to the C(3) substituent, or a protected or latent form, thereof, followed by deprotection or elaboration.

When a starting material of formula IV is desired, instead of converting a starting indole-6-carboxylic acid or indazole-6-carboxylic acid into an amide by a similar process to process (B), (i) the acid may be converted into its corresponding ester, for example, of an alcohol of a formula RhOH; (ii) processes analogous to those described above may be carried out, and (iii) the ester may be decomposed to afford the acid IV using a similar procedure to one of those described for process (A).

In general, an intermediate compound having the value Rb as methyl may be obtained from a corresponding compound having the value Rb as hydrogen by alkylation using, for example, a base such as sodium hydride, a methylating agent such as iodomethane or dimethylsulfate, and a solvent such as N,N-dimethylformamide at a temperature in the range of 0-25 °C.

Also, intermediates of formula XIII may be converted into other corresponding intermediates of formulae III, VIII, IX, X, XI and XII by standard methods of organic chemistry.

Generally, starting esters of formula III or starting nitriles of formula VIII, respectively, may be made using general procedures similar to those described in (D) by using VII, wherein T has the value COORh or CN, respectively, as an alkylating agent. Also, generally, starting esters of formula III or starting nitriles of formula VIII, may be made using similar general procedures to those described in (B) and (C) using corresponding intermediates IV and V but wherein T stands for COORh or CN, as appropriate, in said intermediates.

A nitrile of formula VIII may be obtained from a corresponding compound of formula I wherein M is carboxy by treatment with, for example, chlorosulphonyl isocyanate and N,N-dimethylformamide. Alternatively, a cyano compound of formula VIII may be obtained by conventional dehydration of the primary amide derived from a corresponding carboxylic acid of formula I wherein M is carboxy.

The intermediate alcohols and amines of formula XI, wherein -Y-Z< has the value (a), may be obtained by alkylation of the corresponding compounds of formula V with reagents of the formula U.L.QH (in which QH is optionally protected) using similar procedures to those described in part (C) above.

Alternatively, the intermediate alcohols of formula XI wherein Q is oxygen may be obtained by selective reduction of the corresponding acids of formula IX using, for example, diborane in tetrahydrofuran at ambient temperature.

The intermediates XII may be obtained from the corresponding alcohols XI wherein Q is oxygen by

appropriate transformations, for example, by reaction with p-toluenesulphonyl chloride, with methanesulphonyl chloride, or with triphenylphosphine and carbon tetrachloride or carbon tetrabromide in an appropriate solvent.

The majority of the starting materials of formulae III, IV, V, VI, VIII, IX, X, XI and XII are novel and are provided as further features of the invention based on their utility as chemical intermediates.

As stated previously, the compounds of formula I possess leukotriene antagonist properties. Thus, they antagonise at least one of the actions of one or more of the arachidonic acid metabolites known as leukotrienes, for example, $C_4$, $D_4$, and/or $E_4$, which are known to be powerful spasmogens (particularly in the lung), to increase vascular permeability and which have been implicated in the pathogenesis of asthma and inflammation (see J. L. Marx, Science, 1982, 215, 1380-1383) as well as of endotoxic shock (see J. A. Cook, et al., J. Pharma col. Exp. Ther., 1985, 235, 470) and traumatic shock (see C. Denzlinger, et al., Science, 1985, 230, 330). Thus, the compounds of formula I may be useful in the treatment of diseases in which leukotrienes are implicated and in which antagonism of their action is desired. Such diseases include, for example, allergic pulmonary disorders such as asthma, hay fever and allergic rhinitis and certain inflammatory diseases such as bronchitis, ectopic and atopic eczema, psoriasis, as well as vasospastic cardiovascular disease, and endotoxic and traumatic shock conditions.

The compound of formula I are potent leukotriene antagonists and are useful whenever such activity is desired. For example, the compounds of formula I are of value as pharmacological standards for the development and standardization of new disease models and assays for use in developing new therapeutic agents for treating the diseases in which the leukotrienes are implicated.

When used in the treatment of one or more of the above mentioned diseases, a compound of formula I is generally administered as an appropriate pharmaceutical composition which comprises a compound of formula I as defined hereinbefore together with a pharmaceutically acceptable diluent or carrier, the composition being adapted for the particular route of administration chosen. Such compositions are provided as a further feature of the invention. They may be obtained employing conventional procedures and excipients and binders and may be in a variety of dosage forms. For example, they may be in the form of tablets, capsules, solutions or suspensions for oral administration; in the form of suppositories for rectal administration; in the form of sterile solutions or suspensions for administration by intravenous or intramsucular injection or infusion; in the form of aerosols or nebuliser solutions or suspensions for administration by inhalation; and in the form of powders together with pharmaceutically acceptable inert solid diluents such as lactose for administration by insufflation.

For oral administration a tablet or capsule containing up to 250 mg (and typically 5 to 100 mg) of a compound of formula I may conveniently be used. Similarly, for intravenous or intramuscular injection or infusion a sterile solution or suspension containing up to 10% w/w (and typically 0.05 to 5% w/w) of a compound of formula I may conveniently be used.

The dose of compound of formula I to be administered will necessarily be varied according to principles well known in the art taking account of the route of administration and the severity of the condition and the size and age of the patient under treatment. However, in general, a compound of formula I will be administered to warm-blooded animal (such as man) so that a dose in the range of, for example, 0.05 to 25 mg/kg (and usually 0.5 to 10 mg/kg) is received.

The leukotriene antagonist properties of a compound of formula I may be demonstrated using standard tests. Thus, for example, they may be demonstrated in vitro using the standard guinea-pig tracheal strip preparation described by Krell (J. Pharmacol. Exp. Ther., 1979, 211, 436). Using this procedure, tracheal tissue strips are set up in groups of eight, four being used as time/vehicle (dimethyl sulfoxide) controls and four for each test compound. All of the strips are exposed to $8x10^{-9}M$ leukotriene $E_4(LTE_4)$ following the 50 minutes equilibration period, and the response is recorded. This $8x10^{-9}M$ concentration of $LTE_4$ is that which produces a contraction equal to about 70-80% of the maximal effect of the agonist in this tissue. The $LTE_4$ is washed out for 40-45 minutes and the procedure is repeated twice to ensure that reproducible responses are being obtained with $LTE_4$. Leukotriene $C_4(LTC_4)$ or $D_4(LTD_4)$, at a concentration of $8x10^{-9}M$, may be substituted for $LTE_4$ in the same procedure.

Once tissue reproducibility has been established, test compounds are added to four baths following the 40-45 minute washout period. After a 10 minute incubation with test compound or vehicle, $8x10^{-9}M$ $LTE_4$, $LTD_4$ or $LTC_4$ is added and the response recorded. The percentage inhibition by the test compound or the percentage change in vehicle controls is calculated, for each tissue, according to the following equation: % inhibition = 100 multiplied by (mg tension increase of preceding response minus mg tension increase in presence of compound) divided by mg tension increase of preceding response. The mean percentage changed for vehicle controls and test compound are calculated and evaluated for significant differences by Student's t-test for unpaired data. Tissues exposed to test compounds are retested for responsiveness to

$LTE_4$, $LTD_4$ or $LTC_4$ following a 45 minute washout period. If tissue responsiveness is equal to responsiveness preceding exposure to the test compound additional studies are conducted. If responsiveness is not restored by the washing procedure, the tissues are discarded. The cyclooxygenase inhibitor, indomethacin, is present at $5 \times 10^{-6}$M in all the determinations.

In general, the compounds of formula I tested demonstrated statistically significant activity as $LTC_4$, $LTD_4$ and/or $LTE_4$ antagonists in the above test at a concentration of about $10^{-5}$M or much less.

The selectivity of action of these compounds as leukotriene antagonists as opposed to nonspecific smooth muscle depressants may be shown by carrying out the above in vitro procedure using the nonspecific spasmogen barium chloride at a concentration of $1.5 \times 10^{-3}$M, again in the presence of indomethacin at $5 \times 10^{-6}$M.

Activity as a leukotriene antagonist may also be demonstrated in vivo in laboratory animals, for example, in a routine guinea-pig aerosol test in which guinea-pigs are pre-dosed with test compound (generally between 15 minutes to 1 hour) before an aerosol challenge of leukotriene $LTD_4$ (starting with 3 ml of a 30 microgram/ml solution) and the effect of the test compound on the average time of leukotriene initiated change in breathing pattern (such as onset of dyspnoea) recorded and compared with that in undosed, control guinea-pigs. In general, compounds of formula I tested produced a significant increase in the time of onset of leukotriene initiated breathing changes following either oral or intravenous administration or by inhalation at a dose of about 100 mg/kg, or much less, without any indication of untoward side-effects at several multiples of the minimum effective dose, for example, the compound of Example 8 is effective following oral administration at a dose of 2 mmole/kg and shows no sign of overt toxicity following oral administration at a dose of 30 mmole/kg.

The invention will now be illustrated by the following non-limiting examples in which, unless stated otherwise:

(i) temperatures are given in degrees Celsius (C); operations were carried out at room or ambient temperature, that is, at a temperature in the range of 18-25°;

(ii) evaporation of solvent was carried out using a rotary evaporator under reduced pressure (600-4000 pascals; 4.5-30 mm Hg) with a bath temperature of up to 60°;

(iii) flash chromatography was carried out on Merck Kieselgel (Art 9385) and column chromatography on Merck Kieselgel 60 (Art 7734); [these materials were obtained from E. Merck, Darmstadt, W. Germany]; thin layer chromatography (TLC) was carried out on Analtech 0.25 mm silica gel GHLF plates (Art 21521), obtainable from Analtech, Newark, DE, USA;

(iv) in general, the course of reactions was following by TLC and reaction times are given for illustration only;

(v) melting points are uncorrected and (d) indicates decomposition; the melting points given are those obtained for the materials prepared as described; polymorphism may result in isolation of materials with different melting points in some preparations;

(vi) all final products were essentially pure by TLC and had satisfactory nuclear magnetic resonance (NMR) spectra and microanalytical data;

(vii) yields are given for illustration only;

(viii) when given, NMR data is in the form of delta values for major diagnostic protons, given in parts per million (ppm) relative to tetramethylsilane (TMS) as an internal standard, determined at 80 MHz or 250 MHz using $CDCl_3$, DMSO-$d_6$ or $CD_3OD$ as solvent; conventional abbreviations for signal shape are used, for example: s, singlet; d, doublet; m, multiplet; br, broad; etc.; in addition "Ar" signifies an aromatic group or signal;

(ix) reduced pressures are given as absolute pressures in pascals (Pa); other pressures are given as gauge pressures in bars;

(x) chemical symbols have their usual meanings; the following abbreviations have also been used: v (volume), w (weight); mp (melting point), 1 [liter(s)], ml (milliliters), g [gram(s)], mg [milligram(s)]; and

(xi) solvent ratios are given in volume: volume (v/v) terms.

Example 1

4-[6-(N-Cyclopentylmethylcarbamoyl)-3-[2-(N-methylcarbamoyl)ethyl]indol-1-ylmethyl]-3-methoxybenzoic acid

A mixture of 4-[6-(N-cyclopentylmethylcarbamoyl)-3-(2-methoxycarbonylethyl)indol-1-ylmethyl]-3-methoxybenzoic acid (0.3 g) and 4-(dimethylamino)-pyridine(0.07 g) was combined with condensed methylamine (75 ml) in a pressure vessel. The mixture was stirred for 24 hours. The amine was then

allowed to evaporate. The residue was dissolved in water and acidified with 10% (v/v) hydrochloric acid. The resultant precipitate was collected by filtration and washed with water to give the title compound (0.26 g, 89%) as a white power; mp 274-275°.

Analysis calculated for:

$C_{28}H_{33}N_3O_5$.0.1 $H_2O$: C, 68.13; H, 7.15; N, 8.51

Found: C, 67.83; H, 6.75; N, 8.46

The starting material was prepared as follows:

(a) A solution of methyl 4-methyl-3-nitro-benzoate (4.46 g) in N,N-dimethylformamide (23 ml) was treated with N,N-dimethylformamide dimethyl acetal (8.18 g) and heated at 130°C for 2 hours. The solvent was evaporated and the residue was triturated with ether to give methyl E-4-(2-dimethylaminovinyl)-3-nitrobenzoate (5.58 g, 98%) as a red powder; NMR (80 MHz, $CDCl_3$): 2.98[s, 6H, $N(CH_3)_2$], 5.90(d, 1H, CHN), 7.14(d, 1H, CHCHN), 7.45(d, 1H, $H^5$-Ar), 7.90(dd, 1H, $H^6$-Ar), 8.47(d, 1H, $H^2$-Ar).

(b) A solution of methyl E-4-(2-dimethylaminovinyl)-3-nitrobenzoate (5.58 g) in tetrahydro-furan (100 ml) was hydrogenated at 3.45 bar in the presence of 10% (w/w) palladium on carbon (1.1 g) for 35 minutes. The catalyst was removed by filtration through diatomaceous earth and the filtrate was evaporated. The residue was dissolved in ethyl acetate and the solution obtained was washed successively with 10% (v/v) hydrochloric acid, water, and brine, then dried ($MgSO_4$) and evaporated to give methyl indole-6-carboxylate (3.32 g, 85%) as a white solid; NMR (80 MHz, $CDCl_3$): 3.92(s, 3H, $OCH_3$), 6.57(m, 1H, $H^3$-indole), 7.32(t, 1H, $H^2$-indole), 7.10(d, 1H, $H^4$-indole), 7.87(dd, 1H, $H^5$-indole), 8.16(broad s, 1H, $H^7$-indole).

(c) A solution of methyl indole-6-carboxylate (11.0 g) in a mixture of tetrahydrofuran (150 ml), methanol (150 ml), and water (63 ml) was treated with lithium hydroxide monohydrate (15.8 g). The mixture was stirred at 60°C for 6 hours and then concentrated to remove the organic solvents. The residue was dissolved in water, and the solution was acidified with 50% (v/v) hydrochloric acid. The precipitate which formed was collect by filtration and dried to give indole-6-carboxylic acid (9.6 g, 95%) as a tan powder; mp 253-254°; NMR (80 MHz; $CDCl_3$): 6.51(m, 1H, $H^3$-indole), 8.04(m, 1H, $H^7$-indole), 11.43(broad s, 1H, NH), 12.42(broad s, 1H, OH).

(d) A solution of indole-6-carboxylic acid (9.41 g) and 1,1'-carbonyldiimidazole (10.6 g) in methylene chloride (290 ml) was heated at reflux, under nitrogen, for 30 minutes. The solution was cooled and treated with cyclopentylmethylamine (7.0 g). This mixture was heated to reflux for 30 minutes. The resultant solution was then diluted with methylene chloride, washed successively with 10% (v/v) hydrochloric acid, 20% (w/v) aqueous sodium hydroxide, and brine, dried ($MgSO_4$), and evaporated to give 6-(N-cyclopentylmethylcarbamoyl)indole (14.4 g, 91%) as an ivory powder, mp 148-150°; NMR (80 MHz, DMSO-$d_6$): 3.19(dd, 2H, $CH_2NH$), 6.46(br d, 1H, $H^3$-indole), 7.91(d, 1H, $H^7$-indole), 8.29(t, 1H, $CH_2NH$).

(e) N,N-Dimethylformamide (DMF) (20 ml) was cooled to 0° under an atmosphere of nitrogen and treated cautiously with phosphorus oxychloride (6.6 ml). This solution was stirred at 0° for 15 minutes, warmed to room temperature, and treated with a solution of 6-(N-cyclopentylmethylcarbamoyl)indole (14.3 g) in DMF (100 ml). The yellow mixture was stirred for 2 hours and then brought to pH 14 by the addition of ice and 20% (w/v) aqueous sodium hydroxide. The mixture was heated to reflux for 5 minutes and allowed to cool. The precipitate which formed was collected by filtration and triturated with ether to give 6-(N-cyclopentylmethylcarbamoyl)-3-formylindole (9.6 g, 60%) as a tan powder; mp 224-225°.

(f) A mixture of 6-(N-cyclopentylmethylcarbamoyl)-3-formylindole (0.92 g), t-butyl 4-bromomethyl-3-methoxybenzoate (1.2 g), and potassium carbonate (0.7 g) in N,N-dimethylformamide (17 ml) was stirred for 48 hours under a nitrogen atmosphere. Water was added to give a precipitate which was collected by filtration and dried to yield t-butyl 4-[6-(N-cyclopentylmethylcarbamoyl)-3-formylindol-1-ylmethyl]-3-methoxybenzoate (1.2 g, 71%) as an ivory powder; mp 134-135°.

(g) A solution of t-butyl 4-[6-(N-cyclopentylmethylcarbamoyl-3-formylindol-1-ylmethyl]-3-methoxybenzoate (1.2 g) and methyl (triphenylphosphoranylidene)acetate (1.8 g) in dioxane (12 ml) was heated at reflux for 48 hours. The solvent was evaporated. The resultant residue was purified by flash chromatography on silica gel (600 ml), eluting with 3.7 ethyl acetate:hexane, to give t-butyl 4-[6-(N-cyclopentylmethylcarbamoyl)-3-(2-methoxycarbonyl vinyl)indol-1-ylmethyl]-3-methoxybenzoate (1.1 g, 84%) as a yellow solid; mp 163-164°.

(h) A solution of t-butyl 4-[6-(N-cyclopentylmethylcarbamoyl)-3-(2-methoxycarbonylvinyl)-indol-1-ylmethyl]-3-methoxybenzoate (1.11 g) in methanol (10 ml) was treated with 10% (w/w) palladium on carbon (0.28 g) and shaken under 3.45 bars of hydrogen for 24 hours. The catalyst was removed by filtration through diatomaceous earth, and the filtrate was evaporated to give t-butyl 4-[6-(N-cyclopentylmethylcarbamoyl)-3-(2-methoxycarbonylethyl)indol-1-ylmethyl]-3-methoxybenzoate (1.04 g, 94%) as a

grey foam, mp 58-60°.

(i) A solution of t-butyl 4-[6-(N-cyclopentylmethylcarbamoyl)-3-(2-methoxycarbonylethyl)indol-1-ylmethyl]-3-methoxybenzoate (1.04 g) in dioxane (6 ml) was treated with triethylamine (0.65 ml) and trimethylsilyl trifluoromethanesulphonate (0.8 ml). The solution was stirred for 24 hours and then was diluted with water to give a viscous oil. The liquids were decanted and the oil was triturated successively with water and hexane. The resultant solid was recrystallized from ethyl acetate/hexane to give 4-[6-(N-cyclopentylmethylcarbamoyl)-3-(2-methoxycarbonylethyl)indol-1-ylmethyl]-3-methoxybenzoic acid (0.3 g, 32%) as an ivory powder; mp 181-182°.

The starting bromoester of part (f) was prepared as follows:

(j) A solution of 3-methoxy-4-methylbenzoic acid (10.0 g), concentrated sulfuric acid (1 ml), and condensed isobutylene (200 ml) in methylene chloride (200 ml) was placed in a pressure vessel and stirred for 16 hours. The vessel was then opened to vent unreated isobutylene. The remaining liquid was poured into 10% (w/v) sodium hydroxide solution (150 ml) and extracted twice with ethyl acetate. The combined extracts were washed with brine, dried ($MgSO_4$), and evaporated. The residue was purified by flash chromatography on silica gel (700 ml), eluting with 1:9 ethyl acetate:hexane, to give t-butyl 3-methoxy-4-methylbenzoate (9.1 g, 70%) as a colorless oil; NMR (80 MHz, $CDCl_3$): 1.6[s, 9H, $C(CH_3)_3$], 2.27(s, 3H, $CH_3$), 3.86(s, 3H, $OCH_3$), 7.11(d, 1H), 7.49(m, 2H).

(k) A suspension of t-butyl 3-methoxy-4-methylbenzoate (8.92 g), N-bromosuccinimide (8.57 g), and benzoyl peroxide (0.1 g) in carbon tetrachloride (150 ml) was heated to reflux and irradiated with a sun lamp for 1 hour. After cooling to room temperature, the suspension was filtered; and the filtrate was evaporated. The residue was purified by flash chromatography on silica gel (700 ml), eluting with 5:95 ethyl acetate:hexane to give t-butyl 4-bromomethyl-3-methoxybenzoate (11.52 g, 95%) as a pale yellow oil; NMR (80 MHz, $CDCl_3$): 1.5[s, 9H, $C(CH_3)_3$], 3.9(s, 3H, $OCH_3$, 4.5(s, 2H, $CH_2Br$), 7.15(d, 1H), 7.4(m, 2H).

## Example 2

N-[4-[6-(N-Cyclopentylmethylcarbamoyl)-3-[2-(N-methylcarbamoyl)ethyl]indol-1-ylmethyl]-3-methoxybenzoyl]-2-methylbenzenesulphonamide

A solution of 4-[6-(N-cyclopentylmethylcarbamoyl)-3-[2-(N-methylcarbamoyl)ethyl]indol-1-ylmethyl]-3-methoxybenzoic acid (0.25 g) (prepared as described in Example 1), 4-(dimethylamino)pyridine (0.07 g), 1-(3-dimethylaminopropyl)-3-ethylcarbodiimide hydrochloride (0.12 g), and ortho-toluenesulphonamide (0.09 g) in methylene chloride (3.0 ml) was stirred under a nitrogen atmosphere for 24 hours. The mixture was diluted with methylene chloride, washed sequentially with 10% (v/v) hydrochloric acid, water, and brine, and evaporated. The resulting ivory solid was purified by flash chromatography on silica gel (10 ml), eluting with 1:9 methanol: chloroform, to give the title compound (0.06 g, 17%) as an ivory powder; mp 211-212°C.

Analysis calculated for:

$C_{35}H_{40}N_4O_6S.0.1\ H_2O$: C, 65.02; H, 6.21; N, 8.66

Found: C, 64.73; H, 6.29; N, 8.66

## Example 3

This Example illustrates the preparation of an intermediate of Formula III.

t-Butyl 4-[3-(2-cyanovinyl)-6-(N-cyclopentylmethyl-carbamoyl) indol-1-ylmethyl]-3-methoxybenzoate.

Using a similar procedure to that described in Example 1, part (g), but using cyanomethylenetriphenylphosphorane in place of methyl (triphenylphosphoranylidene)acetate, the title compound was obtained as a yellow solid (78%); partial NMR (80 MHz, $CDCl_3$): 3.41(dd, 2H, $CH_2N$), 3.93(s, 2.1H, $OCH_3$, E-isomer), 3.97-(s, 0.9H, $OCH_3$, Z-isomer), 5.20(d, 0.3H, CHCN, Z-isomer), 5.38(s, 1.4H, $ArCH_2$, E-isomer), 5.44(s, 0.6H, $ArCH_2$, Z-isomer), 5.74(d, 0.7H, CHCN, E-isomer), 6. 19(broad, 1H, NH).

## Example 4

4-[3(2-Cyanovinyl)-6-(N-cyclopentylmethylcarbamoyl)-indol-1-ylmethyl] -3-methoxybenzoic acid

Using a similar procedure to that described in Example 1, part (i), but starting from the compound of

Example 3, the title compound was obtained as a white powder (87%); mp 277-279°.
Analysis calculated for:
$C_{27}H_{27}N_3O_4$: C, 70.88; H, 5.95; N, 9.18
Found: C, 70.68; H, 6.02; N, 9.08

Example 5

N-[4-[3-(2-Cyanovinyl)-6-(N-cyclopentylmethylcarbamoyl)indol-1-ylmethyl]-3-methoxybenzoyl]2-methylbenzenesulfonamide

Using a similar procedure to that described in Example 2, but starting from the compound of Example 4, the title compound was obtained as a white solid (79%), mp 174-176° (d).
Analysis calculated for:
$C_{34}H_{34}N_4O_5S.0.5H_2O$: C, 65.89; H, 5.69; N. 9.04
Found: C, 65.62; H, 5.63; N, 9.00

Example 6

N-[4-[6-(N-Cyclopentylmethylcarbamoyl)-3-[2-(morpholinocarbonyl)ethyl]indol-1-ylmethyl]-3-methoxybenzoyl]benzenesulfonamide

Using a similar procedure to the described in Example 2, but starting from 4-[6-(N-oyclopentylmethylcarbamoyl)-3-[2-(morpholinocarbonyl)ethyl]-indol-1-ylmethyl]-3-methoxybenzoic acid and phenylsulfonamide, the title compound was obtained as a white solid (69%); mp 244-245°.
Analysis calculated for:
$C_{37}H_{42}N_4O_7S$: C, 64.70; H, 6.16; N, 8.16
Found: C, 64.65; H, 6.18; N, 7.96

The starting indole was obtained as follows: A solution of 4-[6-(N-cyclopentylmethylcarbamoyl)-3-(2-methoxycarbonylethyl)indol-1-ylmethyl)]-3-meth oxybenzoic acid (0.68g) (prepared as described in Example 1, parts (a) through (i) and 4-(dimethylamino)pyridine (0.17 g) in morpholine (4 ml) was heated at 80° for 48 hours under a nitrogen atmosphere. The reaction was diluted with water and acidified with 10% (v/v) hyrochloric acid. The resultant precipitate was collected by filtration and washed with water. The product was purified by recrystallization from ethyl acetate to give 4-[6-(N-cyclopentylmethylcarbamoyl)-3-[2-(morpholinocarbonyl)-ethyl]indol-1-ylmethyl]-3-methoxybenzoic acid as a white powder (0.31 g, 41%); partial NMR (80 MHz, $CDCl_3$): 1.2-1.8(m, 9H, cyclopentyl), 2.70(dd, 2H, $CH_2NH$), 3.15(t, 2H, $COCH_2$), 3.2-3.7(m, 8H, morpholino), 3.92(s, 3H, $OCH_3$), 5.35(s, 2H, $ArCH_2$), 6.58(t, 1H, NH), 7.17(d, 1H), 7.9(s, 1H, $H^7$-indole).

Example 7

N-[4-[6-(N-Cyclopentylmethylcarbamoyl)-3-[2-(morpholinocarbonyl]ethyl]indol-1-ylmethyl]-3-methoxybenzoyl]-2-methylbenzenesulphonamide

Using a similar procedure to the one described in Example 2, but starting from 4-[6-(N-cyclopentylmethylcarbamoyl)-3-[2-(morpholinocarbonyl)-ethyl]indol-1-ylmethyl]-3-methoxybenzoic acid, prepared as described in Example 6, the title compound was obtained as a white powder (28%); mp 159-161°.
Analysis calculated for:
$C_{38}H_{44}N_4SO_7$: C, 65.12; H, 6.33; N, 7.99
Found: C, 64.75; H, 6.34; N, 7.88

Example 8

N-[4-[6-(N-Cyclopentylmethylcarbamoyl)-3-[2-(pyrrolidinocarbonyl]ethyl]indol-1-ylmethyl]-3-methoxybenzoyl]-2-methylbenzenesulphonamide

Using a similar procedure to the one described in Example 2, but starting from 4-[6-(N-cyclopentylmethylcarbamoyl)-3-[2-(pyrrolidinocarbonyl)ethyl]indol-1-ylmethyl]-3-methoxybenzoic acid, the title compound was obtained as a white powder (48%); mp 190-191°.
Analysis calculated for:

$C_{38}H_{44}N_4SO_6$: C, 66.64; H, 6.48; N, 8.18
Found: C, 66.44; H, 6.46; N, 8.02

The starting indole was prepared as follows:

A solution of 4-[6-(N-cyclopentylmethylcarbamoyl)-3-(2-methoxycarbonylethyl)indol-1-ylmethyl]-3-methoxybenzoic acid (0.84 g) (prepared as described in Example 1, parts (a) through (i)) and 4-(dimethylamino)pyridine (0.21 g) in pyrrolidine (5 ml) was heated at 80° for 48 hours under a nitrogen atmosphere. The reaction was diluted with water and acidified with 10% (v/v) hydrochloric acid. The resultant precipitate was collected by filtration and washed with water to give 4-[6-(N-cyclopentylmethylcarbamoyl)-3-[2-(pyrrolidinocarbonyl)ethyl]indol-1-ylmethyl]-3-methoxybenzoic acid as a white powder (0.77 g, 85%); partial NMR (80 MHz, DMSO-$d_6$): 1.1-2.0 (m, 12H), 2.1(m, 1H, $NHCH_2C\underline{H}$), 3.9(s, 3H, $OCH_3$), 6.7(d, 1H, Ar), 7.3(s, 1H, $H^2$-indole), 8.3(t, 1H, NH).

Example 9

N-[4-[6-(N-Cyclopentylmethylcarbamoyl)-3-[2-(N,N-dimethylcarbamoyl)]propyl]indol-1-ylmethyl]-3-methoxybenzoyl]-2-methylbenzenesulphonamide.

Using a similar procedure to that described in Example 2, but starting from 4-[6-(N-cyclopentylmethylcarbamoyl)-3-[2-(N,N-dimethylcarbamoyl)propyl]-indol-1-ylmethyl]-3-methoxybenzoic acid, the title compound was obtained as a yellow powder (56%); mp 140-143°.

Analysis calculated for:
$C_{37}H_{44}N_4O_6S.0.5\ H_2O$: C, 65.18; H, 6.65; N, 8.21
Found: C, 65.15; H, 6.65; N, 8.11

The starting material was prepared as follows:

(a) A solution of t-butyl 4-[6-(N-cyclopentylmethylcarbamoyl)-3-formylindol-1-ylmethyl]-3-methoxybenzoate (Example 1, part (f)) (2.8 g) and (carbethoxyethylidene)triphenylphosphorane (4.6 g) in dioxane (29 ml) was heated at reflux for 18 hours. The solvent was evaporated. The resultant residue was purified by flash chromatography on silica gel (192 ml), eluting with 1:4 ethyl acetate:hexane to give t-butyl 4-[6-(N-cyclopentylmethylcarbamoyl)-3-(2-ethoxycarbonyl-1-propenyl)indol-1-ylmethyl]-3-methoxybenzoate (3.3 g, 100%) as a light yellow solid; mp 118-120°; NMR (80 MHz, $CDCl_3$): 2.15(d, 3H, $CC\underline{H}_3$), 3.40(dd, 2H, $NHCH_2$), 5.42(s, 2H, $NCH_2$), 6.22(br t, 1H, NH), 6.78(d, 1H, Ar).

(b) A solution of t-butyl 4-[6-(N-cyclopentylmethylcarbamoyl)-3-(2-ethoxycarbonyl-1-propen yl)indol-1-ylmethyl]-3-methoxybenzoate (3.3 g) in methanol (30 ml) was treated with 10% (w/w) palladium on carbon (0.8 g) and shaken under 3.45 bars of hydrogen for 18 hours. The catalyst was removed by filtration through diatomaceous earth, and the filtrate was evaporated to give t-butyl 4-[6-(N-cyclopentylmethylcarbamoyl)-3-(2-ethoxycarbonylpropyl)-indol-1-ylmethyl]-3-methoxybenzoate (3.3 g, 100%) as a colorless oil; partial NMR (80 MHz, $CDCl_3$): 3.40(t, 2H, $NHCH_2$), 3.94(s, 3H, $OCH_3$), 4.10(q, 2H, $OCH_2$), 5.33(s, 2H, $NCH_2$), 6.14(br t, 1H, NH), 6.63(d, 1H, Ar), 7.04(s, 1H, $H^2$-indole), 7.85(br s, 1H, $H^7$-indole).

(c) A solution of t-butyl 4-[6-(N-cyclopentylmethylcarbamoyl)-3-(2-ethoxycarbonylpropyl)-indol-1-ylmethyl]-3-methoxybenzoate (0.75 g) in a mixture of tetrahydrofuran (3.5 ml), methanol (3.5 ml), and water (1.3 ml) was treated with lithium hydroxide monohydrate (0.33 g). The mixture was stirred at 30° for 6 hours and then concentrated to remove the organic solvents. The residue was dissolved in water, and the solution was acidified with 10% (v/v) hydrochloric acid. The precipitate which formed was collected by filtration and dried to give t-butyl 4-[6-(N-cyclopentylmethylcarbamoyl)-3-(2-carboxypropyl)-indol-1-ylmethyl]-3-methoxybenzoate (0.68 g, 95%) as a white powder; mp 195-197°; partial NMR (80 MHz, $CDCl_3$): 2.55-3.24(m, 3H, $CH_2CHCH_3$), 3.38(t, 2H, $NHCH_2$), 3.91(s, 3H, $OCH_3$), 5.24(s, 2H, $NCH_2$), 6.17(br t, 1H, NH), 6.61(d, 1H, Ar), 7.04(s, 1H, $H^2$-indole), 7.85(br s, 1H, $H^7$-indole).

(d) A solution of t-butyl 4-[6-(N-cyclopentylmethylcarbamoyl)-3-(2-carboxypropyl)indol-1-ylmethyl]-3-methoxybenzoate (0.96 g) and 1,1'-carbonyldiimidazole (0.42 g) in methylene chloride (9 ml) was heated at reflux under nitrogen for one hour. The solution was transferred under nitrogen to a pressure vessel containing condensed dimethylamine (60 ml). After the vessel was sealed, the mixture was heated at 60° for ninety hours. The amine was then allowed to evaporate. The residue was diluted with water, acidified with 50% (v/v) hydrochloric acid, and extracted with methylene chloride. The organic extract was washed with water and brine, dried ($MgSO_4$), and evaporated. The residue was purified by flash chromatography on silica gel (160 ml), eluting with 1:9 methanol:chloroform to give t-butyl 4[6-(N-cyclopentylmethylcarbamoyl)-3-[2-(N,N-dimethylcarbamoyl)propyl]indol-1-ylmethyl]-3-methoxybenzoate (0.53g, 53%) as a white crystalline foam; mp 73-75°; partial NMR (250 MHz; $CDCl_3$): 1.18(d, 3H, $CHC\underline{H}_3$), 1.56(s, 9H, $C(CH_3)_3$), 2.07-2.28(m, 1H, $CH_3C\underline{H}$), 2.75(s, 3H, $NCH_3$), 2.83(s, 3H, $NCH_3$), 2.96-

3.21(m, 2H, $CH_2CHCH_3$), 3.40 (dd, 2H, $NHCH_2$), 6.17(br t, 1H, NH), 6.60(d, 1H, Ar), 7.06(s, 1H, $H^2$-indole), 7.86(br s, 1H, $H^7$-indole).

(e) A solution of t-butyl 4-[6-(N-cyclopentylmethylcarbamoyl)-3-[2-(N,N-dimethylcarbamoyl)-propyl]indol-1-ylmethyl]-3-methoxybenzoate (0.53 g) in dioxane (3 ml) was treated with triethylamine (0.31 ml) and trimethylsilyl trifluoromethanesulfonate (0.38 ml). The solution was heated under nitrogen at reflux for thirty minutes, allowed to cool, and then diluted with water to give a precipitate which was collected by filtration and dried under vacuum to give 4-[6-(N-cyclopentylmethylcarbamoyl)-3-[2-(N,N-dimethylcarbamoyl)propyl]indol-1-ylmethyl]-3-methoxybenzoic acid (0.33 g, 66%) as a yellow powder; mp 120-122°; partial NMR (250 MHz, DMSO-$d_6$): 1.04(D, 3H, $CHCH_3$), 2.07-2.24(m, 1H, $CH_3CH$), 2.71(s, 3H, $NCH_3$), 2.80(s, 3H, $NCH_3$), 3.93(s, 3H, $OCH_3$), 5.42(s, 2H, $NCH_2$), 6.58(d, 1H, Ar), 7.30(s, 1H, $H^2$-indole), 7.40(d, 1H, $H^5$-indole), 7.90(br s, 1H, $H^7$-indole),8.33(br t, 1H, NH).

Example 10

4-[6-(N-Cyclopentylmethylcarbamoyl)-3-[2-(dimethylcarbamoyloxymethyl)indol-1-ylmethyl]-3-methoxybenzoic acid

Using a similar ester hydrolysis to the procedure described in Example 9, part (c), but starting from methyl 4-[6-(N-cyclopentylmethylcarbamoyl)-3-(dimethylcarbamoyloxymethyl)indazol-1-ylmethyl]-3-methoxybenzoate, the title compound may be obtained as a solid .

The starting indazole was made as follows:

(a) To a solution of boron trifluoride etherate (18 ml) in chloroform (450 ml, $Al_2O_3$ treated) at -15° was added a solution of 3-amino-4-methylbenzoic acid (15.1 g) in tetrahydrofuran (150 ml) over 15 minutes and the resulting mixture was then stirred for an additional 5 minutes. To this mixture was added t-butyl nitrite (14 ml), and the reaction was warmed to 5°. After stirring for 1 hour, potassium acetate (49 g) and 18-crown-6 (2.65 g) were added. The reaction mixture was allowed to warm to room temperature and stirred for 72 hours. The reaction mixture was evaporated, and 3:7 acetone:ethyl acetate (500 ml) and 1N hydrochloric acid (150 ml) were added. After stirring for 2 hours, brine (150 ml) was added to the mixture and the mixture filtered. The aqueous filtrate was extracted with 3:7 acetone:ethyl acetate (2 x 100 ml). The combined organic extract was dried ($MgSO_4$) and evaporated. The resulting residue was dissolved in hot acetic acid (250 ml) and 250 ml saturated ethereal HCl and 250 ml ether were added sequentially. After cooling to room temperature, the precipitate was filtered and treated with 3:7 acetone:ethyl acetate (500 ml) and brine (100 ml) for 1 hour. After the phases were separated, the aqueous layer was extracted with ethyl acetate (100 ml). The combined organic extracts were washed with brine, dried ($MgSO_4$) and evaporated to afford 6-carboxyindazole as a brown solid (9.8 g, 57%), mp >250°.

(b) To a solution of 6-carboxyindazole (4.0 g) in acetic acid (140 ml) was added bromine (1.53 ml), and the mixture was stirred in the dark for 24 hours. After the addition of saturated sodium bisulfite (50 ml) and brine (100 ml), the mixture was extracted with ethyl acetate. The organic layer was washed with brine, dried ($MgSO_4$) and evaporated. The resulting solid was powdered and vacuum dried to afford 3-bromo-6-carboxyindazole as a light brown solid (5.88 g, 99%), mp >250°.

(c) To a mixture of 3-bromo-6-carboxyindazole (5.84 g), cyclopentylmethylamine (3.48 ml), 1-(3-dimethylaminopropyl)-3-ethylcarbodiimide hydrochloride (3.25 g), methylene chloride (120 ml) and dimethylformamide (40 ml) was added 4-dimethylaminopyridine (5.10 g). After stirring for 48 hours, the reaction mixture was added to 450 ml ethyl acetate; washed with 1N hydrochloric acid, 0.5 M sodium carbonate and brine; dried ($MgSO_4$); and evaporated. The residue was flash chromatographed over 175g silica gel, eluting sequentially with methylene chloride (350 ml), 15:85 ethyl acetate:methylene chloride (350 ml), and 1:3 ethyl acetate:methylene chloride to afford 3-brom-6-(N-cyclopentylmethylcarbamoyl)-indazole as a light red solid (5.6 g, 72%), mp 119-125°.

(d) A mixture of 60% sodium hydride dispersion (13 mg) was washed with petroleum ether, and dimethylformamide (0.75 ml) was added. This mixture was cooled to 0° and a solution of 3-bromo-6-(N-cyclopentylmethylcarbamoyl)indazole (107 mg) in dimethylformamide (0.75 ml) was added. After stirring for 30 minutes, methyl 4-bromomethyl-3-methoxy benzoate (95 mg) was added. After 15 minutes of stirring at 0°, the mixture was allowed to warm to room temperature. The reaction was stirred for 1.5 hours and ethyl acetate (40 ml) was added. The solution was washed with brine, water and brine; dried ($MgSO_4$); and evaporated. The residue was flash chromatographed over 10 g of silica gel, eluting with 5:95 ethyl acetate:methylene chloride to afford methyl 4-[3-bromo-6-(N-cyclopentylmethylcarbamoyl)-indazol-1-yl-methyl]-3-methoxybenzoate as a white solid (136 mg, 82%), mp 161.0-162.5°;

Analysis calculated for:

$C_{24}H_{27}BrN_3O_4$: C, 57.49; H, 5.42; N, 8.38
Found: C, 57.53; H, 5.29; N, 8.28

(e) To a solution of zinc bromide (6.75 g) (dried at 180° at 67 Pa for 2 hours) in tetrahydrofuran (90 ml) at 0° was added 1M vinylmagnesium bromide (25 ml) in ether. The reaction was allowed to stir at room temperature for 15 minutes before dichloro[1,1')-bis(diphenylphosphino)ferrocene]palladium (II) (183 mg) was added to the reaction, and the reaction was heated at 45° for 120 hours. Additional portions of the palladium reagent (each 183 mg) were added after 48 and 84 hours of stirring. The reaction mixture was cooled to 0°, and 1N hydrochloric acid (50 ml) and ethyl acetate (250 ml) were added. The mixture was stirred for 15 minutes before it was filtered through diatomaceous earth with ethyl acetate washings. The organic layer was washed with water and brine, dried ($MgSO_4$), and evaporated. Flash chromatography of the residue over 200 g silica gel, eluting with methylene chloride (800 ml), 2.5:97.5 ethyl acetate:methylene chloride (500 ml), and 5:95 ethyl acetate:methylene chloride afforded a solid. Recrystallization from methylene chloride and petroleum ether yielded methyl 4-[6-(N-cyclopentylmethylcarbamoyl)-3-vinylindazol-1-ylmethyl]-3-methoxybenzoate as a colorless solid (944 mg, 42%), mp 138-140°, resolidifies and remelts at 168.0-170.0°; mass spectrum (chemical ionization) 448 (M+H).

(f) A solution of methyl 4-[6-(N-cyclopentylmethylcarbamoyl)-3-vinylindazol-1-ylmethyl]-3-methoxybenzoate (700 mg) in 1:1 methylene chloride: methanol (30 ml) at -78° was ozonized for 25 minutes. Excess ozone was removed by passing oxygen through the reaction for 5 minutes. Sodium borohydride (100 mg) was added and the mixture allowed to warm to room temperature. After stirring for 2 hours, the mixture was cooled to 0°, quenched with 1N hydrochloric acid, and extracted with ethyl acetate (100 ml). The organic layer was washed with 1N hydrochloric acid, brine and water. The organic layer containing insoluble precipitate was concentrated to about 50 ml and filtered. The solid was powdered and vacuum dried over potassium hydroxide for 18 hours to afford methyl 4-[6-(N-cyclopentylmethylcarbamoyl)-3-hydroxymethylindazol-1-ylmethyl]-3-methoxybenzoate as a colorless solid (569 mg, 81%), mp 187.0-187.5°; mass spectrum (chemical ionization) 452 (M+H).

(g) To a suspension of 60% sodium hydride dispersion (13 mg) (petroleum ether washed) in tetrahydrofuran (1 ml) at 0° was added a solution of methyl 4-[6-(N-cyclopentylmethylcarbamoyl)-3-hydroxymethylindazol-1-ylmethyl]-3-methoxybenzoate (151 mg) in 7:1 dimethylformamide:tetrahydrofuran (8 ml). After stirring for 45 minutes, dimethylcarbamoyl chloride (0.033 ml) was added. The reaction was stirred for 15 minutes and was allowed to warm to room temperature. After 3 hours, 1N hydrochloric acid was added and the mixture was extracted with ethyl acetate. The organic layer was washed with brine and dried ($MgSO_4$). Evaporation and flash chromatography over 15 g silica gel, eluting with 2:8 ethyl acetate:methylene chloride (50 ml) and 4:6 ethyl acetate:methylene chloride, afforded methyl 4-[6-(N-cyclopentylmethylcarbamoyl)-3-(dimethylcarbamoyloxymethyl)indazol-1-ylmethyl]-3-methoxybenzoate as a colorless solid (40 mg, 23%); mass spectrum (chemical ionization) 523 (M+H).

NOTE: Still further Examples of compounds of the invention are provided by the benzoic acid starting materials for formula I ($M = CO_2H$) described in connection with Examples 6, 8 and 9.

Example 11

The following illustrates representative pharmaceutical dosages forms which may be used for the therapeutic or prophylactic administration of an acidic compound of formula I (that is, M is an acidic group as defined hereinbefore) or of a pharmaceutically acceptable salt thereof (hereinafter referred to as 'Compound X'):

(i) Tablet 1

| Tablet 1 | mg/tablet |
|---|---|
| 'Compound X' | 100 |
| Lactose | 182.75 |
| Croscarmellose Sodium | 12.0 |
| Starch | 2.25 |
| Magnesium stearate | 3.0 |

(ii) Tablet 2

| | mg/tablet |
|---|---|
| 'Compound X' | 20 |
| Microcrystalline cellulose | 420 |
| Polyvinylpyrrolidone | 14.0 |
| Starch | 43.0 |
| Magnesium stearate | 3.0 |

(iii) Capsule

| | mg/capsule |
|---|---|
| 'Compound X' | 10 |
| Lactose | 488.5 |
| Magnesium stearate | 1.5 |

(iv) Injection 1

| | (10 mg/ml) |
|---|---|
| 'Compound X' (free acid form) | 1.0% w/v |
| Sodium phosphate | 3.6% w/v |
| 0.1M Sodium hydroxide solution | 15.0% w/v |
| Water for injection. . . .to 100% | |

(v) Injection 2 (buffered to pH 6)

| | (1 mg/ml) |
|---|---|
| 'Compound X' (free acid form) | 0.1% w/v |
| Sodium phosphate | 2.26% w/v |
| Citric acid | 0.38% w/v |
| Polyethylene glycol 400 | 3.5% w/v |
| Water for injection. . . .to 100% | |

(vi) Aerosol

| | mg/ml |
|---|---|
| 'Compound X' | 0.2 |
| Sorbitan trioleate | 0.27 |
| Trichlorofluoromethane | 70.0 |
| Dichlorodifluoromethane | 280.0 |
| Dichlorotetrafluoroethane | 1094.0 |

It will be appreciated that the above pharmaceutical compositions may be varied according to well known pharmaceutical techniques to accommodate differing amounts and types of active ingredient 'Compound X'. The aerosol (vi) may be used in conjunction with a standard, metered does aerosol dispenser.

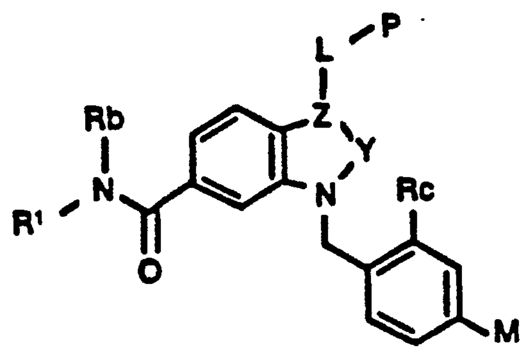

I

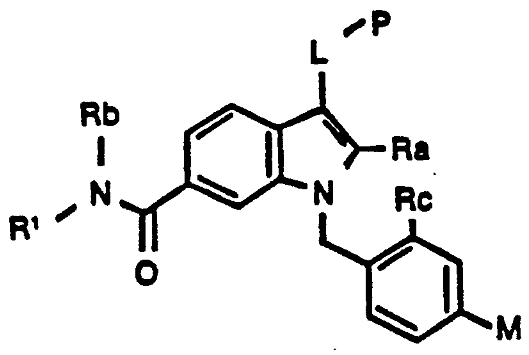

Ia

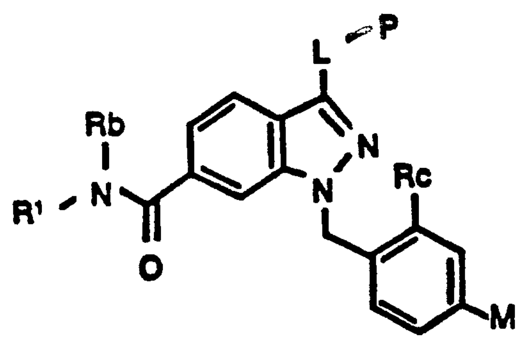

Ib

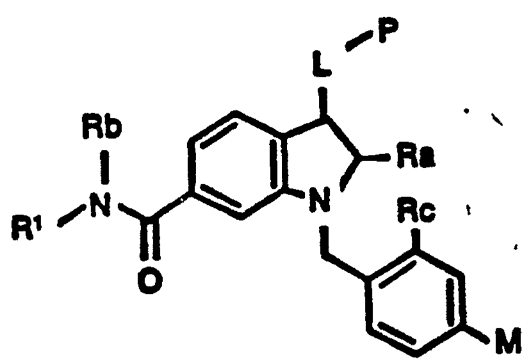

Ic

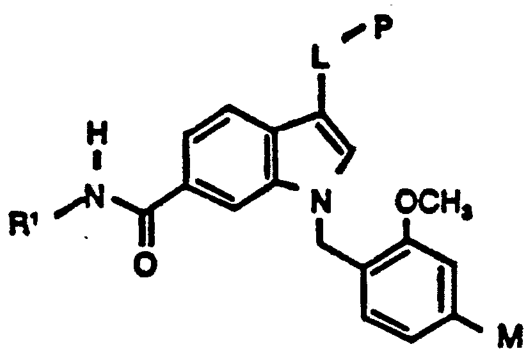

IIa

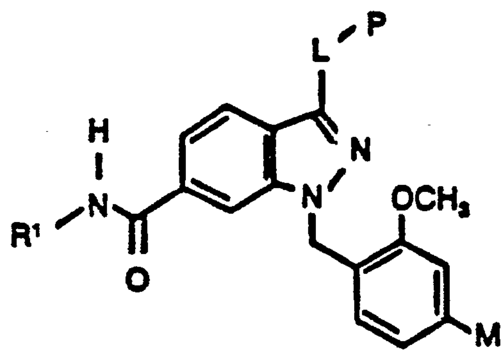

IIb

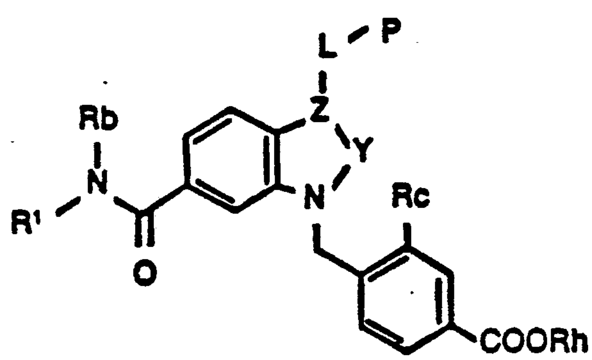

III

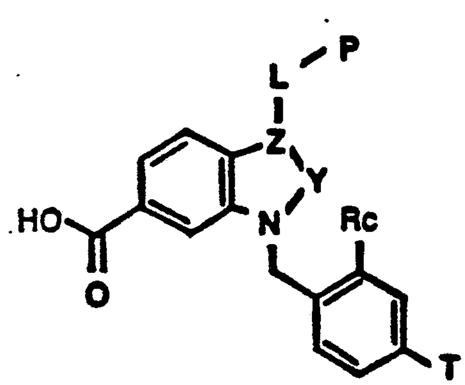

IV

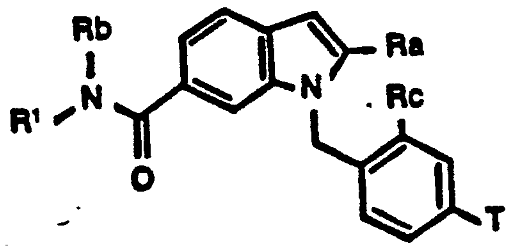

V

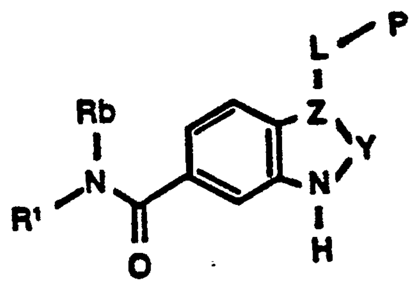

VI

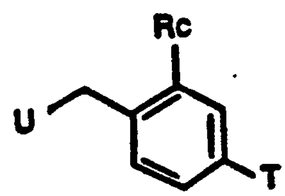

VII

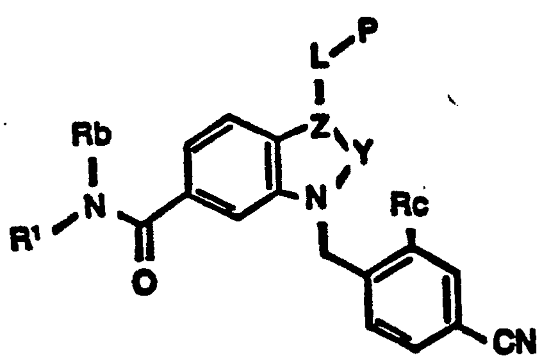

VIII

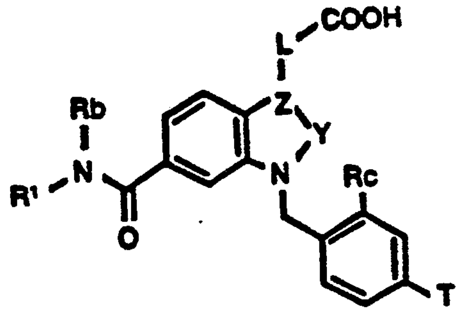

IX

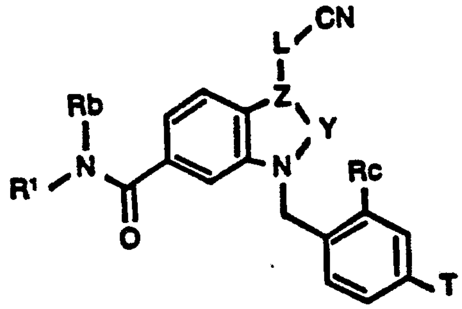

X

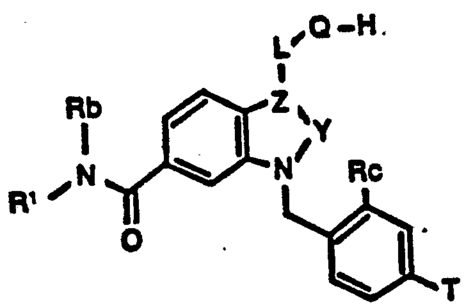

XI

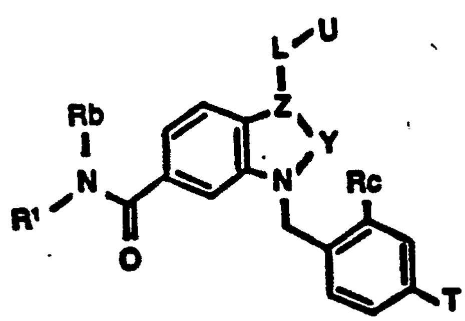

XII

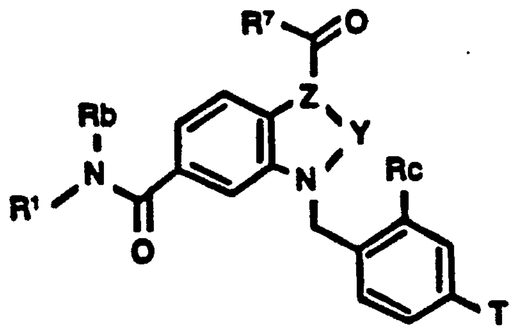

XIII

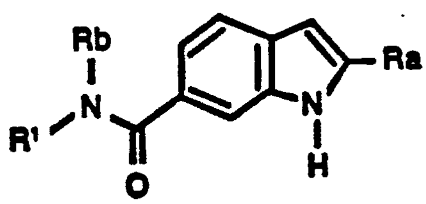

XIV

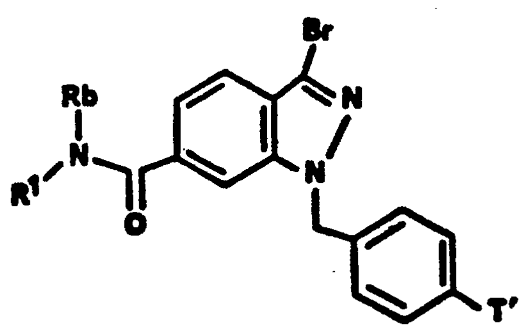

XV

## Claims

**Claims for the following Contracting States : BE, CH, DE, FR, GB, IT, LI, LU, NL, SE**

**1.** A compound of the formula I

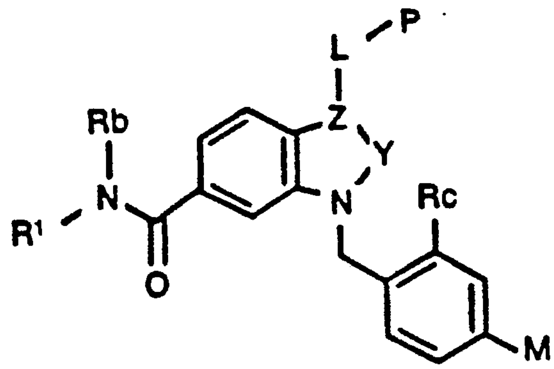

wherein the group -Y-Z< is selected from a group consisting of

(a) -C(Ra) = C<

(b) -N = C< and

(c) -CH(Ra)-CH<

in which "<" indicates two separate bonds;

and wherein

Ra is hydrogen or (1-4C)alkyl;

Rb is hydrogen;

$R^1$ is (2-10C)alkyl optionally containing 1 or more fluorine substituents; or $R^1$ is phenyl-(1-6C)-alkyl in which the (1-6C)alkyl moiety may optionally bear a fluoro or (1-4C)alkoxy substituent and in which the phenyl moiety may optionally bear a substituent selected from a group consisting of halogeno, (1-4C)alkyl, (1-4C)alkoxy and trifluoromethyl; or $R^1$ is (3-8C)cycloalkyl or (3-8C)cycloalkyl-(1-6C)alkyl, the cyclic moiety of any of which optionally may contain one unsaturated linkage and may optionally bear 1 or 2 (1-4C)alkyl substituents;

L is a (1-10C)alkylene link, optionally containing one double or triple bond;

P is a polar group selected from a group consisting of cyano, 1H-tetrazol-5-yl, carbamoyl of formula $CONR^2R^3$, ureido of formula $NR^4CONR^2R^3$, carbamoyloxy of formula $OCONR^2R^3$, a carbamate of formula $NR^4COOR^5$, and an (optionally oxidized) thio group of formula $S(0)_nR^5$ in which

$R^2$ is selected from a group consisting of hydrogen, (1-6C)alkyl, and phenyl, the phenyl moiety of which may optionally bear 1 or 2 substituents selected from a group consisting of halogeno, (1-4C)alkyl, (1-4C)alkoxy and trifluoromethyl, and

$R^3$ and $R^4$ are independently selected from a group consisting of hydrogen and (1-6C)alkyl;

or $R^2$ and $R^3$ together with the adjacent nitrogen form a pyrrolidine, piperidine, morpholine, piperazine or N-(1-6C)alkylpiperazine ring, and $R^4$ is hydrogen or (1-6C)alkyl;

$R^5$ is chosen from a group consisting of (1-4C)alkyl and phenyl, the phenyl moiety of which may optionally bear 1 or 2 substituents selected from a group consisting of halogeno, (1-4C)alkyl, (1-4C)-alkoxy and trifluoromethyl; and

n is the integer 0, 1 or 2;

Rc is selected from a group consisting of hydrogen and (1-4C) alkoxy; and

M is an acidic group selected from a group consisting of carboxy, an acylsulphonamide residue of formula $-CO.NH.SO_2R^6$ and 1H-tetrazol-5-yl in which

$R^6$ is selected from a group consisting of (1-6C)alkyl, (3-8C)cycloalkyl, (6-12C)aryl, heteroaryl selected from furyl, thienyl and pyridyl, and (6-12C)aryl-(1-4C)alkyl, in which any of the aromatic or heteroaromatic moieties may bear 1 or 2 substituents selected from a group consisting of halogeno, (1-4C)alkyl, (1-4C)alkoxy, and trifluoromethyl;

or a pharmaceutically acceptable salt thereof.

**2.** A compound as claimed in Claim 1 selected from a group consisting of the indole derivatives of formula IIa

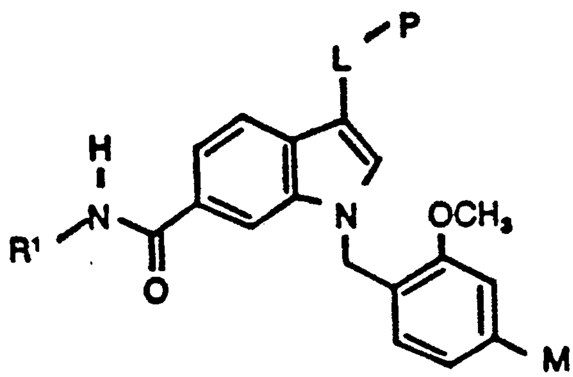

and indazole derivatives of formula IIb

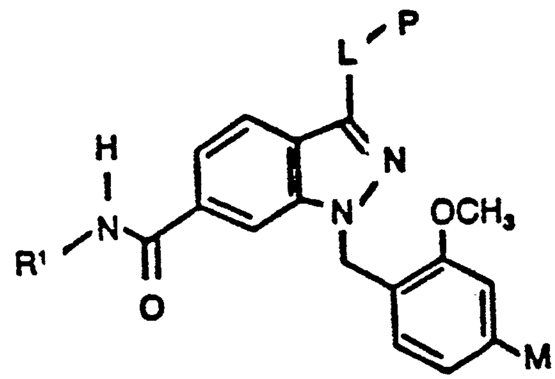

wherein $R^1$, L, M and P have any of the meanings defined in Claim 1.

**3.** A compound as claimed in Claim 1 or 2 wherein

Ra is selected from a group consisting of hydrogen, methyl, ethyl, propyl and isopropyl;

Rb is hydrogen;

$R^1$ is selected from a group consisting of (a) ethyl, propyl, isopropyl, butyl, isobutyl, sec-butyl, t-butyl, pentyl, 1-ethylpropyl, hexyl, heptyl, 1-ethylpentyl or nonyl, each of which may contain 1 or more fluorine substituents; (b) benzyl, 1-phenylethyl, 2-phenylpropyl, 3-phenylpropyl, 1-methyl-1-phenylethyl, 1-phenylbutyl and 1-phenylpentyl, each of which may be substituted on the alkyl moiety by methoxy or ethoxy and each of which may be substituted on the phenyl moiety by a member selected from a group consisting of fluoro, chloro, and bromo, methyl, ethyl, methoxy and ethoxy; (c) cyclopropyl, cyclobutyl, cyclopentyl, cyclohexyl, cycloheptyl, cyclobutylmethyl, cyclopentylmethyl, cyclohexylmethyl, 1-cyclopentylethyl, 2-cyclopentylethyl, 1-cyclopentylpropyl, 1-cyclohexylpropyl, 1-cyclopentylbutyl and 1-cyclohexylbutyl, wherein each of the cyclic moieties may optionally be substituted by methyl, ethyl, or isopropyl; and (d) cyclopentenyl, cyclohexenyl, cyclopentenylmethyl, 1-cyclohexen-4-ylmethyl, and 1-(cyclohexenyl)butyl wherein each of the cyclic moieties may optionally be substituted by methyl, ethyl or isopropyl;

L is selected from a group consisting of (a) methylene, ethylene, ethylidene, trimethylene, tetramethylene, 1,1-dimethylethylene, 2,2-dimethylethylene, pentamethylene, and hexamethylene; (b) vinylene, 1-propenylene, 2-propenylene, 1-butenylene, 2-butenylene, 1,2-dimethylvinylene, 1,1-dimethyl-2-propenylene and 3,3-dimethyl-1-propenylene; and (c) ethynylene, 1-propynylene, 2-propynylene, 2-butynylene, 1,1-dimethyl-2-propynylene and 3,3-dimethyl-1-propynylene;

$R^2$ is selected from a group consisting of hydrogen, methyl, ethyl, propyl, isopropyl, butyl, t-butyl, pentyl, and phenyl, the phenyl moiety of which may optionally bear 1 or 2 substituents selected from a group consisting of fluoro, chloro, bromo, methyl, ethyl, methoxy, ethoxy, and trifluoromethyl, and $R^3$ and $R^4$ are independently selected from a group consisting of hydrogen, methyl, ethyl, propyl, isopropyl, butyl, t-butyl and pentyl;

or

$R^2$ and $R^3$ together with the adjacent nitrogen form a pyrrolidine, piperidine, piperazine, or N-alkyl

piperazine ring in which N-alkyl group is selected from a group consisting of methyl, ethyl, propyl, isopropyl, butyl, t-butyl and pentyl, and $R^4$ is selected from a group consisting of hydrogen, methyl, ethyl, propyl, isopropyl, butyl, t-butyl and pentyl; and

$R^5$ is selected from a group consisting of methyl, ethyl, propyl, isopropyl and phenyl, the phenyl group of which may optionally bear 1 or 2 substituents independently selected from the group defined above for a phenyl group of $R^2$;

Rc is methoxy or ethoxy; and

$R^6$ is methyl, ethyl, propyl, isopropyl, butyl, cyclopentyl, cyclohexyl, phenyl, 1-naphthyl, 2-naphthyl, furyl, thienyl, pyridyl, benzyl, 1-naphthylmethyl or 2-naphthylmethyl wherein each aromatic or heteroaromatic moiety may be optionally substituted as defined above for a phenyl moiety in $R^1$.

**4.** A compound as claimed in Claim 1 or 2 wherein

$R^1$ is ethyl, propyl, isopropyl, butyl, isobutyl, sec-butyl, t-butyl, pentyl, 1-ethylpropyl, hexyl, heptyl, 1-ethylpentyl, nonyl, benzyl, 4-chlorobenzyl, 4-trifluoromethylbenzyl, 4-methylbenzyl, 1-phenylethyl, 2-phenylethyl, 1-methyl-1-phenylethyl, 1-phenylpropyl, 1-phenylpentyl, cyclopentyl, cyclohexyl, cyclobutylmethyl, cyclopentylmethyl, cyclohexylmethyl, 2-cyclopentylethyl, 1-cyclopentylbutyl, 1-cyclohexylpropyl, 1-cyclohexyl-butyl, cyclopentenylmethyl, and 1-cyclohexen-4-yl-methyl;

$R^2$, $R^3$ and $R^4$ are selected from a group consisting of (a) $R^2$ is hydrogen, methyl, ethyl, propyl, isopropyl, t-butyl, phenyl, 2-methylphenyl or 4-chlorophenyl and $R^3$ and $R^4$ are independently selected to be hydrogen, methyl or ethyl; and (b) $R^2$ and $R^3$ together with the adjacent nitrogen form a piperidine, morpholine, or N-methylpiperazine ring and $R^4$ is hydrogen, methyl or ethyl;

$R^5$ is methyl, ethyl, propyl, isopropyl, phenyl, 2-methylphenyl or 4-chlorophenyl;

$R^6$ is methyl, isopropyl, butyl, cyclopentyl, phenyl, 4-chlorophenyl, 4-methylphenyl, 2-chlorophenyl, 2-methylphenyl, 2-methoxyphenyl, naphthyl, thien-2-yl or 6-chloropyrid-3-yl;

Ra is hydrogen or methyl;

Rb is hydrogen; and

Rc is hydrogen or methoxy.

**5.** A compound as claimed in any one of Claims 1-4 wherein

$R^1$ is butyl, 1-ethylpentyl, 1-phenylpropyl, cyclopentyl, or cyclopentylmethyl; M is carboxy or a group of the formula $-CO.NH.SO_2R^6$ wherein $R^6$ is phenyl or 2-methylphenyl; Ra is hydrogen; and Rc is methoxy.

**6.** A compound of the formula IIa or IIb

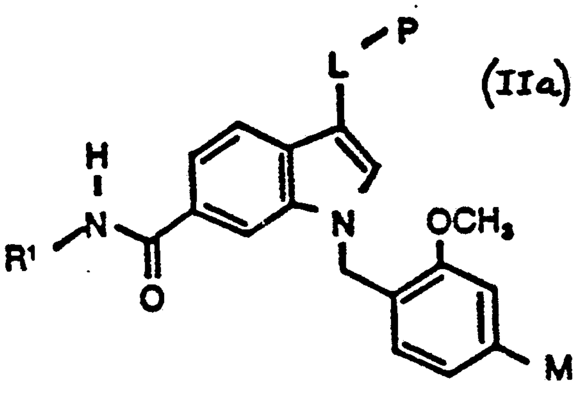
(IIa)

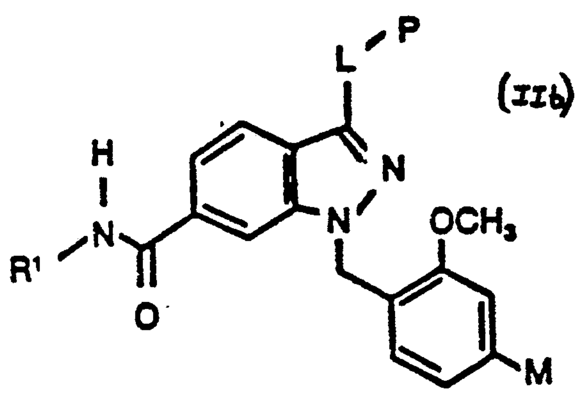
(IIb)

wherein

$R^1$ is (3-6C)cycloalkyl-(1-4C)alkyl; L is methylene, ethylene or vinylene; M is carboxy or an acylsulphonamide residue of the formula $-CO.NH.SO_2R^6$ in which $R^6$ is phenyl optionally bearing a substituent selected from halogeno, (1-4C)alkyl, (1-4C)alkoxy and trifluoromethyl; and P is cyano, carbamoyl of formula $CONR^2R^3$ or carbamoyloxy of formula $OCONR^2R^3$ wherein $R^2$ and $R^3$ are independently hydrogen or methyl, or $R^2$ and $R^3$ together with the adjacent nitrogen form a pyrrolidine or morpholine ring; or a pharmaceutically acceptable salt thereof.

**7.** A compound as claimed in any one of claims 1-6, provided that $R^2$ and $R^3$ together with the adjacent

nitrogen do not form a pyrrolidine ring.

8. A salt as claimed in any preceding claim wherein said salt is derived from a base forming a physiologically acceptable cation.

9. A process for the manufacture of a compound of the formula I or a pharmaceutically acceptable salt thereof as claimed in any one of Claims 1-8 which is characterised by:

(A) for a compound of formula I wherein M is a carboxy group, decomposing an ester of formula III:

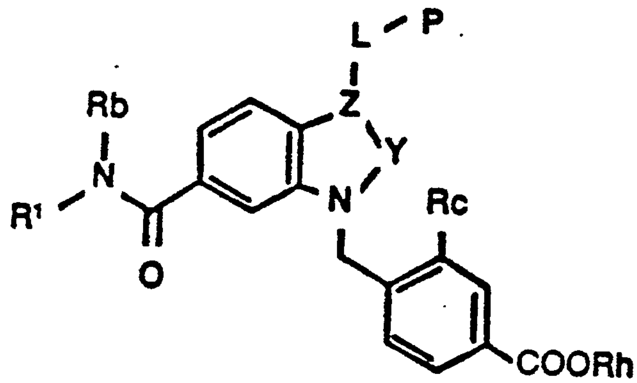

(B) acylating an amine of formula $R^1NHRb$ with a carboxylic acid of formula IV

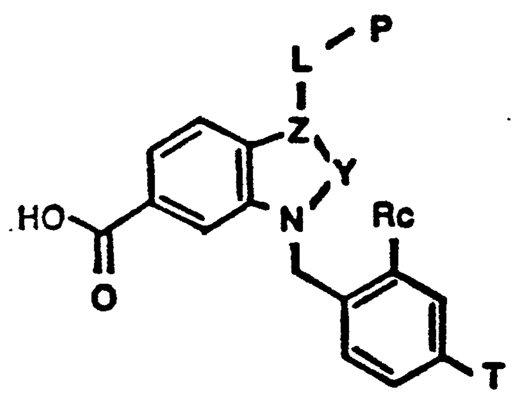

but wherein T is chosen from the values defined for M, or with a reactive derivative thereof;

(C) for a compound of formula I wherein -Y-Z< has the value (a) defined in Claim 1, reacting an indole of formula V,

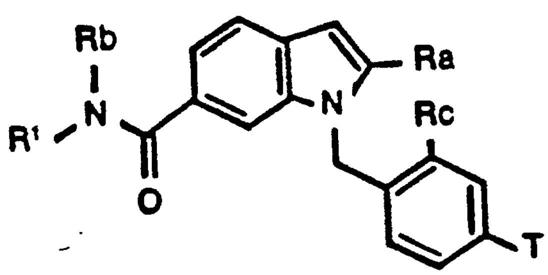

but wherein T is chosen from the values defined for M, with a reagent of formula U.L.P;

(D) reacting an imino compound of formula VI,

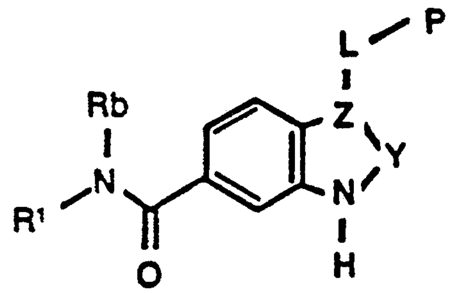

with an alkylating agent of formula VII,

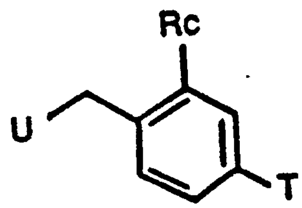

but wherein T is chosen from the values defined for M;

(E) for a compound of formula I wherein M is a 1H-tetrazol-5-yl radical, reacting a cyano derivative of formula VIII

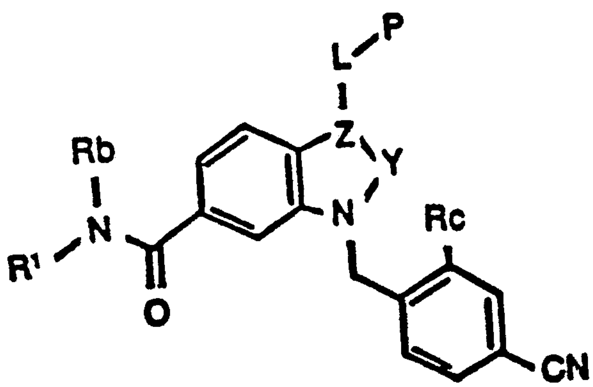

with an azide;

(F) for a compound of formula I wherein M is a group of formula $CO.NH.SO_2.R^6$, reacting a compound of formula I wherein M is carboxy with a sulphonamide derivative of formula $R^6.SO_2.NH_2$ in the presence of a dehydrating agent or reacting a reactive derivative of an acid of formula I with a sulphonamide, or a salt thereof, of formula $R^6.SO_2NH_2$; formula $R^6.SO_2.NH_2$;

(G) for a compound of formula I wherein -Y-Z< has the value (c) defined in Claim I, catalytic hydrogenation of an indole of formula I wherein -Y-Z-< has the value (a) defined in Claim 1;

(H) reduction of the double bond of a compound of formula I in which L contains one double bond to provide the corresponding compound of formula I in which L contains no double bond;

(I) for a compound of formula I in which P is a carbamoyl group of the formula $CONR^2R^3$, acylation of an amine of the formula $HNR^2R^3$ with a corresponding acid, or a reactive derivative thereof, of formula IX:

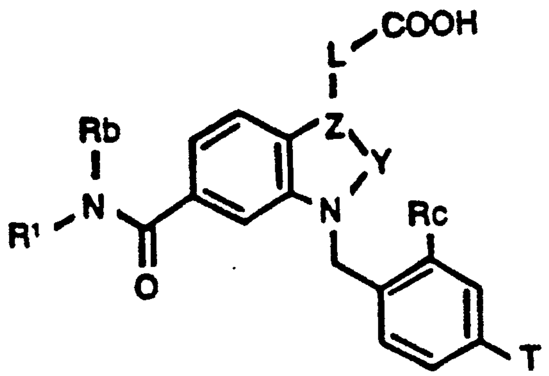

but wherein T is chosen from the values defined for M;

(J) for a compound of formula I in which P is a 1H-tetrasol-5-yl group, reacting a nitrile of formula X,

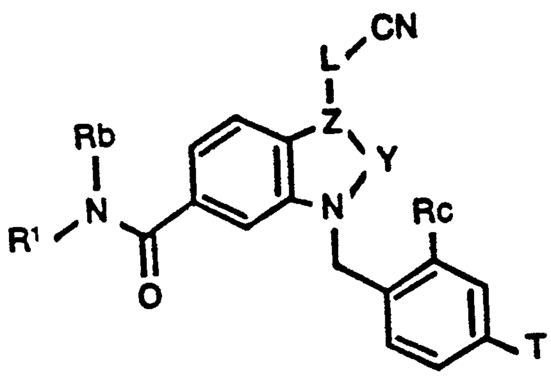

but wherein T is chosen from the values defined for M, with an azide using similar procedures to those described above in part (E);

(K) for a compound of formula I in which P has the value $NR^4CONR^2R^3$, $OCONR^2R^3$, $NR^4COOR^5$, acylating a compound of formula XI,

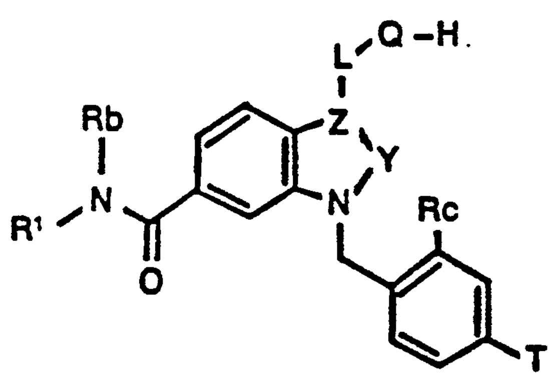

but wherein T is chosen from the values defined for M and wherein QH has the value $NR^4H$ or OH, with an acylating agent;

(L) for a compound of formula I in which P has the value $S(O)_nR^5$ and n is 0, reacting a compound of formula XII,

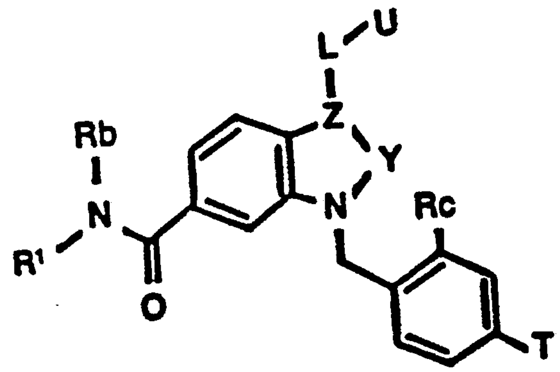

but wherein T is chosen from the values' defined for M, with a mercaptan of formula $R^5SH$;

(M) for a compound of formula I in which P has the value $S(O)_nR^5$ and n is 1 or 2, oxidizing the corresponding compound I in which n is 0 or n is 1; and

(N) for a compound of formula I wherein L contains a double bond adjacent to Z, reacting an aldehyde or ketone of formula XIII,

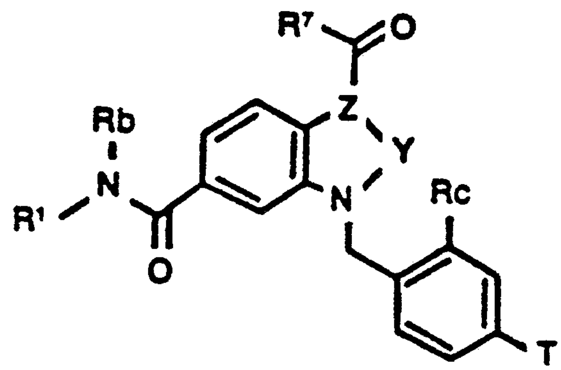

but wherein T is chosen from the values defined for M and wherein $R^7$ is selected from hydrogen and (1-4C)alkyl, with a zwitterionic or carbanionic reagent;

wherein -Y-Z-<, Ra-Rc, $R^1$-$R^6$, L, M, P and n have the meanings defined in any one of Claims 1-7; Rh is selected from phenyl, benzyl, and (1-6C)alkyl optionally bearing an acetoxy, (1-4C)alkoxy or (1-4C)alkylthio substituent; U is chloro, bromo, iodo, methanesulphonyloxy or p-toluenesulphonyloxy; and Ha1 is chloro, bromo or iodo; and

whereafter, when a pharmaceutically acceptable salt is required, reacting an acidic form of the compound of formula I with a base affording a physiologically acceptable cation.

**10.** A compound of the formula III

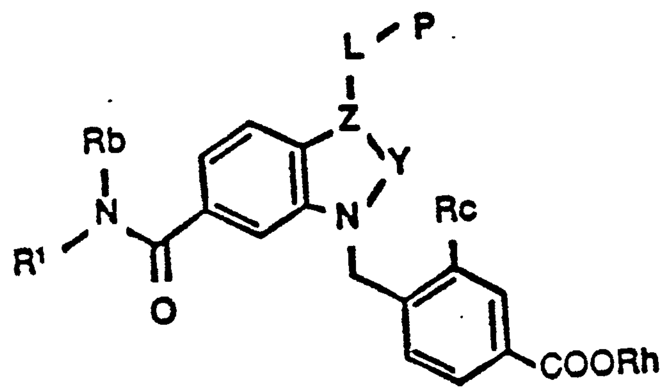

wherein Rh has any of the meanings defined in Claim 9 and $R^1$ , Rb, Rc, L, P and the group -Y-Z< have any of the meanings defined in Claims 1-7.

**11.** A pharmaceutical composition comprising a compound of the formula I or a pharmaceutically acceptable salt thereof as claimed in any one of Claims 1-8 and a pharmaceutically acceptable diluent or carrier.

**Claim for the following Contracting States : AT, ES**

**1.** A process for producing a compound of formula I

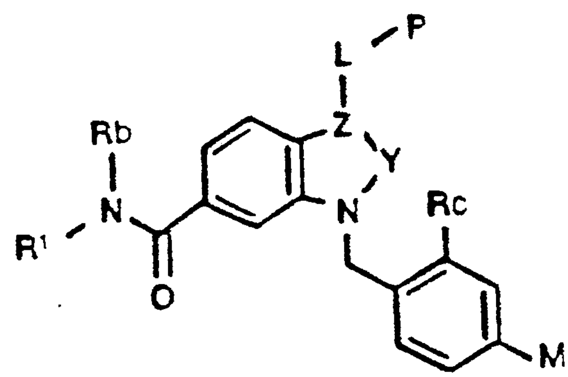

wherein the group -Y-Z< is selected from a group consisting of

(a) -C(Ra) = C<

(b) -N = C< and

(c) -CH(Ra)-CH<

in which "<" indicates two separate bonds;

and wherein

Ra is hydrogen or (1-4C)alkyl;

Rb is hydrogen;

$R^1$ is (2-10C)alkyl optionally containing 1 or more fluorine substituents; or $R^1$ is phenyl-(1-6C)alkyl in which the (1-6C)alkyl moiety may optionally bear a fluoro or (1-4C)alkoxy substituent and in which the phenyl moiety may optionally bear a substituent selected from a group consisting of halogeno, (1-4C)alkyl, (1-4C)alkoxy and trifluoromethyl; or $R^1$ is (3-8C)cycloalkyl or (3-8C)cycloalkyl-(1-6C)alkyl, the cyclic moiety of any of which optionally may contain one unsaturated linkage and may optionally bear 1 or 2 (1-4C)alkyl substituents;

L is a (1-10C)alkylene link, optionally containing one double or triple bond;

P is a polar group selected from a group consisting of cyano, 1H-tetrazol-5-yl, carbamoyl of formula $CONR^2R^3$, ureido of formula $NR^4CONR^2R^3$, carbamoyloxy of formula $OCONR^2R^3$, a carbamate of formula $NR^4COOR^5$, and an (optionally oxidized) thio group of formula $S(O)_nR^5$ in which

$R^2$ is selected from a group consisting of hydrogen, (1-6C)alkyl, and phenyl, the phenyl moiety of which may optionally bear 1 or 2 substituents selected from a group consisting of halogeno, (1-4C)alkyl,

(1-4C)alkoxy and trifluoromethyl, and

$R^3$ and $R^4$ are independently selected from a group consisting of hydrogen and (1-6C)alkyl;

or $R^2$ and $R^3$ together with the adjacent nitrogen form a pyrrolidine, piperidine, morpholine, piperazine or N-(1-6C)alkylpiperazine ring, and $R^4$ is hydrogen or (1-6C)alkyl;

$R^5$ is chosen from a group consisting of (1-4C)alkyl and phenyl, the phenyl moiety of which may optionally bear 1 or 2 substituents selected from a group consisting of halogeno, (1-4C)alkyl, (1-4C)-alkoxy and trifluoromethyl; and

n is the integer 0, 1 or 2;

Rc is selected from a group consisting of hydrogen and (1-4C) alkoxy; and

M is an acidic group selected from a group consisting of carboxy, an acylsulphonamide residue of formula $-CO.NH.SO_2R^6$ and 1H-tetrazol-5-yl in which

$R^6$ is selected from a group consisting of (1-6C)alkyl, (3-8C)cycloalkyl, (6-12C)aryl, heteroaryl selected from furyl, thienyl and pyridyl, and (6-12C)aryl(1-4C)alkyl, in which any of the aromatic or heteroaromatic moieties may bear 1 or 2 substituents selected from a group consisting of halogeno, (1-4C)alkyl, (1-4C)alkoxy, and trifluoromethyl;

or a pharmaceutically acceptable salt thereof, which method is selected from

(A) for a compound of formula I wherein M is a carboxy group, decomposing an ester of formula III:

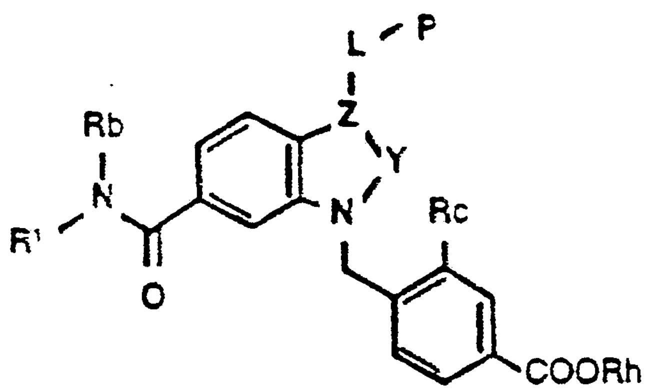

(B) acylating an amine of formula $R^1NHRb$ with a carboxylic acid of formula IV

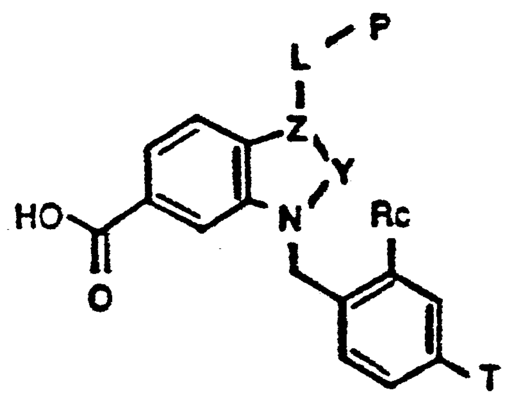

but wherein T is chosen from the values defined for M, or with a reactive derivative thereof;

(C) for a compound of formula I wherein -Y-Z< has the value (a) defined above, reacting an indole of formula V,

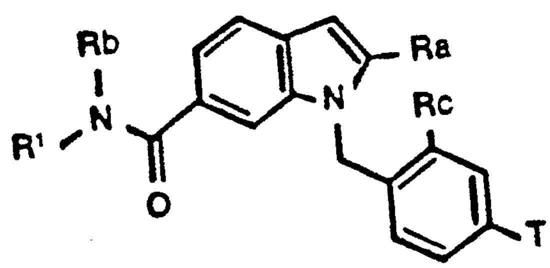

but wherein T is chosen from the values defined for M, with a reagent of formula U.L.P;
(D) reacting an imino compound of formula VI,

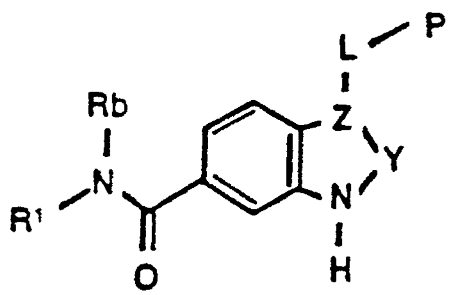

with an alkylating agent of formula VII,

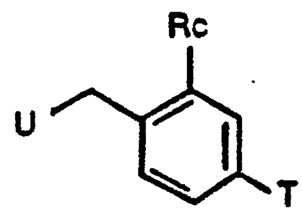

but wherein T is chosen from the values defined for M;
(E) for a compound of formula I wherein M is a 1H-tetrazol-5-yl radical, reacting a cyano derivative of formula VIII

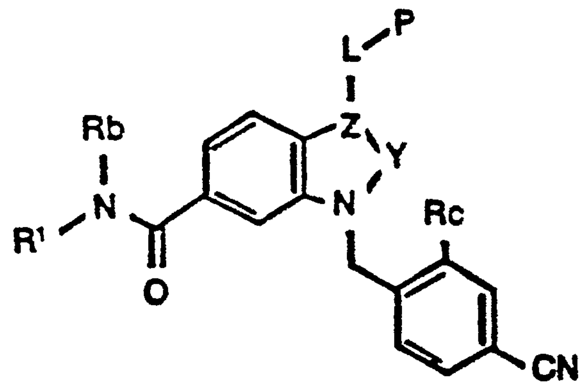

with an azide;
(F) for a compound of formula I wherein M is a group of formula $CO.NH.SO_2.R^6$, reacting a compound of formula I wherein M is carboxy with a sulphonamide derivative of formula $R^6.SO_2.NH_2$ in the presence of a dehydrating agent or reacting a reactive derivative of an acid of formula I with a sulphonamide, or a salt thereof, of formula $R^6.SO_2.NH_2$;

(G) for a compound of formula I wherein -Y-Z< has the value (c) defined above, catalytic hydrogenation of an indole of formula I wherein -Y-Z-< has the value (a) defined above;
(H) reduction of the double bond of a compound of formula I in which L contains one double bond to provide the corresponding compound of formula I in which L contains no double bond;
(I) for a compound of formula I in which P is a carbamoyl group of the formula $CONR^2R^3$, acylation of an amine of the formula $HNR^2R^3$ with a corresponding acid, or a reactive derivative thereof, of formula IX:

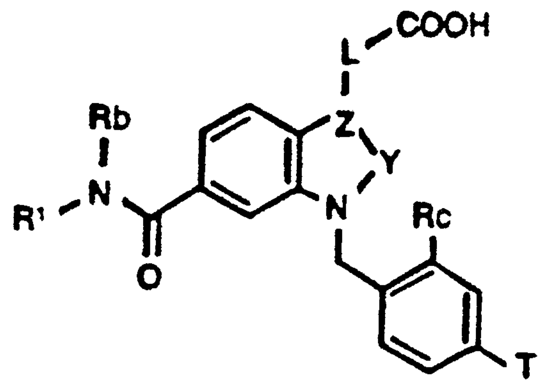

but wherein T is chosen from the values defined for M;
(J) for a compound of formula I in which P is a 1H-tetrazol-5-yl group, reacting a nitrile of formula X,

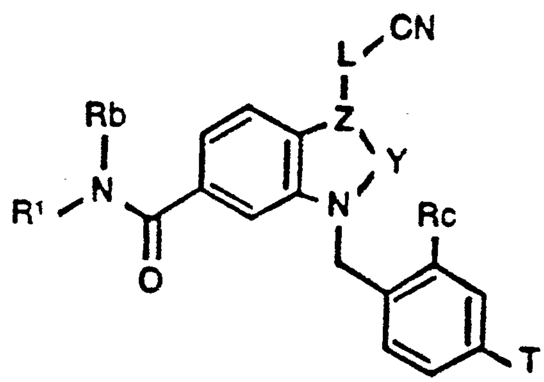

but wherein T is chosen from the values defined for M, with an azide using similar procedures to those described above in part (E);
(K) for a compound of formula I in which P has the value $NR^4CONR^2R^3$, $OCONR^2R^3$, $NR^4COOR^5$, acylating a compound of formula XI,

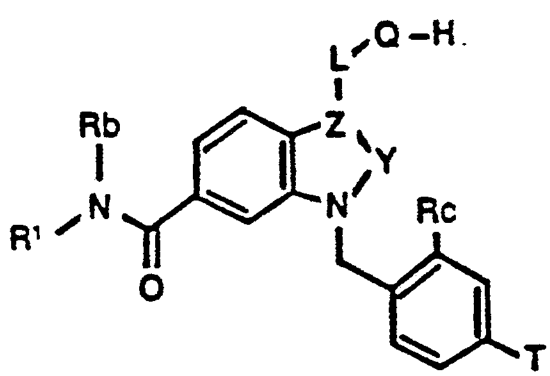

but wherein T is chosen from the values defined for M and wherein QH has the value $NR^4H$ or OH, with an acylating agent;

(L) for a compound of formula I in which P has the value $S(O)_nR^5$ and n is 0, reacting a compound of formula XII,

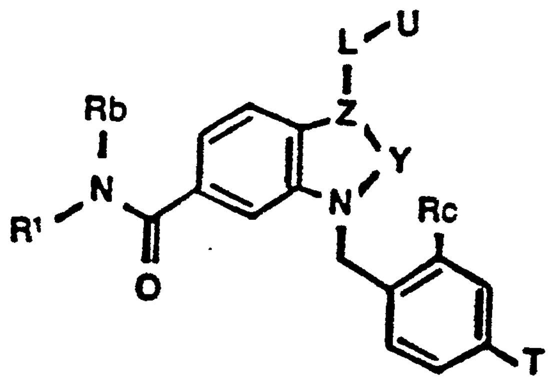

but wherein T is chosen from the values defined for M, with a mercaptan of formula $R^5SH$;

(M) for a compound of formula I in which P has the value $S(O)_nR^5$ and n is 1 or 2, oxidizing the corresponding compound I in which n is 0 or n is 1; and

(N) for a compound of formula I wherein L contains a double bond adjacent to Z, reacting an aldehyde or ketone of formula XIII,

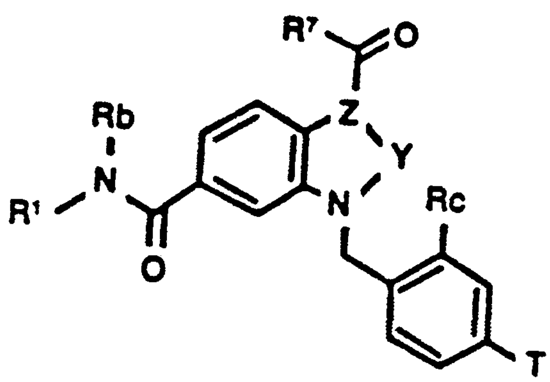

but wherein T is chosen from the values defined for M and wherein $R^7$ is selected from hydrogen and (1-4C)alkyl, with a zwitterionic or carbanionic reagent;

wherein -Y-Z-<, Ra-Rc, $R^1$-$R^6$, L, M, P and n have the meanings defined above; Rh is selected from phenyl, benzyl, and (1-6C)alkyl optionally bearing an acetoxy, (1-4C)-alkoxy or (1-4C)alkylthio substituent; U is chloro, bromo, iodo, methanesulphonyloxy or p-toluenesulphonyloxy; and Ha1 is chloro, bromo or iodo; and

whereafter, when a pharmaceutically acceptable salt is required, reacting an acidic form of the compound of formula I with a base affording a physiologically acceptable cation.

**Claims for the following Contracting State : GR**

1. A process for producing a compound of formula I

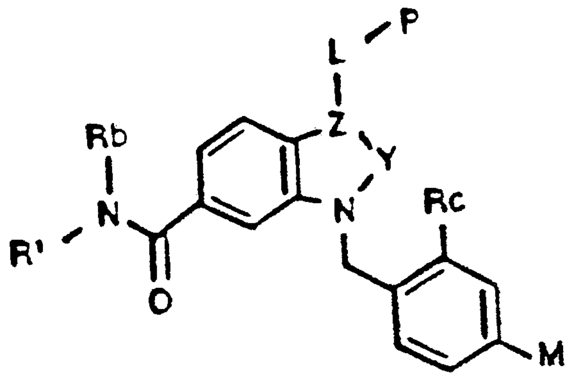

wherein the group -Y-Z< is selected from a group consisting of

(a) -C(Ra) = C<

(b) -N = C< and

(c) -CH(Ra)-CH<

in which "<" indicates two separate bonds;

and wherein

Ra is hydrogen or (1-4C)alkyl;

Rb is hydrogen;

$R^1$ is (2-10C)alkyl optionally containing 1 or more fluorine substituents; or $R^1$ is phenyl-(1-6C)-alkyl in which the (1-6C)alkyl moiety may optionally bear a fluoro or (1-4C)alkoxy substituent and in which the phenyl moiety may optionally bear a substituent selected from a group consisting of halogeno, (1-4C)alkyl, (1-4C)alkoxy and trifluoromethyl; or $R^1$ is (3-8C)cycloalkyl or (3-8C)cycloalkyl-(1-6C)alkyl, the cyclic moiety of any of which optionally may contain one unsaturated linkage and may optionally bear 1 or 2 (1-4C)alkyl substituents;

L is a (1-10C)alkylene link, optionally containing one double or triple bond;

P is a polar group selected from a group consisting of cyano, 1H-tetrazol-5-yl, carbamoyl of formula $CONR^2R^3$, ureido of formula $NR^4CONR^2R^3$, carbamoyloxy of formula $OCONR^2R^3$, a carbamate of formula $NR^4COOR^5$, and an (optionally oxidized) thio group of formula $S(O)_nR^5$ in which

$R^2$ is selected from a group consisting of hydrogen, (1-6C)alkyl, and phenyl, the phenyl moiety of which may optionally bear 1 or 2 substituents selected from a group consisting of halogeno, (1-4C)alkyl, (1-4C)alkoxy and trifluoromethyl, and

$R^3$ and $R^4$ are independently selected from a group consisting of hydrogen and (1-6C)alkyl;

or $R^2$ and $R^3$ together with the adjacent nitrogen form a pyrrolidine, piperidine, morpholine, piperazine or N-(1-6C)alkylpiperazine ring, and $R^4$ is hydrogen or (1-6C)alkyl;

$R^5$ is chosen from a group consisting of (1-4C)alkyl and phenyl, the phenyl moiety of which may optionally bear 1 or 2 substituents selected from a group consisting of halogeno, (1-4C)alkyl, (1-4C)-alkoxy and trifluoromethyl; and

n is the integer 0, 1 or 2;

Rc is selected from a group consisting of hydrogen and (1-4C) alkoxy; and

M is an acidic group selected from a group consisting of carboxy, an acylsulphonamide residue of formula $-CO.NH.SO_2R^6$ and 1H-tetrazol-5-yl in which

$R^6$ is selected from a group consisting of (1-6C)alkyl, (3-8C)cycloalkyl, (6-12C)aryl, heteroaryl selected from furyl, thienyl and pyridyl, and (6-12C)aryl-(1-4C)alkyl, in which any of the aromatic or heteroaromatic moieties may bear 1 or 2 substituents selected from a group consisting of halogeno, (1-4C)alkyl, (1-4C)alkoxy, and trifluoromethyl;

or a pharmaceutically acceptable salt thereof, which method is selected from

(A) for a compound of formula I wherein M is a carboxy group, decomposing an ester of formula III:

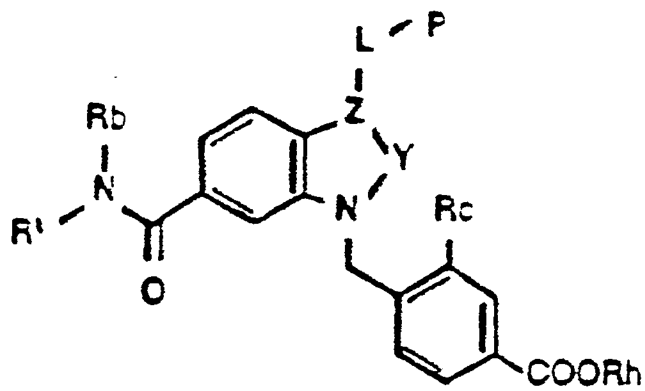

(B) acylating an amine of formula $R^1NHRb$ with a carboxylic acid of formula IV

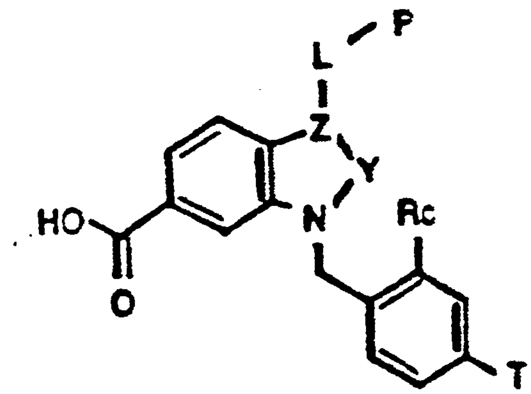

but wherein T is chosen from the values defined for M, or with a reactive derivative thereof;

(C) for a compound of formula I wherein -Y-Z< has the value (a) defined above, reacting an indole of formula V,

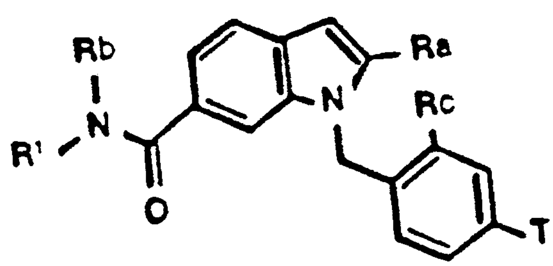

but wherein T is chosen from the values defined for M, with a reagent of formula U.L.P;

(D) reacting an imino compound of formula VI,

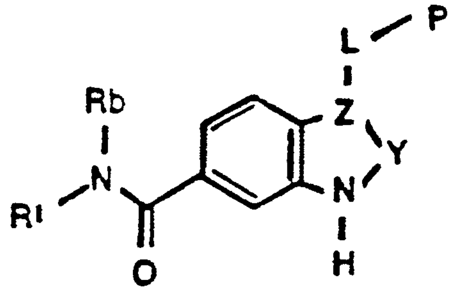

with an alkylating agent of formula VII,

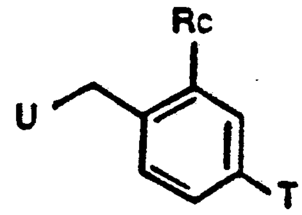

but wherein T is chosen from the values defined for M;

(E) for a compound of formula I wherein M is a 1H-tetrazol-5-yl radical, reacting a cyano derivative of formula VIII

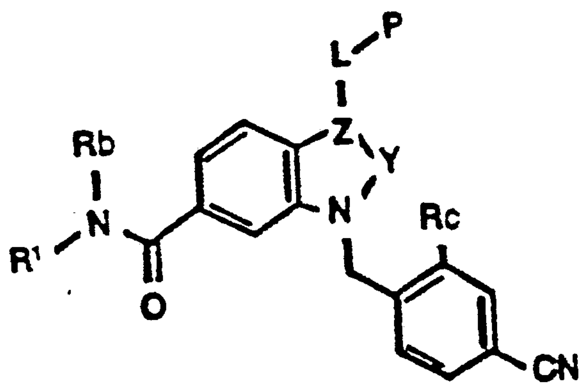

with an azide;

(F) for a compound of formula I wherein M is a group of formula $CO.NH.SO_2.R^6$, reacting a compound of formula I wherein M is carboxy with a sulphonamide derivative of formula $R^6.SO_2.NH_2$ in the presence of a dehydrating agent or reacting a reactive derivative of an acid of formula I with a sulphonamide, or a salt thereof, of formula $R^6.SO_2.NH_2$;

(G) for a compound of formula I wherein -Y-Z< has the value (c) defined above, catalytic hydrogenation of an indole of formula I wherein -Y-Z-< has the value (a) defined above;

(H) reduction of the double bond of a compound of formula I in which L contains one double bond to provide the corresponding compound of formula I in which L contains no double bond;

(I) for a compound of formula I in which P is a carbamoyl group of the formula $CONR^2R^3$, acylation of an amine of the formula $HNR^2R^3$ with a corresponding acid, or a reactive derivative thereof, of formula IX:

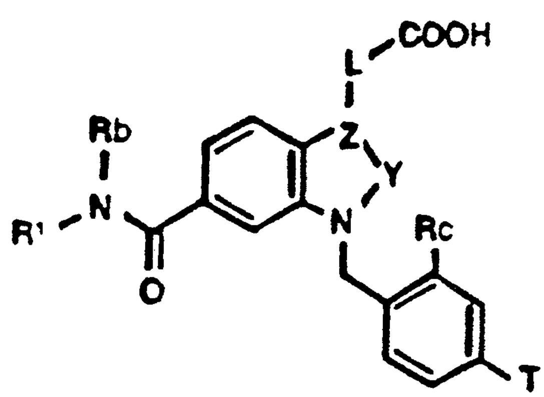

but wherein T is chosen from the values defined for M;

(J) for a compound of formula I in which P is a 1H-tetrazol-5-yl group, reacting a nitrile of formula X,

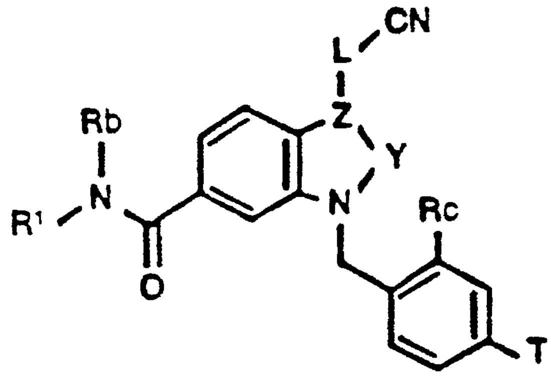

but wherein T is chosen from the values defined for M, with an azide using similar procedures to those described above in part (E);

(K) for a compound of formula I in which P has the value $NR^4CONR^2R^3$, $OCONR^2R^3$, $NR^4COOR^5$, acylating a compound of formula XI,

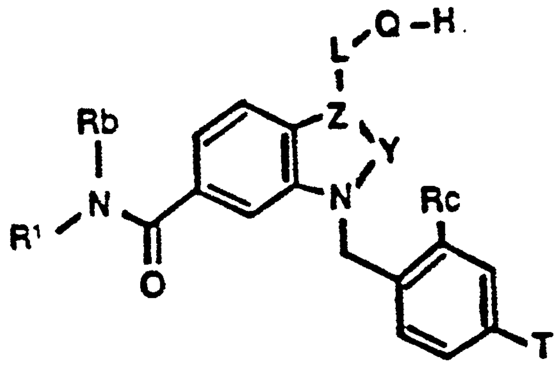

but wherein T is chosen from the values defined for M and wherein QH has the value $NR^4H$ or OH, with an acylating agent;

(L) for a compound of formula I in which P has the value $S(O)_nR^5$ and n is 0, reacting a compound of formula XII,

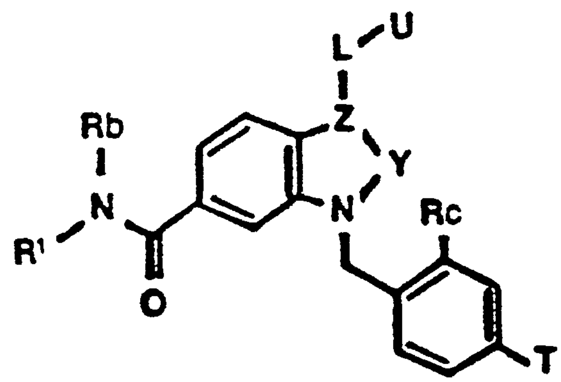

but wherein T is chosen from the values defined for M, with a mercaptan of formula $R^5SH$;

(M) for a compound of formula I in which P has the value $S(O)_nR^5$ and n is 1 or 2, oxidizing the corresponding compound I in which n is 0 or n is 1; and

(N) for a compound of formula I wherein L contains a double bond adjacent to Z, reacting an aldehyde or ketone of formula XIII,

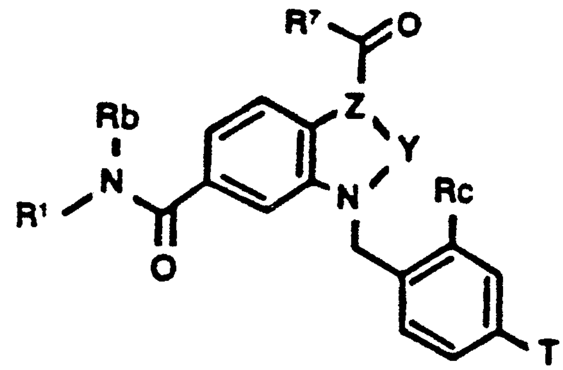

but wherein T is chosen from the values defined for M and wherein $R^7$ is selected from hydrogen and (1-4C)alkyl, with a zwitterionic or carbanionic reagent;

wherein -Y-Z-<, Ra-Rc, $R^1$-$R^6$, L, M, P and n have the meanings defined above; Rh is selected from phenyl, benzyl, and (1-6C)alkyl optionally bearing an acetory, (1-4C)-alkoxy or (1-4C)alkylthio substituent; U is chloro, bromo, iodo, methanesulphonyloxy or p-toluenesulphonyloxy; and Hal is chloro, bromo or iodo; and

whereafter, when a pharmaceutically acceptable salt is required, reacting an acidic form of the compound of formula I with a base affording a physiologically acceptable cation.

**2.** A compound of the formula III

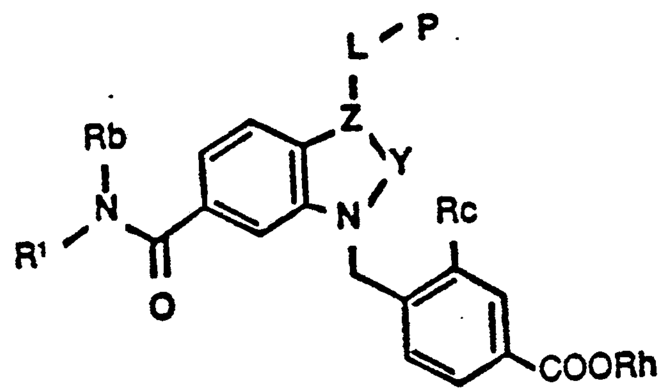

wherein Rh has any of the meanings defined in Claim 1 and $R^1$, Rb, Rc, L, P and the group -Y-Z< have any of the meanings defined in Claim 1.

**Revendications**

**Revendications pour les Etats contractants suivants : BE, CH, DE, FR, GB, IT, LI, LU, NL, SE**

**1.** Composé de formule I

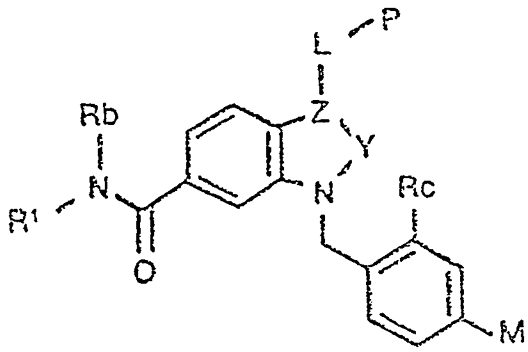

dans laquelle le groupe -Y-Z< est choisi dans le groupe constitué de

(a) -C(Ra) = C<

(b) -N = C< et

(c) -CH(Ra)-CH<

où "<" indique deux liaisons séparées ;

et dans laquelle

Ra est l'hydrogène ou un groupe alkyle en $C_1$ à $C_4$ ;

Rb est l'hydrogène ;

$R^1$ est un groupe alkyle en $C_2$ à $C_{10}$ contenant facultativement un ou plusieurs substituants fluor ; ou bien $R^1$ est un groupe phényl-(alkyle en $C_1$ à $C_6$) dont la partie alkyle en $C_1$ à $C_6$ peut porter facultativement un substituant fluoro ou alkoxy en $C_1$ à $C_4$ et dans lequel la partie phényle peut porter facultativement un substituant choisi dans le groupe constitué des substituants halogéno, alkyle en $C_1$ à $C_4$, alkoxy en $C_1$ à $C_4$ et trifluorométhyle ; ou bien $R^1$ est un groupe cycloalkyle en $C_3$ à $C_8$ ou (cycloalkyle en $C_3$ à $C_8$)(alkyle en $C_1$ à $C_6$), la portion cyclique de n'importe lequel d'entre eux pouvant contenir une liaison de non-saturation et pouvant facultativement porter 1 ou 2 substituants alkyle en $C_1$ à $C_4$ ;

L est une jonction alkylène en $C_1$ à $C_{10}$, contenant facultativement une liaison double ou triple ;

P est un groupe polaire choisi entre un groupe cyano, 1H-tétrazole-5-yle, carbamoyle de formule $CONR^2R^3$, uréido de formule $NR^4CONR^2R^3$, carbamoyloxy de formule $OCONR^2R^3$, un carbamate de formule $NR^4COOR^5$ et un groupe thio (facultativement oxydé) de formule $S(O)_nR^5$, où

$R^2$ est choisi dans un groupe comprenant l'hydrogène, un groupe alkyle en $C_1$ à $C_6$ et un groupe phényle, lequel peut porter facultativement 1 ou 2 substituants choisis dans le groupe comprenant des substituants halogéno, alkyle en $C_1$ à $C_4$, alkoxy en $C_1$ à $C_4$ et trifluorométhyle ; et

$R^3$ et $R^4$ sont choisis indépendamment dans un groupe comprenant l'hydrogène et un radical alkyle en $C_1$ à $C_6$ ;

ou bien $R^2$ et $R^3$ forment conjointement avec l'atome adjacent d'azote un noyau de pyrrolidine, de pipéridine, de morpholine, de pipérazine ou de N-(alkyle en $C_1$ à $C_6$)-pipérazine, et $R^4$ est l'hydrogène ou un groupe alkyle en $C_1$ à $C_6$ ;

$R^5$ est choisi dans un groupe comprenant un radical alkyle en $C_1$ à $C_4$ et le radical phényle, lequel peut porter facultativement 1 ou 2 substituants choisis dans le groupe des substituants halogéno, alkyle en $C_1$ à $C_4$, alkoxy en $C_1$ à $C_4$ et trifluorométhyle ; et

n est le nombre 0, 1 ou 2 ;

Rc est choisi dans un groupe comprenant l'hydrogène et un radical alkoxy en $C_1$ à $C_4$ ; et

M est un groupe acide choisi dans un groupe comprenant un reste carboxy, un résidu acylsulfonamide de formule $-CO.NH.SO_2R^6$ et un reste 1H-tétrazole-5-yle, où

$R^6$ est choisi dans un groupe comprenant des radicaux alkyle en $C_1$ à $C_6$, cycloalkyle en $C_3$ à $C_8$, aryle en $C_6$ à $C_{12}$, hétéroaryle choisi entre furyle, thiényle et pyridyle, et (aryle en $C_6$ à $C_{12}$)-(alkyle en $C_1$ à $C_4$), où l'un quelconque des groupements aromatiques ou hétéro-aromatiques peut porter 1 ou 2 substituants choisis dans le groupe des substituants halogéno, alkyle en $C_1$ à $C_4$, alkoxy en $C_1$ à $C_4$ et trifluorométhyle ;

ou un sel pharmaceutiquement acceptable de ce composé.

**2.** Composé suivant la revendication 1, choisi dans un groupe comprenant les dérivés indoliques de formule IIa

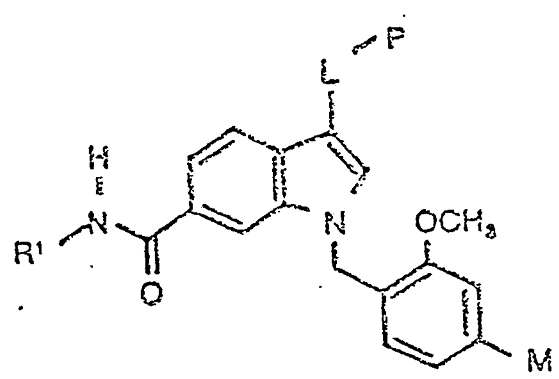

et les dérivés d'indazole de formule IIb

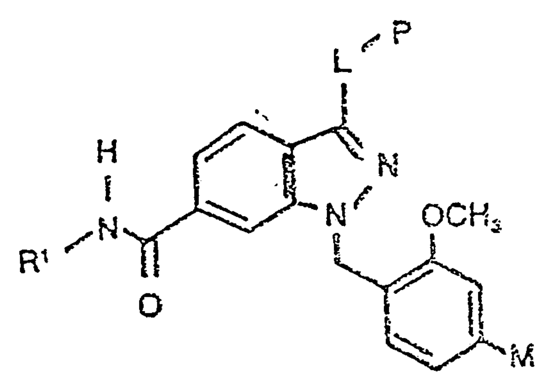

où $R^1$, L, M et P ont l'une quelconque des définitions données dans la revendication 1.

3. Composé suivant la revendication 1 ou 2, dans lequel

Ra est choisi dans le groupe comprenant l'hydrogène et les radicaux méthyle, éthyle, propyle et isopropyle ;

Rb est l'hydrogène ;

$R^1$ est choisi dans un groupe comprenant (a) les radicaux éthyle, propyle, isopropyle, butyle, isobutyle, sec.-butyle, tertiobutyle, pentyle, 1-éthylpropyle, hexyle, heptyle, 1-éthylpentyle ou nonyle, chacun d'entre eux pouvant contenir un ou plusieurs substituants fluoro ; (b) les radicaux benzyle, 1-phényléthyle, 2-phénylpropyle, 3-phénylpropyle, 1-méthyl-1-phényléthyle, 1-phénylbutyle et 1-phénylpentyle, chacun d'entre eux pouvant être substitué sur la portion alkyle par un radical méthoxy ou éthoxy et chacun d'entre eux pouvant être substitué sur la portion phényle par un représentant du groupe comprenant les radicaux fluoro, chloro et bromo, méthyle, éthyle, méthoxy et éthoxy ; (c) un radical cyclopropyle, cyclobutyle, cyclopentyle, cyclohexyle, cycloheptyle, cyclobutylméthyle, cyclopentylméthyle, cyclohexylméthyle, 1-cyclopentyléthyle, 2-cyclopentyléthyle, 1-cyclopentylpropyle, 1-cyclohexylpropyle, 1-cyclopentylbutyle et 1-cyclohexylbutyle, où chacun des groupements cycliques peut facultativement être substitué par un radical méthyle, éthyle ou isopropyle ; et (d) un radical cyclopentényl, cyclohexényle, cyclopenténylméthyle, 1-cyclohexène-4-yl-méthyle, et 1-(cyclohexényl)-butyle dont chacun des groupements cycliques peut facultativement être substitué par un radical méthyle, éthyle ou isopropyle ;

L est choisi dans un groupe comprenant (a) un radical méthylène, éthylène, éthylidène, triméthylène, tétraméthylène, 1,1-diméthyléthylène, 2,2-diméthyléthylène, pentaméthylène et hexaméthylène ; (b) un radical vinylène, 1-propénylène, 2-propénylène, 1-buténylène, 2-buténylène, 1,2-diméthylvinylène, 1,1-diméthyl-2-propénylène et 3,3-diméthyl-1-propénylène ; et (c) un radical éthynylène, 1-propynylène, 2-propynylène, 2-butynylène, 1,1-diméthyl-2-propynylène et 3,3-diméthyl-1-propynylène ;

$R^2$ est choisi dans un groupe comprenant l'hydrogène et les radicaux méthyle, éthyle, propyle, isopropyle, butyle, tertiobutyle, pentyle et phényle, ce dernier pouvant facultativement porter 1 ou 2 substituants choisis dans un groupe comprenant les radicaux fluoro, chloro, bromo, méthyle, éthyle,

méthoxy, éthoxy et trifluorométhyle, et $R^3$ et $R^4$ sont choisis indépendamment dans un groupe comprenant l'hydrogène et les radicaux méthyle, éthyle, propyle, isopropyle, butyle, tertiobutyle et pentyle ; ou bien

$R^2$ et $R^3$ forment conjointement avec l'atome adjacent d'azote un noyau de pyrrolidine, Pipéridine, pipérazine ou un noyau de N-alkylpipérazine dont le groupe N-alkyle est choisi dans un groupe comprenant les radicaux méthyle, éthyle, propyle, isopropyle, butyle, tertiobutyle et pentyle, et $R^4$ est choisi dans un groupe comprenant l'hydrogène et les radicaux méthyle, éthyle, propyle, isopropyle, butyle, tertiobutyle et pentyle ; et

$R^5$ est choisi dans un groupe comprenant les radicaux méthyle, éthyle, propyle, isopropyle et phényle, ce dernier pouvant facultativement porter 1 ou 2 substituants choisis indépendamment dans le groupe défini ci-dessus pour le groupe phényle de $R^2$ ;

Rc est un groupe méthoxy ou éthoxy ; et

$R^6$ est un groupe méthyle, éthyle, propyle, isopropyle, butyle, cyclopentyle, cyclohexyle, phényle, 1-naphtyle, 2-naphtyle, furyle, thiényle, pyridyle, benzyle, 1-naphtylméthyle ou 2-naphtylméthyle, dont chacun des groupements aromatiques ou hétéro-aromatiques peut être facultativement substitué comme défini ci-dessus pour un groupement phényle dans $R^1$.

**4.** Composé suivant la revendication 1 ou 2, dans lequel

$R^1$ est un groupe éthyle, propyle, isopropyle, butyle, isobutyl, sec.-butyle, tertiobutyle, pentyle, 1-éthylpropyle, hexyle, heptyle, 1-éthylpentyle, nonyle, benzyle, 4-chlorobenzyle, 4-triflurométhylbenzyle, 4-méthylbenzyle, 1-phényléthyle, 2-phényléthyle, 1-méthyl-1-phényléthyle, 1-phénylpropyle, 1-phénylpentyle, cyclopentyle, cyclohexyle, cyclobutylméthyle, cyclopentylméthyle, cyclohexylméthyle, 2-cyclopentyléthyle, 1-cyclopentylbutyle, 1-cyclohexylpropyle, 1-cyclohexylbutyle, cyclopenténylméthyle et 1-cyclohexène-4-ylméthyle ;

$R^2$, $R^3$ et $R^4$ sont choisis dans un groupe dans lequel (a) $R^2$ représente l'hydrogène, un radical méthyle, éthyle, propyle, isopropyle, tertiobutyle, phényle, 2-méthylphényle ou 4-chlorophényle, et $R^3$ et $R^4$ sont choisis indépendamment entre l'hydrogène et le radical méthyle ou éthyle ; et (b) $R^2$ et $R^3$ forment conjointement avec l'atome adjacent d'azote, un noyau de pipéridine, de morpholine ou de N-méthylpipérazine, et $R^4$ est l'hydrogène ou un radical méthyle ou éthyle ;

$R^5$ est un radical méthyle, éthyle, propyle, isopropyle, phényle, 2-méthylphényle ou 4-chlorophényle ;

$R^6$ est un radical méthyle, isopropyle, butyle, cyclopentyle, phényle, 4-chlorophényle, 4-méthylphényle, 2-chlorophényle, 2-méthylphényle, 2-méthoxyphényle, naphtyle, thién-2-yle ou 6-chloropyrid-3-yle ;

Ra est l'hydrogène ou un groupe méthyle ;

Rb est l'hydrogène ; et

Rc est l'hydrogène ou un groupe méthoxy.

**5.** Composé suivant l'une quelconque des revendications 1 à 4, dans lequel

$R^1$ est un groupe butyle, 1-éthylpentyle, 1-phényl propyle, cyclopentyle ou cyclopentylméthyle ; M est un groupe carboxy ou un groupe de formule $-CO.NK.SO_2R^6$ dans laquelle $R^6$ est un radical phényle ou 2-méthylphényle ; Ra est l'hydrogène ; et Rc est un radical méthoxy.

**6.** Composé de formule IIa ou IIb

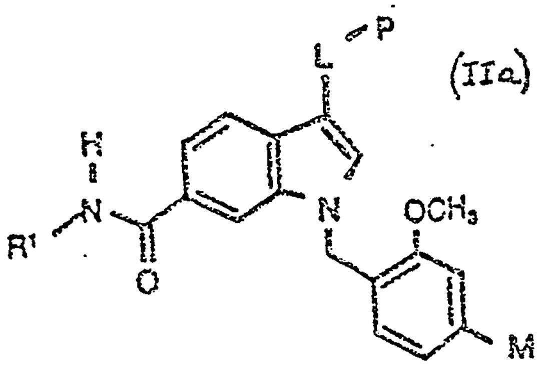

(IIa)

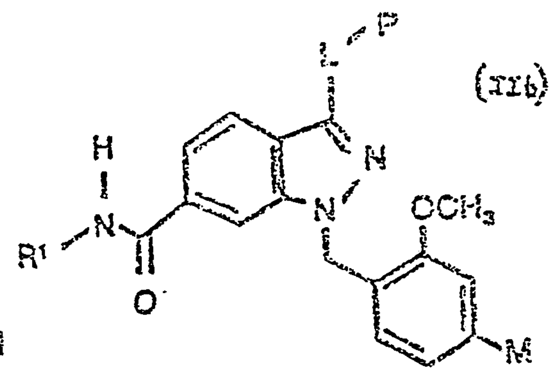

(IIb)

dans laquelle

$R^1$ est un groupe (cycloalkyle en $C_3$ à $C_6$)-(alkyleen $C_1$ à $C_4$) ; L est un groupe méthylène, éthylène ou vinylène ; M est un groupe carboxy ou un résidu acylsulfonamide de formule $-CO.NH.SO_2R^6$ dans laquelle $R^6$ est un groupe phényle portant facultativement un substituant choisi entre des substituants halogéno, alkyle en $C_1$ à $C_4$, alkoxy en $C_1$ à $C_4$ et trifluorométhyle ; et P est un groupe cyano, carbamoyle de formule $CONR^2R^3$ ou carbamoyloxy de formule $OCONR^2R^3$ où $R^2$ et $R^3$ représentent indépendamment l'hydrogène ou un radical méthyle, ou bien $R^2$ et $R^3$ forment conjointement avec l'atome adjacent d'azote un noyau de pyrrolidine ou de morpholine ; ou un sel pharmaceutiquement acceptable de ce composé.

7. Composé suivant l'une quelconque des revendications 1 à 6, sous réserve que $R^2$ et $R^3$ ne forment pas conjointement avec l'atome adjacent d'azote un noyau de pyrrolidine.

8. Sel suivant l'une quelconque des revendications précédentes, qui est dérivé d'une base formant un cation physiologiquement acceptable.

9. Procédé de préparation d'un composé de formule I ou d'un sel pharmaceutiquement acceptable de ce composé suivant l'une quelconque des revendications 1 à 8, qui est caractérisé par :

(A) pour un composé de formule I dans laquelle M est un groupe carboxy, la décomposition d'un ester de formule III :

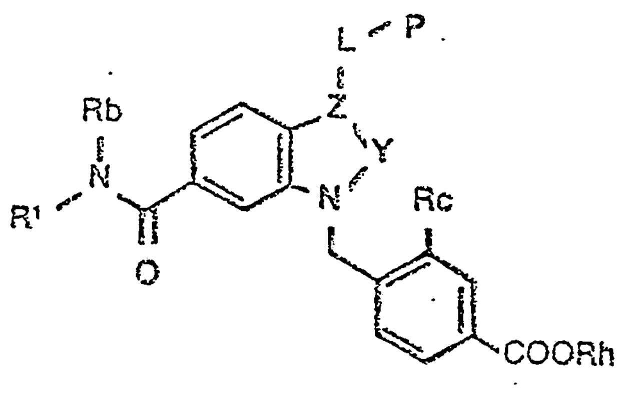

(B) l'acylation d'une amine de formule $R^1NHRb$ avec un acide carboxylique de formuule IV

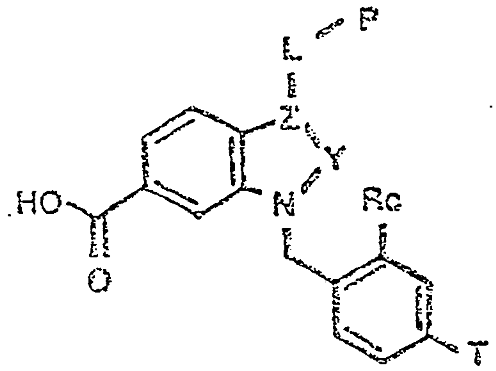

mais dans laquelle T est choisi parmi des valeurs définies pour M, ou avec un dérivé réactif de cet acide ;

(C) pour un composé de formule I dans laquelle -Y-Z< a la valeur (a) définie dans la revendication 1, la réaction d'un indole de formule V

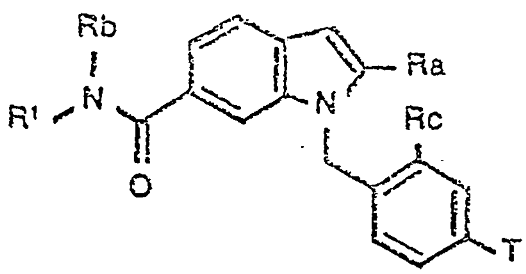

mais dans laquelle T est choisi parmi les valeurs définies pour M, avec un réactif de formule U.L.P. ;

(D) la réaction d'un composé imino de formule VI,

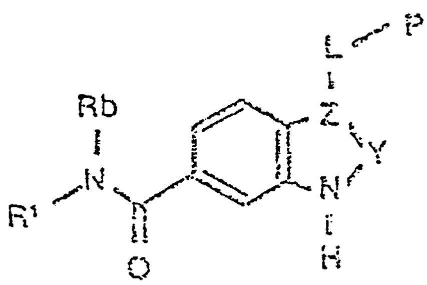

avec un agent alkylant de formule VII,

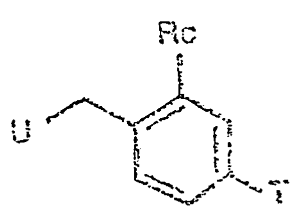

mais dans laquelle T est choisi parmi les valeurs définies pour M ;

(E) pour un composé de formule L dans laquelle M est un radical 1H-tétrazole-5-yle, la réaction d'un dérivé cyano de formule VIII

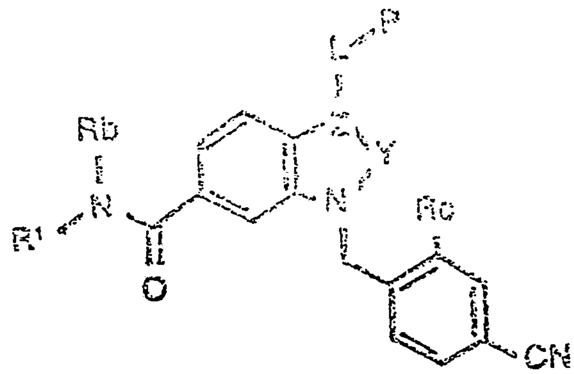

avec un azide ;

(F) pour un composé de formule I dans laquelle M est un groupe de formule $CO.NH.SO_2.R^6$, la réaction d'un composé de formule I dans laquelle M est un groupe carboxy avec un dérivé sulfonamidique de formule $R^6.SO_2.NH_2$ en présence d'un agent déshydratant ou la réaction d'un dérivé réactif d'un acide de formule I avec un sulfonamide ou un sel d'un sulfonamide de formule $R^6.SO_2.NH_2$.

(G) pour un composé de formule I dans laquelle -Y-Z< a la valeur (c) définie dans la revendication 1, l'hydrogénation catalytique d'un indole de formule I dans laquelle -Y-Z< a la valeur (a) définie dans la revendication 1 ;

(H) la réduction d'une double liaison d'un composé de formule I dans laquelle L contient une double liaison pour former le composé correspondant de formule I dans laquelle L ne contient pas de double liaison ;

(I) pour un composé de formule L dans laquelle P est un groupe carbamoyle de formule $CONR^2R^3$, l'acylation d'une amine de formule $HNR^2R^3$ avec un acide correspondant, ou un dérivé réactif de cet acide, de formule IX :

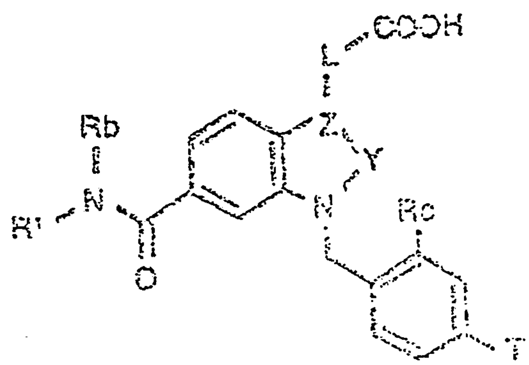

mais dans laquelle T est choisi parmi les valeurs définies pour M ;

(J) pour un composé de formule I dans laquelle P est un groupe 1H-tétrazole-5-yle, la réaction d'un nitrile de formule X,

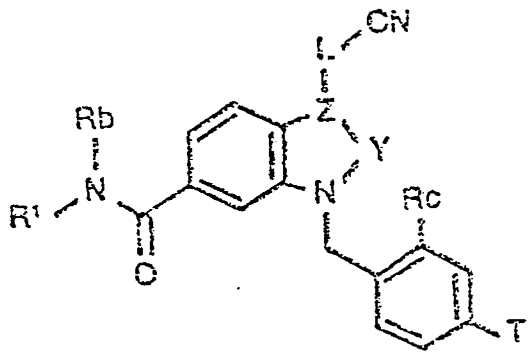

mais dans laquelle T est choisi parmi les valeurs définies pour M, avec un azide en utilisant des modes opératoires analogues à ceux qui ont été décrits ci-dessus dans la partie (E) ;

(K) pour un composé de formule L dans laquelle P a la valeur $NR^4CONR^2R^3$, $OCONR^2R^3$, $NR^4COOR^5$, l'acylation d'un composé de formule XI,

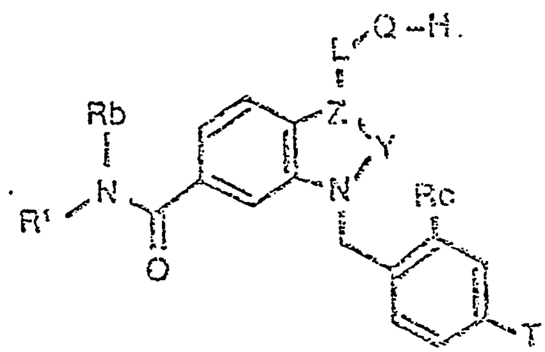

mais dans laquelle T est choisi parmi les valeurs définies pour M et dans laquelle QH a la valeur $NR^4H$ ou OH, avec un agent acylant ;

(L) pour un composé de formule I dans laquelle P a la valeur $S(O)_nR^5$ et n est égal à 0, la réaction d'un composé de formule XII,

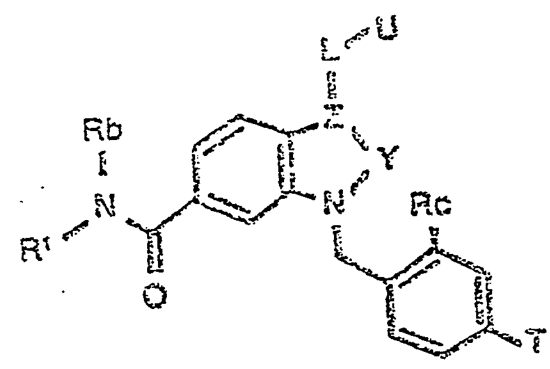

mais dans laquelle T est choisi parmi les valeurs définies pour M, avec un mercaptan de formule $R^5SH$ ;

(M) pour un composé de formule I dans laquelle P a la valeur $S(O)_nR^5$ et n a la valeur 1 ou 2, l'oxydation du composé I correspondant dans lequel n est égal à 0 ou n est égal à 1 ; et

(N) pour un composé de formule I dans laquelle L contient une double liaison adjacente à Z, la réaction d'un aldéhyde ou d'une cétone de formule XIII,

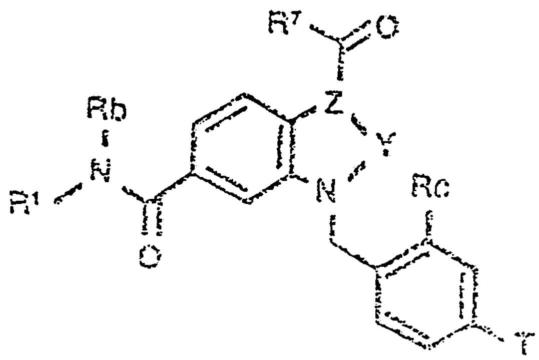

mais dans laquelle T est choisi parmi les valeurs définies pour M et $R^7$ est choisi entre l'hydrogène et des groupes alkyle en $C_1$ à $C_4$, avec un réactif zwitterionique ou carbanionique ;

où -Y-Z<, Ra-Rc, $R^1$-$R^6$, L, M, P et n ont les définitions données dans l'une quelconque des revendications 1 à 7 ; Rh est choisi entre les radicaux benzyle, phényle et alkyle en $C_1$ à $C_6$ portant facultativement un substituant acétoxy, alkoxy en $C_1$ à $C_4$ ou alkylthio en $C_1$ à $C_4$ ; U est un radical chloro, bromo, iodo, méthanesulfonyloxy ou p-toluènesulfonyloxy ; et Hal est un radical chloro, bromo ou iodo ; après quoi

lorsqu'on désire un sel pharmaceutiquement acceptable, on fait réagir une forme acide du composé de formule I avec une base apportant un cation physiologiquement acceptable.

**10.** Composé de formule III

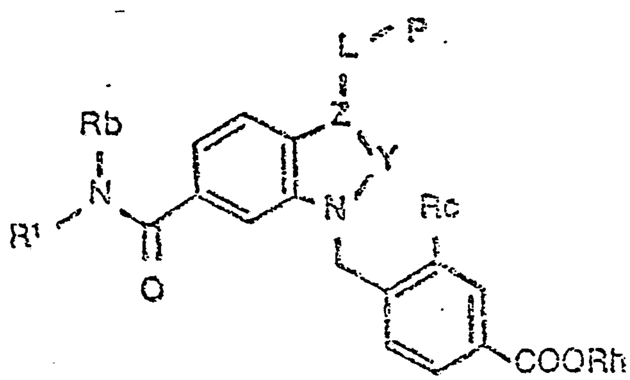

dans laquelle Rh a l'une quelconque des définitions données dans la revendication 9 et $R^1$, Rb, Rc, L, p et le groupe -Y-Z< ont l'une quelconque des définitions données dans les revendications 1 à 7.

**11.** Composition pharmaceutique comprenant un composé de formule I ou un sel pharmaceutiquement acceptable de ce composé suivant l'une quelconque des revendications 1 à 8 et un diluant ou support acceptable du point de vue pharmaceutique.

**Revendication pour les Etats contractants suivants : AT, ES**

**1.** Procédé de production d'un composé de formule I

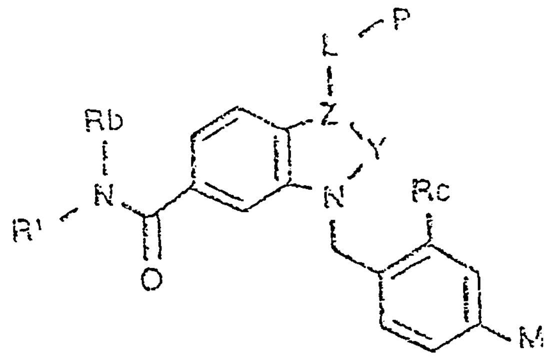

dans laquelle le groupe -Y-Z< est choisi dans le groupe constitué de

(a) -C(Ra) = C<

(b) -N = C< et

(c) -CH(Ra)-CH<

où "<" indique deux liaisons séparées ;

et dans laquelle

Ra est l'hydrogène ou un groupe alkyle en $C_1$ à $C_4$ ;

Rb est l'hydrogène ;

$R^1$ est un groupe alkyle en $C_2$ à $C_{10}$ contenant facultativement un ou plusieurs substituants fluor ; ou bien $R^1$ est un groupe phényl-(alkyle en $C_1$ à $C_6$) dont la partie alkyle en $C_1$ à $C_6$ peut porter facultativement un substituant fluoro ou alkoxy en $C_1$ à $C_4$ et dans lequel la partie phényle peut porter facultativement un substituant choisi dans le groupe constitué des substituants halogéno, alkyle en $C_1$ à $C_4$, alkoxy en $C_1$ à $C_4$ et trifluorométhyle ; ou bien $R^1$ est un groupe cycloalkyle en $C_3$ à $C_8$ ou (cycloalkyle en $C_3$ à $C_8$)(alkyle en $C_1$ à $C_6$), la portion cyclique de n'importe lequel d'entre eux pouvant contenir une liaison de non-saturation et pouvant facultativement porter 1 ou 2 substituants alkyle en $C_1$ à $C_4$ ;

L est une jonction alkylène en $C_1$ à $C_{10}$, contenant facultativement une liaison double ou triple :

P est un groupe polaire choisi entre un groupe cyano, 1H-tétrazole-5-yle, carbamoyle de formule $CONR^2R^3$, uréido de formule $NR^4CONR^2R^3$, carbamoyloxy de fomule $OCONR^2R^3$, un carbamate de formule $NR^4COOR^5$ et un groupe thio (facultativement oxydé) de formule $S(O)_nR^5$, où

$R^2$ est choisi dans un groupe comprenant l'hydrogène, un groupe alkyle en $C_1$ à $C_6$ et un groupe phényle, lequel peut porter facultativement 1 ou 2 substituants choisis dans le groupe comprenant des substituants halogéno, alkyle en $C_1$ à $C_4$, alkoxy en $C_1$ à $C_4$ et trifluorométhyle ; et

$R^3$ et $R^4$ sont choisis indépendamment dans un groupe comprenant l'hydrogène et un radical alkyle en $C_1$ à $C_6$ ;

ou bien $R^2$ et $R^3$ forment conjointement avec l'atome adjacent d'azote un noyau de pyrrolidine, de pipéridine, de morpholine, de pipérazine ou de N-(alkyle en $C_1$ à $C_6$)-pipérazine, et $R^4$ est l'hydrogène ou un groupe alkyle en $C_1$ à $C_6$ ;

$R^5$ est choisi dans un groupe comprenant un radical alkyle en $C_1$ à $C_4$ et le radical phényle, lequel peut porter facultativement 1 ou 2 substituants choisis dans le groupe des substituants halogéno, alkyle en $C_1$ à $C_4$, alkoxy en $C_1$ à $C_4$ et trifluorométhyle ; et

n est le nombre 0, 1 ou 2 ;

Rc est choisi dans un groupe comprenant l'hydrogène et un radical alkoxy en $C_1$ à $C_4$ ; et

M est un groupe acide choisi dans un groupe comprenant un reste carboxy, un résidu acylsulfonamide de formule $-CO.NH.SO_2R^6$ et un reste 1H-tétrazole-5-yle, où

$R^6$ est choisi dans un groupe comprenant des radicaux alkyle en $C_1$ à $C_6$, cycloalkyle en $C_3$ à $C_8$, aryle en $C_6$ à $C_{12}$, hétéroaryle choisi entre furyle, thiényle et pyridyle, et (aryle en $C_6$ à $C_{12}$)-(alkyle en $C_1$ à $C_4$), où l'un quelconque des groupements aromatiques ou hétéro-aromatiques peut porter 1 ou 2 substituants choisis dans le groupe des substituants halogéno, alkyle en $C_1$ à $C_4$, alkoxy en $C_1$ à $C_4$ et trifluorométhyle ;

ou d'un sel d'addition d'acide pharmaceutiquement acceptable de ce composé, procédé qui est choisi entre

(A) pour un composé de formule I dans laquelle M est un groupe carboxy, la décomposition d'un ester de formule III :

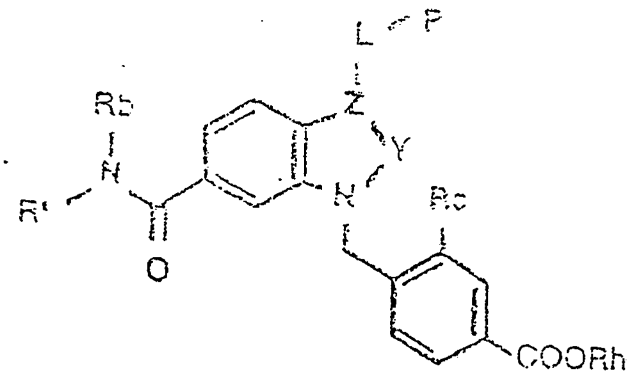

(B) l'acylation d'une amine de formule $R^1NHRb$ avec un acide carboxylique de formule IV

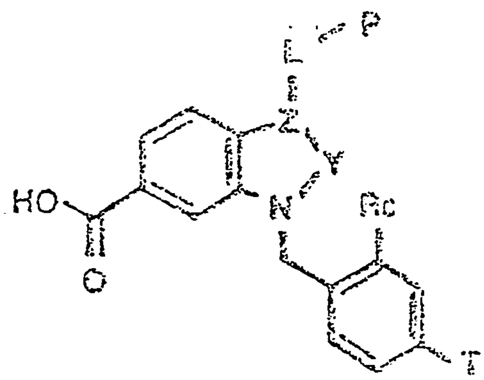

mais dans laquelle T est choisi parmi les valeurs définies pour M, ou avec un dérivé réactif de cet acide ;

(C) pour un composé de formule I dans laquelle -Y-Z< a la valeur (a) définie ci-dessus, la réaction d'un indole de formule V

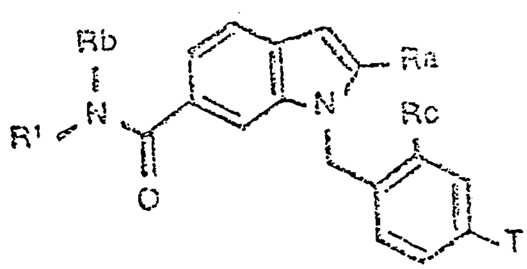

mais dans laquelle T est choisi parmi les valeurs définies pour M, avec un réactif de formule U.L.P. ;

(D) la réaction d'un composé imino de formule VI,

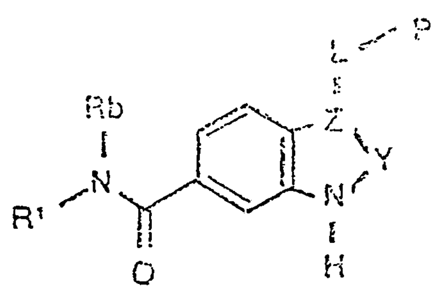

avec un agent alkylant de formule VII,

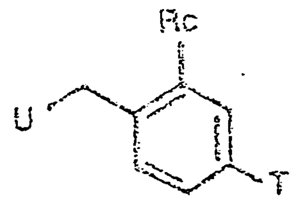

mais dans laquelle T est choisi parmi les valeurs définies pour M ;

(E) pour un composé de formule I dans laquelle M est un radical 1H-tétrazole-5-yle, la réaction d'un dérivé cyano de formule VIII

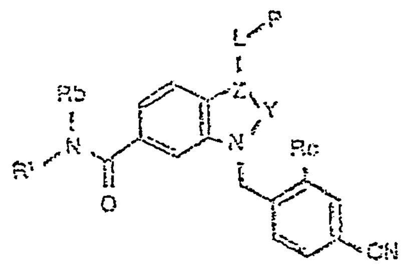

avec un azide ;

(F) pour un composé de formule I dans laquelle M est un groupe de formule $CO.NH.SO_2.R^6$, la réaction d'un composé de formule I dans laquelle M est un groupe carboxy avec un dérivé sulfonamidique de formule $R^6.SO_2.NH_2$ en présence d'un agent déshydratant, ou la réaction d'un dérivé réactif d'un acide de formule I avec un sulfonamide ou un sel d'un sulfonamide de formule $R^6.SO_2.NH_2$ ;

(G) pour un composé de formule I dans laquelle -Y-Z< a la valeur (c) définie ci-dessus, l'hydrogénation catalytique d'un indole de formule I dans laquelle -Y-Z< a la valeur (a) définie ci-dessus ;

(H) la réduction d'une double liaison d'un composé de formule I dans laquelle L contient une double liaison pour former le composé correspondant de formule I dans laquelle L ne contient pas de double liaison ;

(I) pour un composé de formule I dans laquelle P est un groupe carbamoyle de formule $CONR^2R^3$, l'acylation d'une amine de formule $HNR^2R^3$ avec un acide correspondant, ou un dérivé réactif de cet acide, de formule IX :

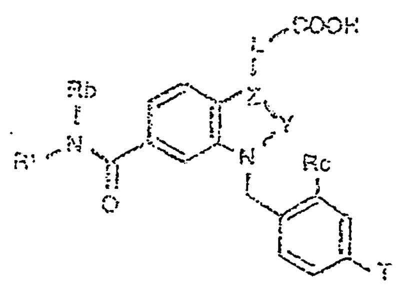

mais dans laquelle T est choisi parmi les valeurs définies pour M ;

(J) pour un composé de formule I dans laquelle P est un groupe 1H-tétrazole-5-yle, la réaction d'un nitrile de formule X,

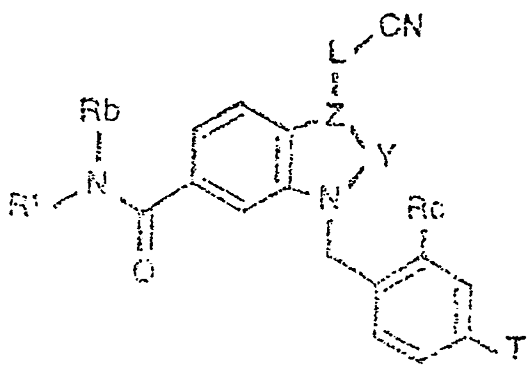

mais dans laquelle T est choisi parmi les valeurs définies pour M, avec un azide en utilisant des modes opératoires analogues à ceux qui ont été décrits ci-dessus dans la partie (E) ;
(K) pour un composé de formule I dans laquelle P a la valeur $NR^4CONR^2R^3$, $OCONR^2R^3$, $NR^4COOR^5$, l'acylation d'un composé de formule XI,

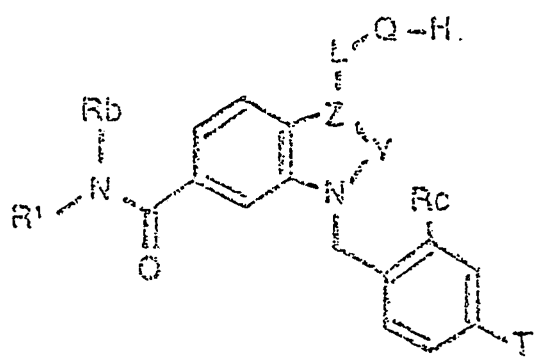

mais dans laquelle T est choisi parmi les valeurs définies pour M et dans laquelle QH a la valeur $NR^4H$ ou OH, avec un agent acylant ;
(L) pour un composé de formule I dans laquelle P a la valeur $S(O)_nR^5$ et n est égal à 0, la réaction d'un composé de formule XII,

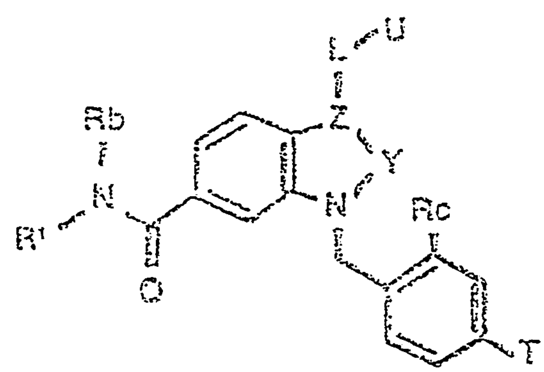

mais dans laquelle T est choisi parmi les valeurs définies pour M, avec un mercaptan de formule $R^5SH$ ;
(M) pour un composé de formule I dans laquelle P a la valeur $S(O)_nR^5$ et n a la valeur 1 ou 2, l'oxydation du composé I correspondant dans lequel n est égal à 0 ou n est égal à 1 ; et
(N) pour un composé de formule I dans laquelle L contient une double liaison adjacente à Z, la réaction d'un aldéhyde ou d'une cétone de formule XIII,

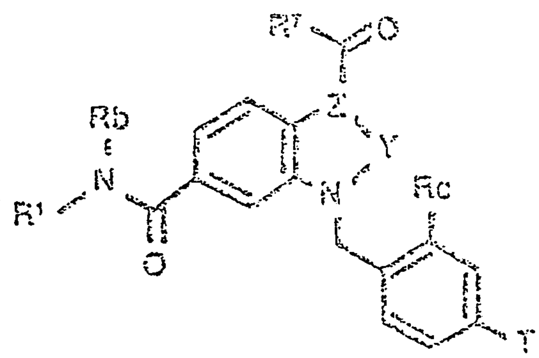

mais dans laquelle T est choisi parmi les valeurs définies pour M et $R^7$ est choisi entre l'hydrogène et des groupes alkyle en $C_1$ à $C_4$, avec un réactif zwitterionique ou carbanionique ;

où -Y-Z<, Ra-Rc, $R^1$-$R^6$, L, M, P et n ont les définitions données ci-dessus ; Rh est choisi entre les radicaux benzyle, phényle et alkyle en $C_1$ à $C_6$ portant facultativement un substituant acétoxy, alkoxy en $C_1$ à $C_4$ ou alkylthio en $C_1$ à $C_4$ ; U est un radical chloro, bromo, iodo, méthanesulfonyloxy ou p-toluènesulfonyloxy ; et Hal est un radical chloro, bromo ou iodo ; après quoi

lorsqu'on désire un sel pharmaceutiquement acceptable, on fait réagir une forme acide du composé de formule I avec une base apportant un cation physiologiquement acceptable.

**Revendications pour l'Etat contractant suivant : GR**

**1.** Procédé de production d'un composé de formule I

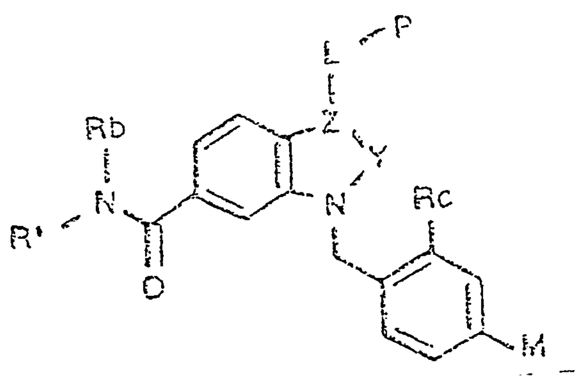

dans laquelle le groupe -Y-Z< est choisi dans le groupe constitué de

(a) -C(Ra) = C<

(b) -N = C< et

(c) -CH(Ra)-CH<

où "<" indique deux liaisons séparées ;

et dans laquelle

Ra est l'hydrogène ou un groupe alkyle en $C_1$ à $C_4$ ;

Rb est l'hydrogène ;

$R^1$ est un groupe alkyle en $C_2$ à $C_{10}$ contenant facultativement un ou plusieurs substituants fluor ; ou bien $R^1$ est un groupe phényl-(alkyle en $C_1$ à $C_6$) dont la partie alkyle en $C_1$ à $C_6$ peut porter facultativement un substituant fluoro ou alkoxy en $C_1$ à $C_4$ et dans lequel la partie phényle peut porter facultativement un substituant choisi dans le groupe constitué des substituants halogéno, alkyle en $C_1$ à $C_4$, alkoxy en $C_1$ à $C_4$ et trifluorométhyle ; ou bien $R^1$ est un groupe cycloalkyle en $C_3$ à $C_8$ ou (cycloalkyle en $C_3$ à $C_8$)(alkyle en $C_1$ à $C_6$), la portion cyclique de n'importe lequel d'entre eux pouvant contenir une liaison de non-saturation et pouvant facultativement porter 1 ou 2 substituants alkyle en $C_1$ à $C_4$ ;

L est une jonction alkylène en $C_1$ à $C_{10}$, contenant facultativement une liaison double ou triple ;

P est un groupe polaire choisi entre un groupe cyano, 1H-tétrazole-5-yle, carbamoyle de formule $CONR^2R^3$, uréido de formule $NR^4CONR^2R^3$, carbamoyloxy de formule $OCONR^2R^3$, un carbamate de formule $NR^4COOR^5$ et un groupe thio (facultativement oxydé) de formule $S(O)_nR^5$ , où

$R^2$ est choisi dans un groupe comprenant l'hydrogène, un groupe alkyle en $C_1$ à $C_6$ et un groupe phényle, lequel peut porter facultativement 1 ou 2 substituants choisis dans le groupe comprenant des substituants halogéno, alkyle en $C_1$ à $C_4$, alkoxy en $C_1$ à $C_4$ et trifluorométhyle ; et

$R^3$ et $R^4$ sont choisis indépendamment dans un groupe comprenant l'hydrogène et un radical alkyle en $C_1$ à $C_6$ ;

ou bien $R^2$ et $R^3$ forment conjointement avec l'atome adjacent d'azote un noyau de pyrrolidine, de pipéridine, de morpholine, de pipérazine ou de N-(alkyle en $C_1$ à $C_6$)-pipérazine, et $R^4$ est l'hydrogène ou un groupe alkyle en $C_1$ à $C_6$ ;

$R^5$ est choisi dans un groupe comprenant un radical alkyle en $C_1$ à $C_4$ et le radical phényle, lequel peut porter facultativement 1 ou 2 substituants choisis dans le groupe des substituants halogéno, alkyle en $C_1$ à $C_4$, alkoxy en $C_1$ à $C_4$ et trifluorométhyle ; et

n est le nombre 0, 1 ou 2 ;

Rc est choisi dans un groupe comprenant l'hydrogène et un radical alkoxy en $C_1$ à $C_4$ ; et

M est un groupe acide choisi dans un groupe comprenant un reste carboxy, un résidu acylsulfonamide de formule $-CO.NH.SO_2R^6$ et un reste 1H-tétrazole-5-yle, où

$R^6$ est choisi dans un groupe comprenant des radicaux alkyle en $C_1$ à $C_6$, cycloalkyle en $C_3$ à $C_8$, aryle en $C_6$ à $C_{12}$, hétéroaryle choisi entre furyle, thiényle et pyridyle, et (aryle en $C_6$ à $C_{12}$)-(alkyle en $C_1$ à $C_4$), où l'un quelconque des groupements aromatiques ou hétéro-aromatiques peut porter 1 ou 2 substituants choisis dans le groupe des substituants halogéno, alkyle en $C_1$ à $C_4$, alkoxy en $C_1$ à $C_4$ et trifluorométhyle ;

ou d'un sel d'addition d'acide pharmaceutiquement acceptable de ce composé, procédé qui est choisi entre

(A) pour un composé de formule I dans laquelle M est un groupe carboxy, la décomposition d'un ester de formule III :

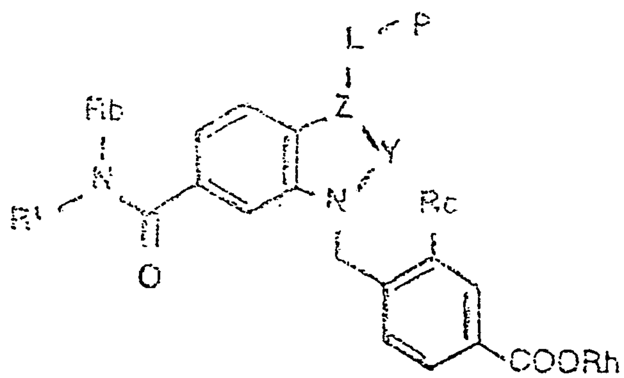

(B) l'acylation d'une amine de formule $R^1NHRb$ avec un acide carboxylique de formule IV

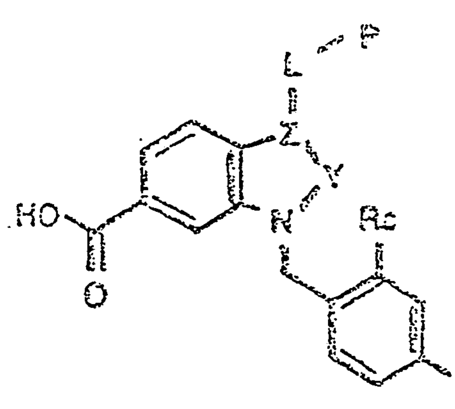

mais dans laquelle T est choisi parmi des valeurs définies pour M, ou avec un dérivé réactif de cet acide ;
(C) pour un composé de formule I dans laquelle -Y-Z< a la valeur (a) définie ci-dessus, la réaction d'un indole de formule V

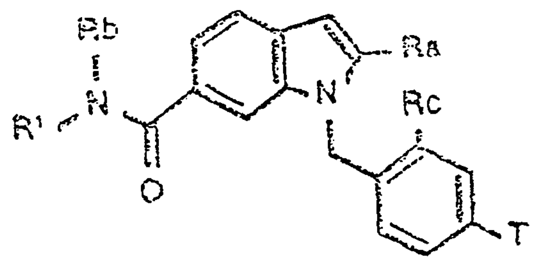

mais dans laquelle T est choisi parmi les valeurs définies pour M, avec un réactif de formule U.L.P. ;
(D) la réaction d'un composé imino de formule VI,

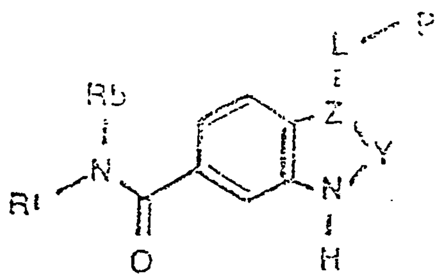

avec un agent alkylant de formule VII,

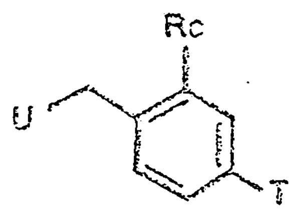

mais dans laquelle T est choisi parmi les valeurs définies pour M ;
(E) pour un composé de formule I dans laquelle M est un radical 1H-tétrazole-5-yle, la réaction d'un dérivé cyano de formule VIII

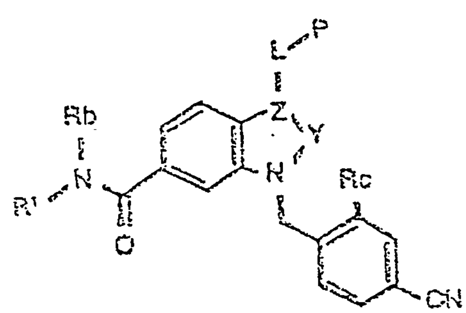

avec un azide ;
(F) pour un composé de formule I dans laquelle M est un groupe de formule $CO.NH.SO_2.R^6$, la réaction d'un composé de formule I dans laquelle M est un groupe carboxy avec un dérivé

sulfonamidique de formule $R^6.SO_2.NH_2$ en présence d'un agent déshydratant, ou la réaction d'un dérivé réactif d'un acide de formule I avec un sulfonamide ou un sel d'un sulfonamide de formule $R^6.SO_2.NH_2$ ;

(G) pour un composé de formule I dans laquelle -Y-Z< a la valeur (c) définie ci-dessus, l'hydrogénation catalytique d'un indole de formule I dans laquelle -Y-Z< a la valeur (a) définie ci-dessus ;

(H) la réduction d'une double liaison d'un composé de formule I dans laquelle L contient une double liaison pour former le composé correspondant de formule I dans laquelle L ne contient pas de double liaison ;

(I) pour un composé de formule I dans laquelle P est un groupe carbamoyle de formule $CONR^2R^3$, l'acylation d'une amine de formule $HNR^2R^3$ avec un acide correspondant, ou un dérivé réactif de cet acide, de formule IX :

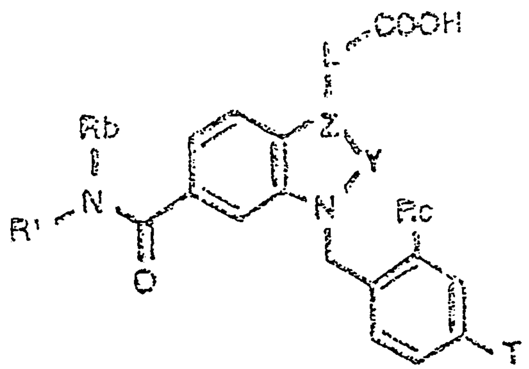

mais dans laquelle T est choisi parmi les valeurs définies pour M ;

(J) pour un composé de formule I dans laquelle P est un groupe 1H-tétrazole-5-yle, la réaction d'un nitrile de formule X,

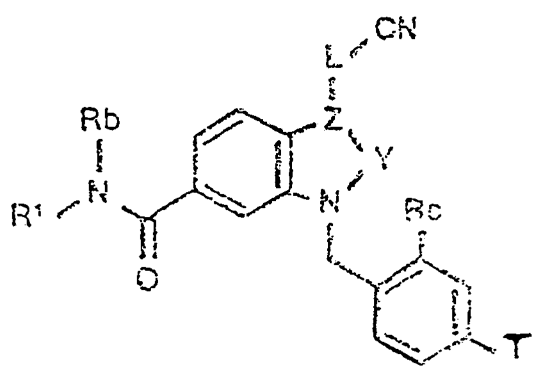

mais dans laquelle T est choisi parmi les valeurs définies pour M, avec un azide en utilisant des modes opératoires analogues à ceux qui ont été décrits ci-dessus dans la partie (E) ;

(K) pour un composé de formule I dans laquelle P a la valeur $NR^4CONR^2R^3$, $OCONR^2R^3$, $NR^4COOR^5$, l'acylation d'un composé de formule XI,

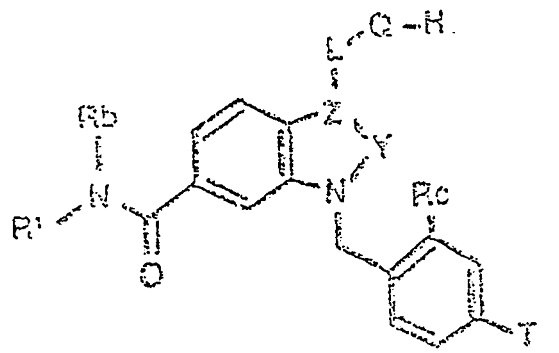

mais dans laquelle T est choisi parmi les valeurs définies pour M et dans laquelle QH a la valeur $NR^4H$ ou OH, avec un agent acylant ;

(L) pour un composé de formule I dans laquelle P a la valeur $S(O)_nR^5$ et n est égal à 0, la réaction d'un composé de formule XII,

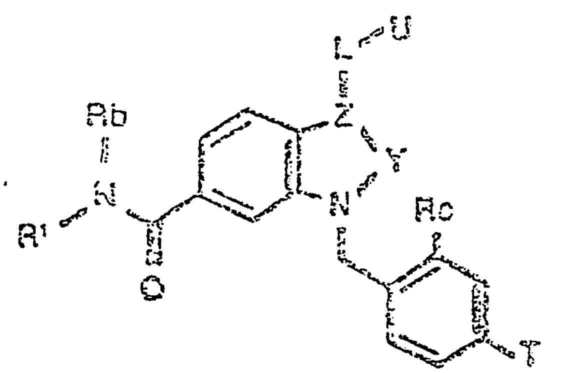

mais dans laquelle T est choisi parmi les valeurs définies pour M, avec un mercaptan de formule $R^5SH$ ;

(M) pour un composé de formule I dans laquelle P a la valeur $S(O)_nR^5$ et n a la valeur 1 ou 2, l'oxydation du composé I correspondant dans lequel n est égal à 0 ou n est égal à 1 ; et

(N) pour un composé de formule I dans laquelle L contient une double liaison adjacente à Z, la réaction d'un aldéhyde ou d'une cétone de formule XIII,

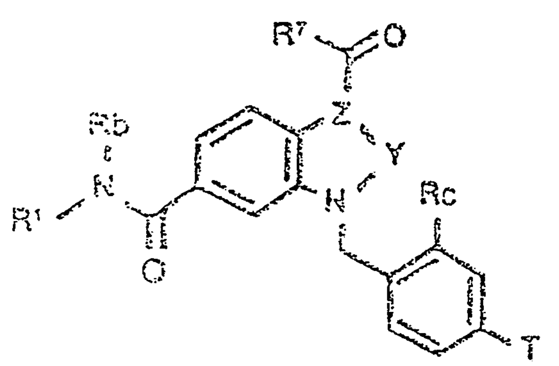

mais dans laquelle T est choisi parmi les valeurs définies pour M et $R^7$ est choisi entre l'hydrogène et des groupes alkyle en $C_1$ à $C_4$, avec un réactif zwitterionique ou carbanionique ;

où -Y-Z<, Ra-Rc, $R^1$-$R^6$, L, M, P et n ont les définitions données ci-dessus ; Rh est choisi entre les radicaux benzyle, phényle et alkyle en $C_1$ à $C_6$ portant facultativement un substituant acétoxy, alkoxy en $C_1$ à $C_4$ ou alkylthio en $C_1$ à $C_4$ ; U est un radical chloro, bromo, iodo, méthanesulfonyloxy ou p-toluènesulfonyloxy ; et Ha1 est un radical chloro, bromo ou iodo ; après quoi

lorsqu'on désire un sel pharmaceutiquement acceptable, on fait réagir une forme acide du composé de formule I avec une base apportant un cation physiologiquement acceptable.

**2.** Composé de formule III

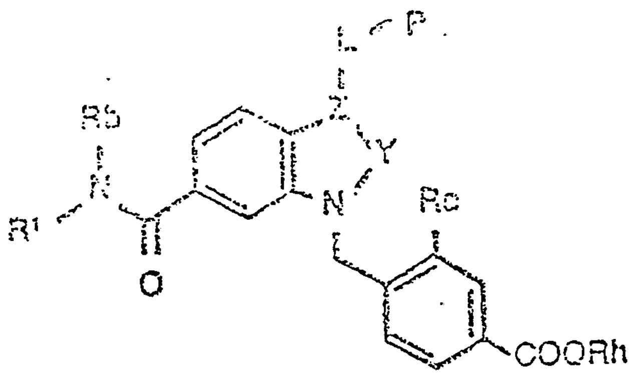

dans laquelle Rh a l'une quelconque des définitions données dans la revendication 1 et $R^1$, Rb, Rc, L, P et le groupe -Y-Z< ont l'une quelconque des définitions données dans la revendication 1.

**Patentansprüche**

**Patentansprüche für folgende Vertragsstaaten : BE, CH, DE, FR, GB, IT, LI, LU, NL, SE**

**1.** Verbindung der Formel I

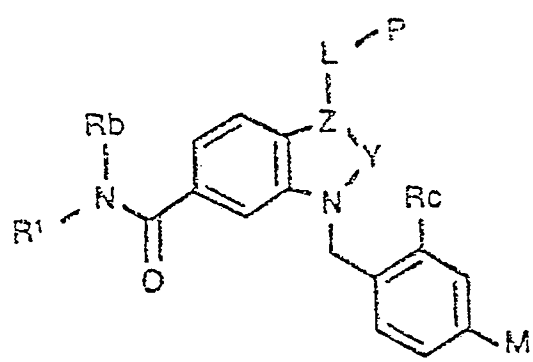

worin
die Gruppe -Y-Z< ausgewählt ist aus
(a) -C(Ra) = C<
(b) -N = C< und
(c) -CH(Ra)-CH<
wobei "<" zwei gesonderte Bindungen bezeichnet;
und worin

Ra für Wasserstoff oder (1-4C)Alkyl steht;

Rb für Wasserstoff steht;

$R^1$ für (2-10C)Alkyl steht, das gegebenenfalls einen oder mehrere Fluoro-Substituenten trägt; oder $R^1$ für Phenyl(1-6C)alkyl steht, wobei der (1-6C)Alkyl-Teil gegebenenfalls einen Fluoro- oder (1-4C)-Alkoxy-Substituenten trägt und wobei der Phenyl-Teil gegebenenfalls einen aus Halogeno, (1-4C)-Alkyl, (1-4C)Alkoxy und Trifluoromethyl ausgewählten Substituenten trägt; oder $R^1$ für (3-8C)Cycloalkyl oder (3-8C)Cycloalkyl-(1-6C)alkyl steht, wobei der cyclische Teil der beiden gegebenenfalls eine ungesättigte Bindung enthält und gegebenenfalls einen oder zwei (1-4C)Alkyl-Substituenten trägt;

L für eine (1-10C)Alkylen-Gruppierung steht, die gegebenenfalls eine Doppel- oder Dreifachbindung enthält;

P für eine polare Gruppe steht, die ausgewählt ist aus Cyano, 1H-Tetrazol-5-yl, Carbamoyl der Formel $CONR^2R^3$, Ureido der Formel $NR^4CONR^2R^3$, Carbamoyloxy der Formel $OCONR^2R^3$, einem Carbamat der Formel $NR^4COOR^5$ und einer (gegebenenfalls oxidierten) Thio-Gruppe der Formel $S(O)_nR^5$, worin

$R^2$ ausgewählt ist aus Wasserstoff, (1-6C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt und $R^3$ und $R^4$ unabhängig ausgewählt sind aus Wasserstoff und (1-6C)Alkyl;

oder $R^2$ und $R^3$ gemeinsam mit dem benachbarten Stickstoff einen Pyrrolidin, Piperidin, Morpholin, Piperazin oder N-(1-6C)Alkylpiperazin-Ring bilden und $R^4$ für Wasserstoff oder (1-6C)Alkyl steht;

$R^5$ ausgewählt ist aus (1-4C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt; und

n für die Ganzzahl 0, 1 oder 2 steht;

Rc ausgewählt ist aus Wasserstoff und (1-4C)Alkoxy; und

M für eine saure Gruppe steht, die ausgewählt ist aus Carboxy, einem Acylsulfonamid-Rest der Formel $-CO.NH.SO_2R^6$ und 1H-Tetrazol-5-yl, wobei

$R^6$ ausgewählt ist aus (1-6C)Alkyl, (3-8C)Cycloalkyl, (6-12C)Aryl, einem der Heteroaryle Furyl, Thienyl und Pyridyl, und (6-12C)Aryl-(1-4C)alkyl, wobei die aromatischen und heteroaromatischen Teile gegebenenfalls einen oder zwei aus Halogeno, (1-4C)Alkyl, (1-4C)Alkoxy und Trifluoromethyl ausgewählte Substituenten tragen;

oder ein pharmazeutisch zulässiges Salz davon.

2. Verbindung nach Anspruch 1, welche ausgewählt ist aus den Indol-Derivaten der Formel IIa

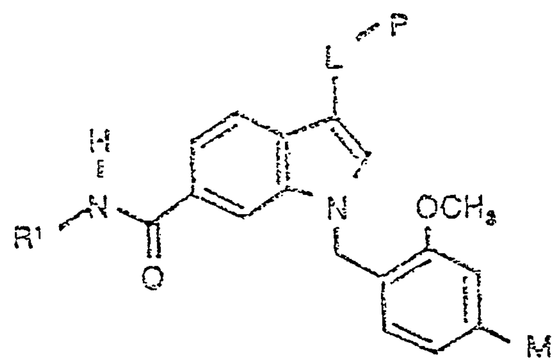

und den Indazol-Derivaten der Formel IIb

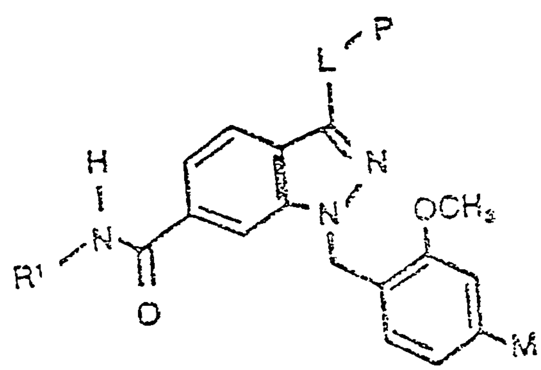

worin $R^1$, L, M und P die in Anspruch 1 angegebenen Bedeutungen besitzen.

3. Verbindung nach Anspruch 1 oder 2, worin

Ra ausgewählt ist aus Wasserstoff, Methyl, Ethyl, Propyl und Isopropyl;

Rb für Wasserstoff steht;

$R^1$ ausgewählt ist aus (a) Ethyl, Propyl, Isopropyl, Butyl, Isobutyl, sec-Butyl, t-Butyl, Pentyl, 1-Ethylpropyl, Hexyl, Heptyl, 1-Ethylpentyl oder Nonyl, von denen jedes einen oder mehrere Fluoro-Substituenten enthalten kann; (b) Benzyl, 1-Phenylethyl, 2-Phenylpropyl, 3-Phenylpropyl, 1-Methyl-1-phenylethyl, 1-Phenylbutyl und 1-Phenylpentyl, von denen jedes am Alkyl-Teil durch Methoxy oder Ethoxy substituiert sein kann und von jedes am Phenyl-Teil durch einen aus Fluoro, Chloro, Bromo, Methyl, Ethyl, Methoxy und Ethoxy ausgewählten Substituenten substituiert sein kann; (c) Cyclopropyl, Cyclobutyl, Cyclopentyl, Cyclohexyl, Cycloheptyl, Cyclobutylmethyl, Cyclopentylmethyl, Cyclohexylmethyl, 1-Cyclopentylethyl, 2-Cyclopentylethyl, 1-Cyclopentylpropyl, 1-Cyclohexylpropyl, 1-Cyclopentylbutyl und 1-Cyclohexylbutyl, wobei jeder der cyclischen Teile gegebenenfalls durch Methyl, Ethyl oder Isopropyl substituiert ist; und (d) Cyclopentenyl, Cyclohexenyl, Cyclopentenylmethyl, 1-Cyclohexen-4-ylmethyl und 1-(Cyclohexenyl)butyl, wobei jeder der cyclischen Teile gegebenenfalls durch Methyl, Ethyl oder Isopropyl substituiert ist;

L ausgewählt ist aus (a) Methylen, Ethylen, Ethyliden, Trimethylen, Tetramethylen, 1,1-Dimethylethylen, 2,2-Dimethylethylen, Pentamethylen und Hexamethylen; (b) Vinylen, 1-Propenylen, 2-Propenylen, 1-Butenylen, 2-Butenylen, 1,2-Dimethylvinylen, 1,1-Dimethyl-2-propenylen und 3,3-Dimethyl-1-propenylen; und (c) Ethinylen, 1-Propinylen, 2-Propinylen, 2-Butinylen, 1,1-Dimethyl-2-propinylen und 3,3-Dimethyl-1-propinylen;

$R^2$ ausgewählt ist aus Wasserstoff, Methyl, Ethyl, Propyl, Isopropyl, Butyl, t-Butyl, Pentyl und Phenyl, wobei der Phenyl-Teil gegebenenfalls einen oder zwei aus Fluoro, Chloro, Bromo, Methyl, Ethyl, Methoxy, Ethoxy und Trifluoromethyl ausgewählte Substituenten trägt, und $R^3$ und $R^4$ unabhängig ausgewählt sind aus Wasserstoff, Methyl, Ethyl, Propyl, Isopropyl, Butyl, t-Butyl und Pentyl;

oder $R^2$ und $R^3$ gemeinsam mit dem benachbarten Stickstoff einen Pyrrolidin-, Piperidin-, Piperazin- oder N-Alkylpiperazin-Ring bilden, bei dem die N-Alkyl-Gruppe ausgewählt ist aus Methyl, Ethyl, Propyl, Isopropyl, Butyl, t-Butyl und Pentyl, und $R^4$ ausgewählt ist aus Wasserstoff, Methyl, Ethyl, Propyl, Isopropyl, Butyl, t-Butyl und Pentyl; und

$R^5$ ausgewählt ist aus Methyl, Ethyl, Propyl, Isopropyl und Phenyl, wobei die Phenyl-Gruppe gegebenenfalls einen oder zwei Substituenten trägt, die unabhängig aus den oben für eine Phenyl-Gruppe von $R^2$ definierten Substituenten ausgewählt sind;

Rc für Methoxy oder Ethoxy steht; und

$R^6$ für Methyl, Ethyl, Propyl, Isopropyl, Butyl, Cyclpentyl, Cyclohexyl, Phenyl, 1-Naphthyl, 2-Naphthyl, Furyl, Thienyl, Pyridyl, Benzyl, 1-Naphthylmethyl oder 2-Naphthylmethyl steht, wobei jeder aromatische oder heteroaromatische Teil gegebenenfalls substituiert ist, wie es oben für einen Phenyl-Teil in $R^1$ definiert ist.

4. Verbindung nach Anspruch 1 oder 2, worin

$R^1$ für Ethyl, Propyl, Isopropyl, Butyl, Isobutyl, sec-Butyl, t-Butyl, Pentyl, 1-Ethylpropyl, Hexyl, Heptyl, 1-Ethylpentyl, Nonyl, Benzyl, 4-Chlorobenzyl, 4-Trifluoromethylbenzyl, 4-Methylbenzyl, 1-Phenylethyl, 2-Phenylethyl, 1-Methyl-1-phenylethyl, 1-Phenylpropyl, 1-Phenylpentyl, Cyclopentyl, Cyclohexyl, Cyclobutylmethyl, Cyclopentylmethyl, Cyclohexylmethyl, 2-Cyclopentylethyl, 1-Cyclopentylbutyl, 1-Cyclohexylpropyl, 1-Cyclohexylbutyl, Cyclopentenylmethyl oder 1-Cyclohexen-4-yl-methyl steht;

$R^2$, $R^3$ und $R^4$ wie folgt ausgewählt sind: (a) $R^2$ steht für Wasserstoff, Methyl, Ethyl, Propyl, Isopropyl, t-Butyl, Phenyl, 2-Methylphenyl oder 4-Chlorophenyl und $R^3$ und $R^4$ stehen unabhängig für Wasserstoff, Methyl oder Ethyl; und (b) $R^2$ und $R^3$ bilden zusammen mit dem benachbarten Stickstoff einen Piperidin-, Morpholin- oder N-Methylpiperazin-Ring und $R^4$ steht für Wasserstoff, Methyl oder Ethyl;

$R^5$ für Methyl, Ethyl, Propyl, Isopropyl, Phenyl, 2-Methylphenyl oder 4-Chlorophenyl steht;

$R^6$ für Methyl, Isopropyl, Butyl, Cyclopentyl, Phenyl, 4-Chlorophenyl, 4-Methylphenyl, 2-Chlorophenyl, 2-Methylphenyl, 2-Methoxyphenyl, Naphthyl, Thien-2-yl oder 6-Chloropyrid-3-yl steht;

Ra für Wasserstoff oder Methyl steht;

Ab für Wasserstoff steht; und

Rc für Wasserstoff oder Methoxy steht.

5. Verbindung nach einem der Ansprüche 1 bis 4, worin $R^1$ für Butyl, 1-Ethylpentyl, 1-Phenylpropyl, Cyclopentyl oder Cyclopentylmethyl steht; M für Carboxy oder eine Gruppe der Formel $-CO.NH.SO_2R^6$ steht, worin $R^6$ Phenyl oder 2-Methylphenyl bedeutet; Ra für Wasserstoff steht; und Rc für Methoxy steht.

6. Verbindung der Formel IIa oder IIb

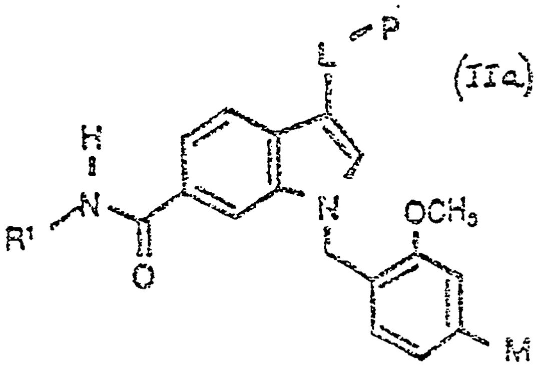
(IIa)

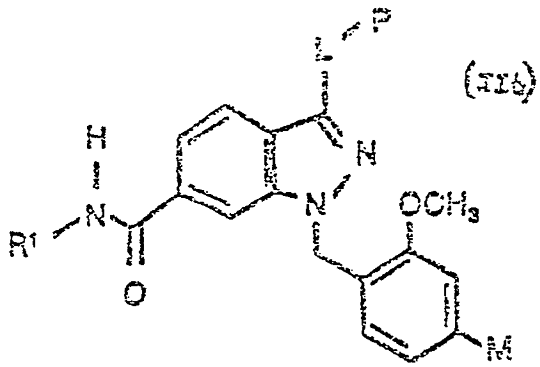
(IIb)

worin $R^1$ für (3-6C)Cycloalkyl-(1-4C)alkyl steht; L für Methylen, Ethylen oder Vinylen steht; M für Carboxy oder einen Acylsulfonamid-Rest der Formel $-CO.NH.SO_2R^6$ steht, worin $R^6$ Phenyl bedeutet, das gegebenenfalls einen aus Halogeno, (1-4C)Alkyl, (1-4C)Alkoxy und Trifluoromethyl ausgewählten Substituenten trägt; und P für Cyano, Carbamoyl der Formel $CONR^2R^3$ oder Carbamoyloxy der Formel $OCONR^2R^3$ steht, worin $R^2$ und $R^3$ unabhängig Wasserstoff oder Methyl bedeuten oder $R^2$ und $R^3$ zusammen mit dem benachbarten Stickstoff einen Pyrrolidin- oder Morpholin-Ring bilden; oder ein pharmazeutisch zulässiges Salz davon.

7. Verbindung nach einem der Ansprüche 1 bis 6, bei welcher $R^2$ und $R^3$ zusammen mit der benachbarten Stickstoff keinen Pyrrolidin-Ring bilden.

8. Salz nach einem der vorhergehenden Ansprüche, welches sich von einer Base ableitet, die ein physiologisch zulässiges Kation bildet.

9. Verfahren zur Herstellung einer Verbindung der Formel I oder eines pharmazeutisch zulässigen Salzes davon, wie sie in einem der Ansprüche 1 bis 8 definiert sind, dadurch gekennzeichnet, daß
(A) für eine Verbindung der Formel I, worin M für eine Carboxy-Gruppe steht, ein Ester der Formel III

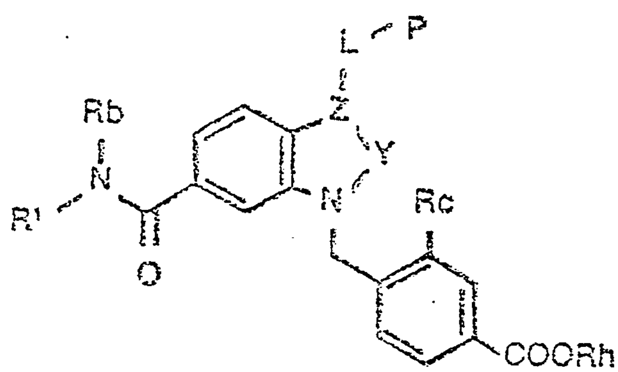

zersetzt wird;
(B) ein Amin der Formel $R^1NHRb$ mit einer Carbonsäure der Formel IV

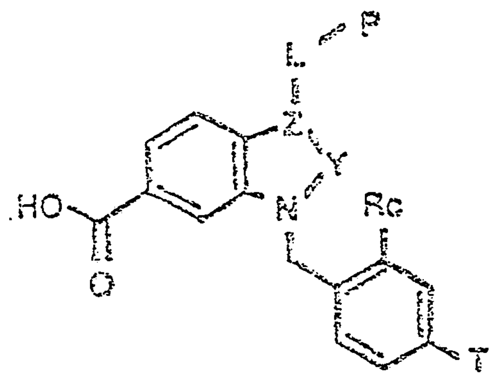

worin T aus den für M definierten Werten ausgewählt ist, oder mit einem reaktiven Derivat davon acyliert wird;

(C) für eine Verbindung der Formel I, worin -Y-Z< den oben in Anspruch 1 definierten Wert (a) besitzt, ein Indol der Formel V

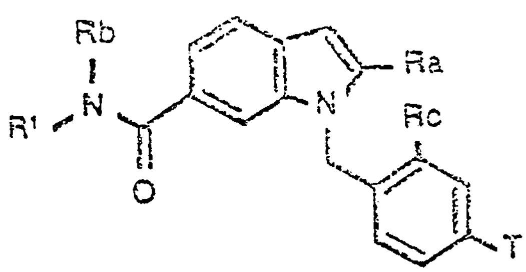

worin T aus den für M definierten Werten ausgewählt ist, mit einem Reagenz der Formel U.L.P umgesetzt wird;

(D) eine Imino-Verbindung der Formel VI

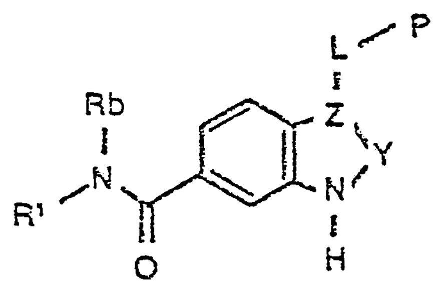

mit einem Alkylierungsmittel der Formel VII

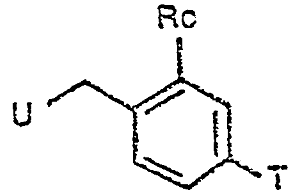

worin T aus den für M definierten Werten ausgewählt ist, umgesetzt wird;

(E) für eine Verbindung der Formel I, worin M für ein 1H-Tetrazol-5-yl-Radikal steht, ein Cyano-Derivat der Formel VIII

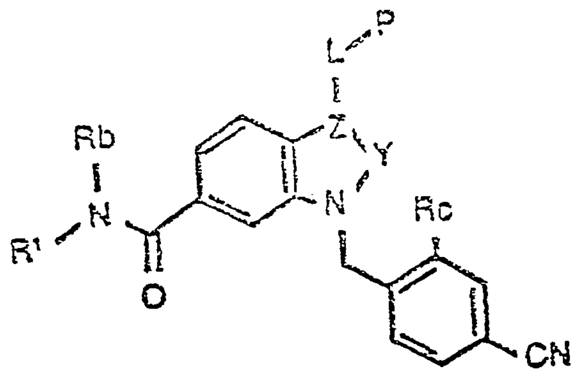

mit einem Azid umgesetzt wird;

(F) für eine Verbindung der Formel I, worin M für eine Gruppe der Formel $CO.NH.SO_2.R^6$ steht, eine Verbindung der Formel I, worin M für Carboxy steht, mit einem Sulfonamid-Derivat der Formel $R^6.SO_2.NH_2$ in Gegenwart eines Dehydratisierungsmittels umgesetzt wird oder ein reaktives Derivat einer Säure der Formel I mit einem Sulfonamid der Formel $R^6.SO_2.NH_2$ oder einem Salz davon umgesetzt wird;

(G) für eine Verbindung der Formel I, worin -Y-Z< den in Anspruch 1 definierten Wert (c) aufweist, ein Indol der Formel I, worin -Y-Z< den in Anspruch 1 definierten Wert (a) aufweist, katalytisch hydriert wird;

(H) die Doppelbindung einer Verbindung der Formel I, worin L eine Doppelbindung enthält, reduziert wird, um die entsprechende Verbindung der Formel I herzustellen, in der L keine Doppelbindung enthält;

(I) für eine Verbindung der Formel I, worin P für eine Carbamoyl-Gruppe der Formel $CONR^2R^3$ steht, ein Amin der Formel $HNR^2R^3$ mit einer entsprechenden Säure der Formel IX

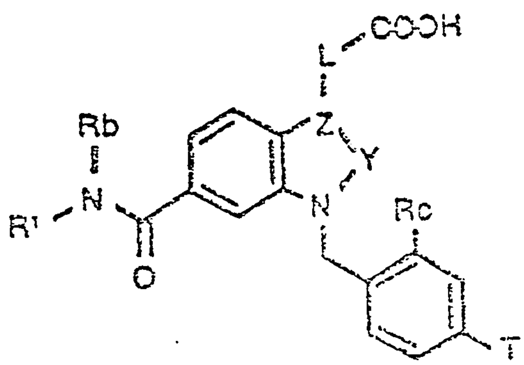

worin T aus den für M definierten Werten ausgewählt ist, oder einem reaktiven Derivat davon acyliert wird;

(J) für eine Verbindung der Formel I, worin P für eine 1H-Tetrazol-5-yl-Gruppe steht, ein Nitril der Formel X

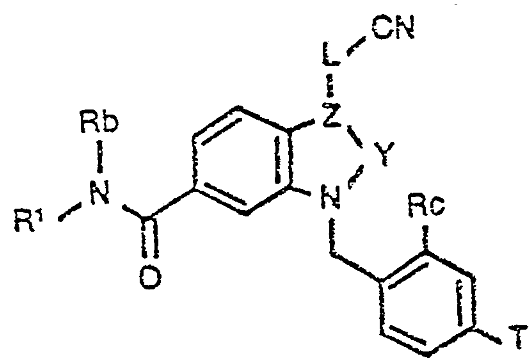

worin T aus den für M definierten Werten ausgewählt ist, mit einem Azid umgesetzt wird, wobei ähnliche Verfahrensweisen verwendet werden, wie sie oben in Teil (E) beschrieben sind;
(K) für eine Verbindung der Formel I, worin P den Wert $NR^4CONR^2R^3$ , $OCONR^2R^3$ oder $NR^4COOR^5$ hat, eine Verbindung der Formel XI

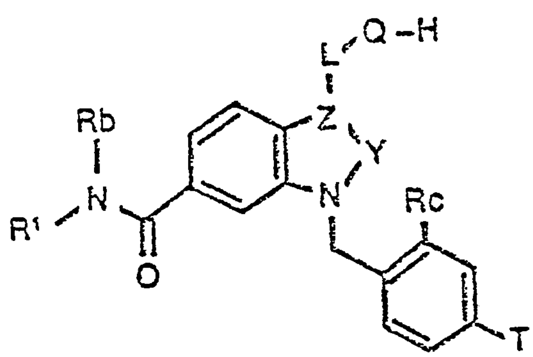

worin T aus den für M definierten Werten ausgewählt ist und worin QH den Wert $NR^4H$ oder OH aufweist, mit einem Acylierungsmittel acyliert wird;
(L) für eine Verbindung der Formel I, worin P den Wert $S(O)_nR^5$ aufweist und n für 0 steht, eine Verbindung der Formel XII

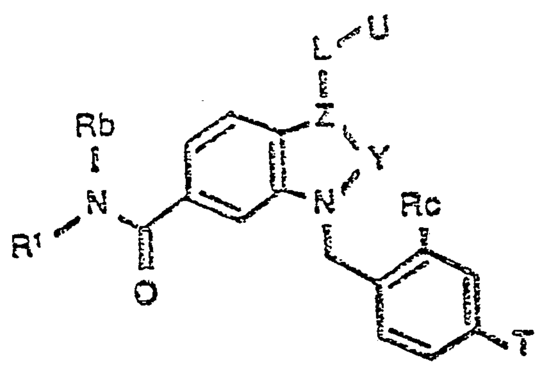

worin T aus den für M definierten Werten ausgewählt ist, mit einem Mercaptan der Formel $R^5SH$ umgesetzt wird;
(M) für eine Verbindung der Formel I, worin P den Wert $S(O)_nR^5$ hat und n für 1 oder 2 steht, die entsprechende Verbindung I, worin n für 0 oder 1 steht, oxidiert wird; und
(N) für eine Verbindung der Formel I, worin L eine Doppelbindung in Nachbarschaft zu Z enthält, ein Aldehyd oder Keton der Formel XIII

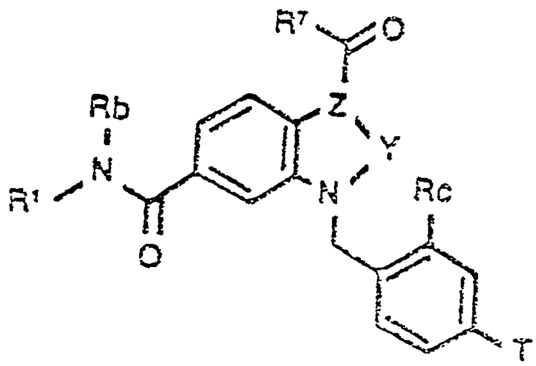

worin T aus den für M definierten Werten ausgewählt ist und $R^7$ aus Wasserstoff und (1-4C)Alkyl ausgewählt ist, mit einem zwitterionischen oder carbanionischen Reagenz umgesetzt wird; wobei -Y-Z< , Ra bis Rc, $R^1$ bis $R^6$, L, M, P und n die in einem der Ansprüche 1 bis 7 angegebenen Bedeutungen besitzen; Rh ausgewählt ist aus Phenyl, Benzyl und (1-6C)Alkyl, das gegebenenfalls einen Acetoxy-, (1-4C)Alkoxy- oder (1-4C)Alkylthio-Substituenten trägt; U für Chloro, Bromo, Jodo, Methansulfonyloxy oder p-Toluolsulfonyloxy steht; und Hal für Chloro, Bromo oder Jodo steht; worauf, wenn ein pharmazeutisch zulässiges Salz gewünscht wird, eine saure Form der Verbindung der Formel I mit einer Base umgesetzt wird, die ein physiologisch zulässiges Kation liefert.

**10.** Verbindung der Formel III

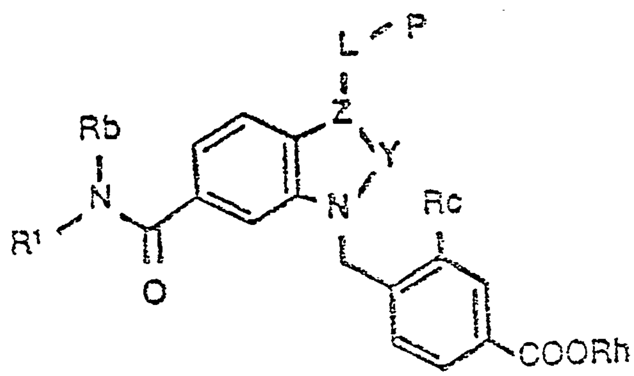

worin Rh eine der in Anspruch 9 angegebenen Bedeutungen besitzt und $R^1$, Rb, Rc, L, P und die Gruppe -Y-Z< eine der in Anspruch 1 bis 7 angegebenen Bedeutungen besitzen.

**11.** Pharmazeutische Zusammensetzung, welche eine Verbindung der Formel I oder ein pharmazeutisch zulässiges Salz davon nach einem der Ansprüche 1 bis 8 und ein pharmazeutisch zulässiges Verdünnungs- oder Trägermittel enthält.

**Patentansprüch für folgende Vertragsstaaten : AT, ES**

**1.** Verfahren zur Herstellung einer Verbindung der Formel I

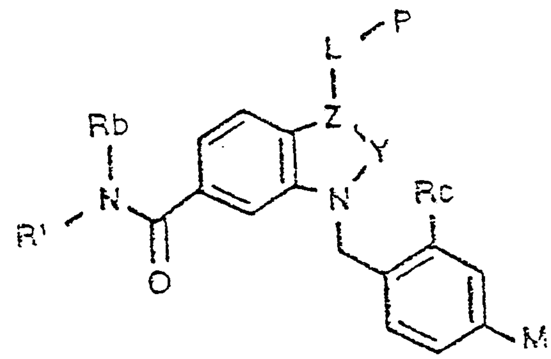

worin
die Gruppe -Y-Z< ausgewählt ist aus
(a) -C(Ra)=C<
(b) -N=C< und
(c) -CH(Ra)-CH<
wobei "<" zwei gesonderte Bindungen bezeichnet;
und worin

Ra für Wasserstoff oder (1-4C)Alkyl steht;

Rb für Wasserstoff steht;

$R^1$ für (2-10C)Alkyl steht, das gegebenenfalls einen oder mehrere Fluoro-Substituenten trägt; oder $R^1$ für Phenyl(1-6C)alkyl steht, wobei der (1-6C)Alkyl-Teil gegebenenfalls einen Fluoro- oder (1-4C)-Alkoxy-Substituenten trägt und wobei der Phenyl-Teil gegebenenfalls einen aus Halogeno, (1-4C)Alkyl, (1-4C)Alkoxy und Trifluoromethyl ausgewählten Substituenten trägt; oder $R^1$ für (3-8C)Cycloalkyl oder (3-8C)Cycloalkyl-(1-6C)alkyl steht, wobei der cyclische Teil der beiden gegebenenfalls eine ungesättigte Bindung enthält und gegebenenfalls einen oder zwei (1-4C)Alkyl-Substituenten trägt;

L für eine (1-10)Alkylen-Gruppierung steht, die gegebenenfalls eine Doppel- oder Dreifachbindung enthält;

P für eine polare Gruppe steht, die ausgewählt ist aus Cyano, 1H-Tetrazol-5-yl, Carbamoyl der Formel $CONR^2R^3$, Ureido der Formel $NR^4CONR^2R^3$, Carbamoyloxy der Formel $OCONR^2R^3$, einem Carbamat der Formel $NR^4COOR^5$ und einer (gegebenenfalls oxidierten) Thio-Gruppe der Formel $S(O)_nR^5$, worin

$R^2$ ausgewählt ist aus Wasserstoff, (1-6C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt und $R^3$ und $R^4$ unabhängig ausgewählt sind aus Wasserstoff und (1-6C)Alkyl;

oder $R^2$ und $R^3$ gemeinsam mit dem benachbarten Stickstoff einen Pyrrolidin, Piperidin, Morpholin, Piperazin oder N-(1-6C)Alkylpiperazin-Ring bilden und $R^4$ für Wasserstoff oder (1-6C)Alkyl steht;

$R^5$ ausgewählt ist aus (1-4C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt; und

n für die Ganzzahl 0, 1 oder 2 steht;

Rc ausgewählt ist aus Wasserstoff und (1-4C)Alkoxy; und

M für eine saure Gruppe steht, die ausgewählt ist aus Carboxy, einem Acylsulfonamid-Rest der Formel $-CO.NH.SO_2R^6$ und 1H-Tetrazol-5-yl, wobei

$R^6$ ausgewählt ist aus (1-6C)Alkyl, (3-8C)Cycloalkyl, (6-12C)Aryl, einem der Heteroaryle Furyl, Thienyl und Pyridyl, und (6-12C)Aryl-(1-4C)alkyl, wobei die aromatischen und heteroaromatischen Teile gegebenenfalls einen oder zwei aus Halogeno, (1-4C)Alkyl, (1-4C)Alkoxy und Trifluoromethyl ausgewählte Substituenten tragen;

oder eines pharmazeutisch zulässigen Salzes davon, bei welchem

(A) für eine Verbindung der Formel I, worin M für eine Carboxy-Gruppe steht, ein Ester der Formel III

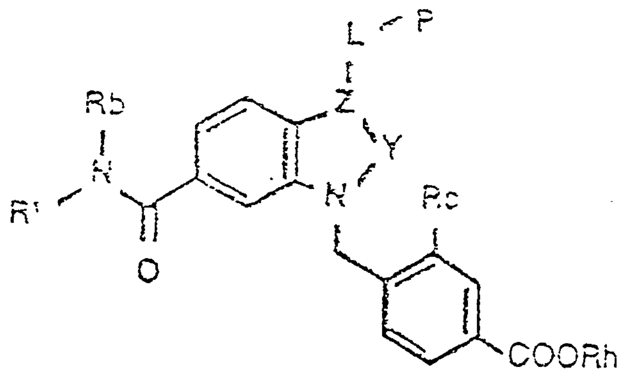

zersetzt wird;
(B) ein Amin der Formel $R^1NHRb$ mit einer Carbonsäure der Formel IV

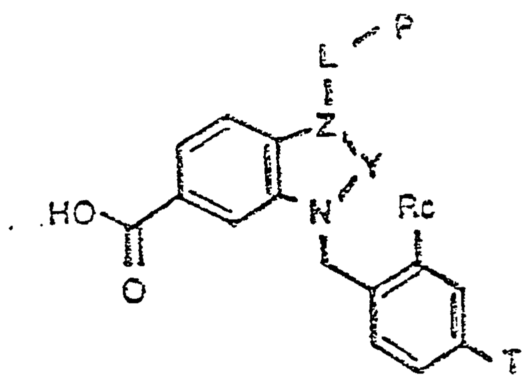

worin T aus den für M definierten Werten ausgewählt ist, oder mit einem reaktiven Derivat davon acyliert wird;
(C) für eine Verbindung der Formel I, worin -Y-Z< den oben definierten Wert (a) besitzt, ein Indol der Formel V

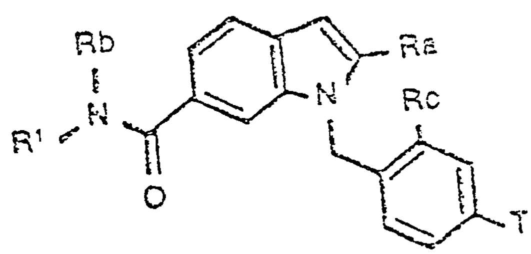

worin T aus den für M definierten Werten ausgewählt ist, mit einem Reagenz der Formel U.L.P umgesetzt wird;
(D) eine Imino-Verbindung der Formel VI

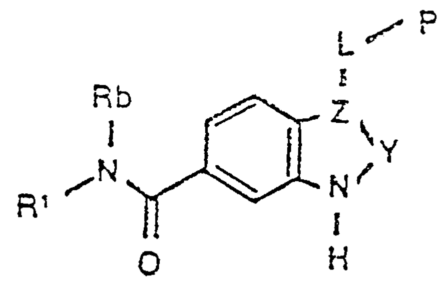

mit einem Alkylierungsmittel der Formel VII

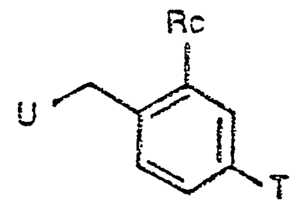

worin T aus den für M definierten Werten ausgewählt ist, umgesetzt wird;

(E) für eine Verbindung der Formel I, worin M für ein 1H-Tetrazol-5-yl-Radikal steht, ein Cyano-Derivat der Formel VIII

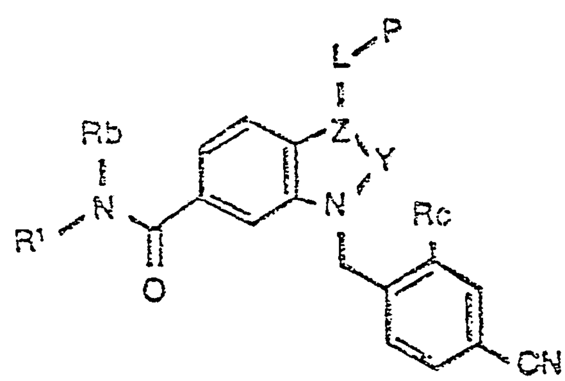

mit einem Azid umgesetzt wird;

(F) für eine Verbindung der Formel I, worin M für eine Gruppe der Formel $CO.NH.SO_2.R^6$ steht, eine Verbindung der Formel I, worin M für Carboxy steht, mit einem Sulfonamid-Derivat der Formel $R^6.SO_2.NH_2$ in Gegenwart eines Dehydratisierungsmittels umgesetzt wird oder ein reaktives Derivat einer Säure der Formel I mit einem Sulfonamid der Formel $R^6.SO_2.NH_2$ oder einem Salz davon umgesetzt wird;

(G) für eine Verbindung der Formel I, worin -Y-Z< den oben definierten Wert (c) aufweist, ein Indol der Formel I, worin -Y-Z< den oben definierten Wert (a) aufweist, katalytisch hydriert wird;

(H) die Doppelbindung einer Verbindung der Formel I, worin L eine Doppelbindung enthält, reduziert wird, um die entsprechende Verbindung der Formel I herzustellen, in der L keine Doppelbindung enthält;

(I) für eine Verbindung der Formel I, worin P für eine Carbamoyl-Gruppe der Formel $CONR^2R^3$ steht, ein Amin der Formel $HNR^2R^3$ mit einer entsprechenden Säure der Formel IX

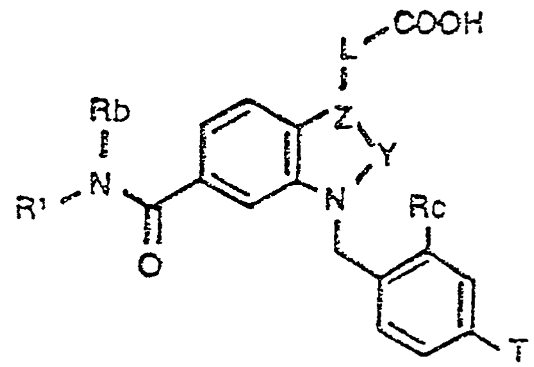

worin T aus den für M definierten Werten ausgewählt ist, oder einem reaktiven Derivat davon acyliert wird;
(J) für eine Verbindung der Formel I, worin P für eine 1H-Tetrazol-5-yl-Gruppe steht, ein Nitril der Formel X

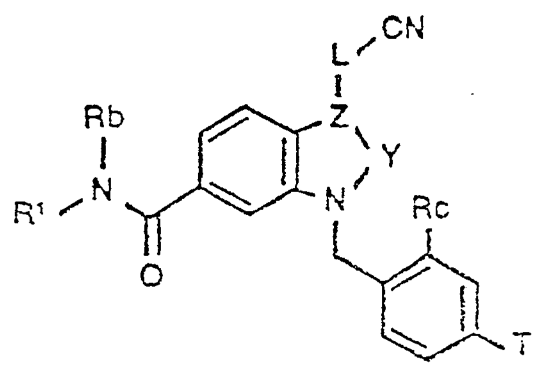

worin T aus den für M definierten Werten ausgewählt ist, mit einem Azid umgesetzt wird, wobei ähnliche Verfahrensweisen verwendet werden, wie sie oben in Teil (E) beschrieben sind;
(K) für eine Verbindung der Formel I, worin P den Wert $NR^4CONR^2R^3$, $OCONR^2R^3$ oder $NR^4COOR^5$ hat, eine Verbindung der Formel XI

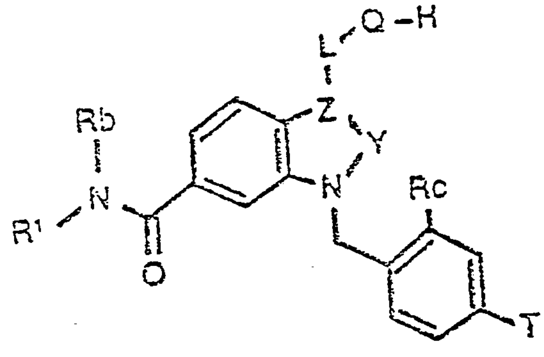

worin T aus den für M definierten Werten ausgewählt ist und worin QH den Wert $NR^4H$ oder OH aufweist, mit einem Acylierungsmittel acyliert wird;
(L) für eine Verbindung der Formel I, worin P den Wert $S(O)_nR^5$ aufweist und n für 0 steht, eine Verbindung der Formel XII

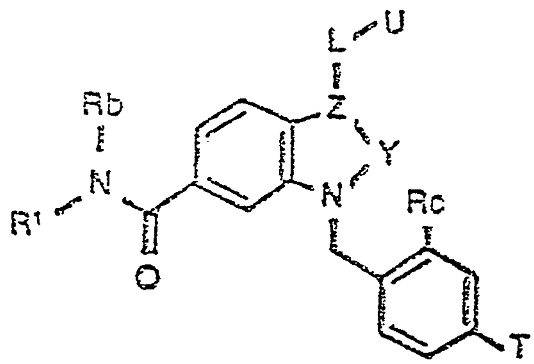

worin T aus den für M definierten Werten ausgewählt ist, mit einem Mercaptan der Formel $R^5SH$ umgesetzt wird;

(M) für eine Verbindung der Formel I, worin P den Wert $S(O)_nR^5$ hat und n für 1 oder 2 steht, die entsprechende Verbindung I, worin n für 0 oder 1 steht, oxidiert wird; und

(N) für eine Verbindung der Formel I, worin L eine Doppelbindung in Nachbarschaft zu Z enthält, ein Aldehyd oder Keton der Formel XIII

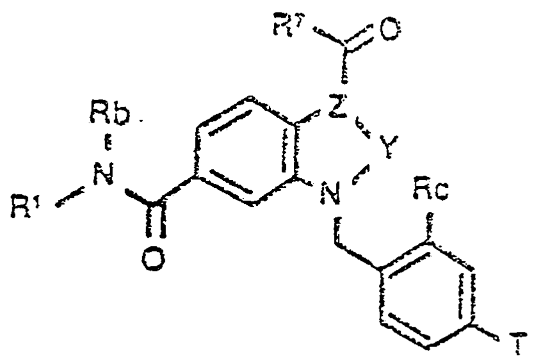

worin T aus den für M definierten Werten ausgewählt ist und $R^7$ aus Wasserstoff und (1-4C)Alkyl ausgewählt ist, mit einem zwitterionischen oder carbanionischen Reagenz umgesetzt wird;

wobei -Y-Z< , Ra bis Rc, $R^1$ bis $R^6$, L, M, P und n die oben angegebenen Bedeutungen besitzen; Rh ausgewählt ist aus Phenyl, Benzyl und (1-6C)Alkyl, das gegebenenfalls einen Acetoxy-, (1-4C)-Alkoxy- oder (1-4C)Alkylthio-Substituenten trägt; U für Chloro, Bromo, Jodo, Methansulfonyloxy oder p-Toluolsulfonyloxy steht; und Ha1 für Chloro, Bromo oder Jodo steht;

worauf, wenn ein pharmazeutisch zulässiges Salz gewünscht wird, eine saure Form der Verbindung der Formel I mit einer Base umgesetzt wird, die ein physiologisch zulässiges Kation liefert.

**Patentansprüche für folgenden Vertragsstaat : GR**

**1.** Verfahren zur Herstellung einer Verbindung der Formel I

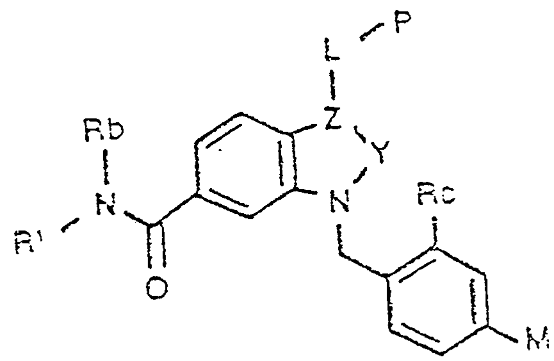

worin
die Gruppe -Y-Z< ausgewählt ist aus

(a) -C(Ra) = C<
(b) -N = C< und
(c) -CH(Ra)-CH<

wobei "<" zwei gesonderte Bindungen bezeichnet;
und worin

Ra für Wasserstoff oder (1-4C)Alkyl steht;

Rb für Wasserstoff steht;

$R^1$ für (2-10C)Alkyl steht, das gegebenenfalls einen oder mehrere Fluoro-Substituenten trägt; oder $R^1$ für Phenyl-(1-6C)alkyl steht, wobei der (1-6C)Alkyl-Teil gegebenenfalls einen Fluoro- oder (1-4C)-Alkoxy-Substituenten trägt und wobei der Phenyl-Teil gegebenenfalls einen aus Halogeno, (1-4C)Alkyl, (1-4C)Alkoxy und Trifluoromethyl ausgewählten Substituenten trägt; oder $R^1$ für (3-8C)Cycloalkyl oder (3-8C)Cycloalkyl-(1-6C)alkyl steht, wobei der cyclische Teil der beiden gegebenenfalls eine ungesättigte Bindung enthält und gegebenenfalls einen oder zwei (1-4C)Alkyl-Substituenten trägt;

L für eine (1-10)Alkylen-Gruppierung steht, die gegebenenfalls eine Doppel- oder Dreifachbindung enthält;

P für eine polare Gruppe steht, die ausgewählt ist aus Cyano, 1H-Tetrazol-5-yl, Carbamoyl der Formel $CONR^2R^3$, Ureido der Formel $NR^4CONR^2R^3$, Carbamoyloxy der Formel $OCONR^2R^3$, einem Carbamat der Formel $NR^4COOR^5$ und einer (gegebenenfalls oxidierten) Thio-Gruppe der Formel $S(O)_nR^5$, worin

$R^2$ ausgewählt ist aus Wasserstoff, (1-6C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt und $R^3$ und $R^4$ unabhängig ausgewählt sind aus Wasserstoff und (1-6C)Alkyl;

oder $R^2$ und $R^3$ gemeinsam mit dem benachbarten Stickstoff einen Pyrrolidin, Piperidin, Morpholin, Piperazin oder N-(1-6C)Alkylpiperazin-Ring bilden und $R^4$ für Wasserstoff oder (1-6C)Alkyl steht;

$R^5$ ausgewählt ist aus (1-4C)Alkyl und Phenyl, wobei der Phenyl-Teil gegebenenfalls einen oder zwei aus Halogeno, (1-4C)Alkyl, (1-4C)Alkoxy und Trifluoromethyl ausgewählte Substituenten trägt; und

n für die Ganzzahl 0, 1 oder 2 steht;

Rc ausgewählt ist aus Wasserstoff und (1-4C)Alkoxy; und

M für eine saure Gruppe steht, die ausgewählt ist aus Carboxy, einem Acylsulfonamid-Rest der Formel $-CO.NH.SO_2R^6$ und 1H-Tetrazol-5-yl, wobei

$R^6$ ausgewählt ist aus (1-6C)Alkyl, (3-8C)Cycloalkyl, (6-12C)Aryl, einem der Heteroaryle Furyl, Thienyl und Pyridyl, und (6-12C)Aryl-(1-4C)alkyl, wobei die aromatischen und heteroaromatischen Teile gegebenenfalls einen oder zwei aus Halogeno, (1-4C)Alkyl, (1-4C)Alkoxy und Trifluoromethyl ausgewählte Substituenten tragen;

oder eines pharmazeutisch zulässigen Salzes davon, bei welchem

(A) für eine Verbindung der Formel I, worin M für eine Carboxy-Gruppe steht, ein Ester der Formel III

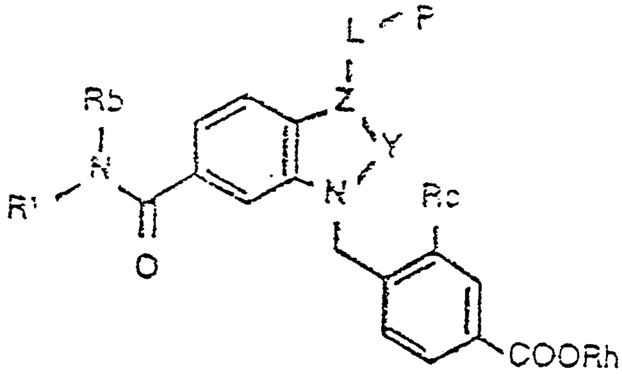

zersetzt wird;

(B) ein Amin der Formel $R^1NHRb$ mit einer Carbonsäure der Formel IV

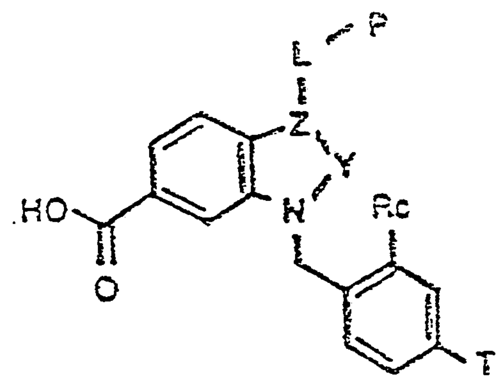

worin T aus den für M definierten Werten ausgewählt ist, oder mit einem reaktiven Derivat davon acyliert wird;

(C) für eine Verbindung der Formel I, worin -Y-Z< den oben definierten Wert (a) besitzt, ein Indol der Formel V

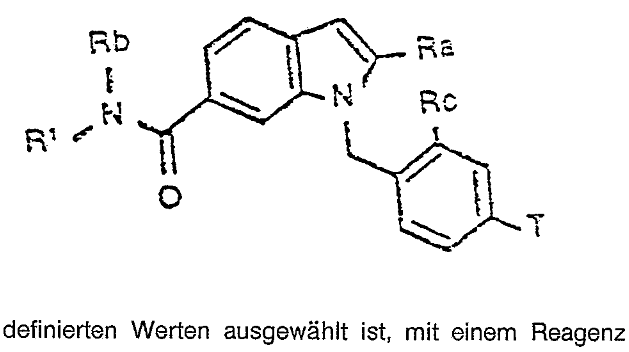

worin T aus den für M definierten Werten ausgewählt ist, mit einem Reagenz der Formel U.L.P umgesetzt wird;

(D) eine Imino-Verbindung der Formel VI

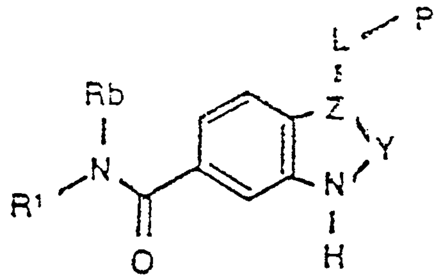

mit einem Alkylierungsmittel der Formel VII

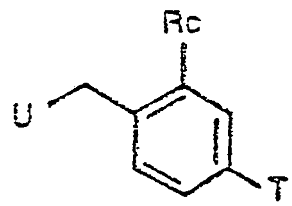

worin T aus den für M definierten Werten ausgewählt ist, umgesetzt wird;

(E) für eine Verbindung der Formel I, worin M für ein 1H-Tetrazol-5-yl-Radikal steht, ein Cyano-Derivat der Formel VIII

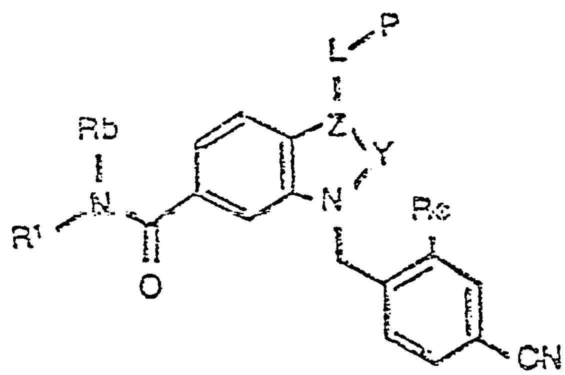

mit einem Azid umgesetzt wird;

(F) für eine Verbindung der Formel I, worin M für eine Gruppe der Formel $CO.NH.SO_2.R^6$ steht, eine Verbindung der Formel I, worin M für Carboxy steht, mit einem Sulfonamid-Derivat der Formel $R^6.SO_2.NH_2$ in Gegenwart eines Dehydratisierungsmittels umgesetzt wird oder ein reaktives Derivat einer Säure der Formel I mit einem Sulfonamid der Formel $R^6.SO_2.NH_2$ oder einem Salz davon umgesetzt wird;

(G) für eine Verbindung der Formel I, worin -Y-Z< den oben definierten Wert (c) aufweist, ein Indol der Formel I, worin -Y-Z< den oben definierten Wert (a) aufweist, katalytisch hydriert wird;

(H) die Doppelbindung einer Verbindung der Formel I, worin L eine Doppelbindung enthält, reduziert wird, um die entsprechende Verbindung der Formel I herzustellen, in der L keine Doppelbindung enthält;

(I) für eine Verbindung der Formel I, worin P für eine Carbamoyl-Gruppe der Formel $CONR^2R^3$ steht, ein Amin der Formel $HNR^2R^3$ mit einer entsprechenden Säure der Formel IX

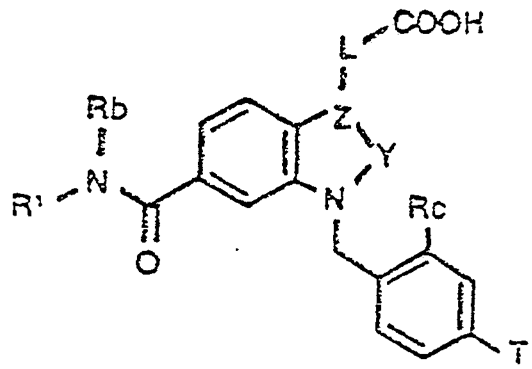

worin T aus den für M definierten Werten ausgewählt ist, oder einem reaktiven Derivat davon acyliert wird;

(J) für eine Verbindung der Formel I, worin P für eine 1H-Tetrazol-5-yl-Gruppe steht, ein Nitril der Formel X

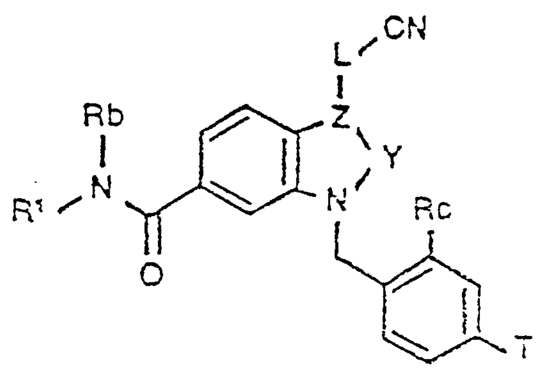

worin T aus den für M definierten Werten ausgewählt ist, mit einem Azid umgesetzt wird, wobei ähnliche Verfahrensweisen verwendet werden, wie sie oben in Teil (E) beschrieben sind;

(K) für eine Verbindung der Formel I, worin P den Wert $NR^4CONR^2R^3$, $OCONR^2R^3$ oder $NR^4COOR^5$ hat, eine Verbindung der Formel XI

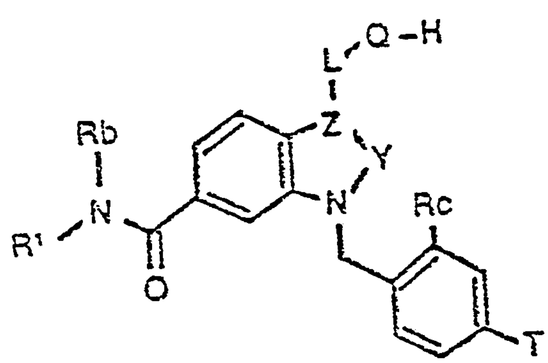

worin T aus den für M definierten Werten ausgewählt ist und worin QH den Wert $NR^4H$ oder OH aufweist, mit einem Acylierungsmittel acyliert wird;

(L) für eine Verbindung der Formel I, worin P den Wert $S(O)_nR^5$ aufweist und n für 0 steht, eine Verbindung der Formel XII

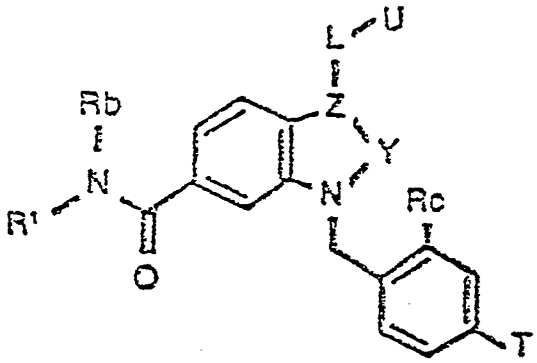

worin T aus den für M definierten Werten ausgewählt ist, mit einem Mercaptan der Formel $R^5SH$ umgesetzt wird;

(M) für eine Verbindung der Formel I, worin P den Wert $S(O)_nR^5$ hat und n für 1 oder 2 steht, die entsprechende Verbindung I, worin n für 0 oder 1 steht, oxidiert wird; und

(N) für eine Verbindung der Formel I, worin L eine Doppelbindung in Nachbarschaft zu Z enthält, ein Aldehyd oder Keton der Formel XIII

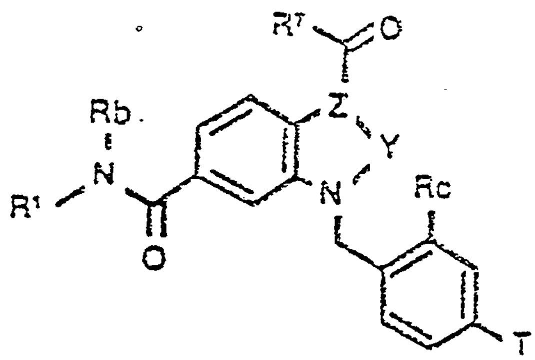

worin T aus den für M definierten Werten ausgewählt ist und $R^7$ aus Wasserstoff und (1-4C)Alkyl ausgewählt ist, mit einem zwitterionischen oder carbanionischen Reagenz umgesetzt wird;

wobei -Y-Z< , Ra bis Rc, $R^1$ bis $R^6$, L, M, P und n die oben angegebenen Bedeutungen besitzen; Rh ausgewählt ist aus Phenyl, Benzyl und (1-6C)Alkyl, das gegebenenfalls einen Acetoxy-, (1-4C)-Alkoxy- oder (1-4C)Alkylthio-Substituenten trägt; U für Chloro, Bromo, Jodo, Methansulfonyloxy oder p-Toluolsulfonyloxy steht; und Ha1 für Chloro, Bromo oder Jodo steht;

worauf, wenn ein pharmazeutisch zulässiges Salz gewünscht wird, eine saure Form der Verbindung der Formel I mit einer Base umgesetzt wird, die ein physiologisch zulässiges Kation liefert.

**2.** Verbindung der Formel III

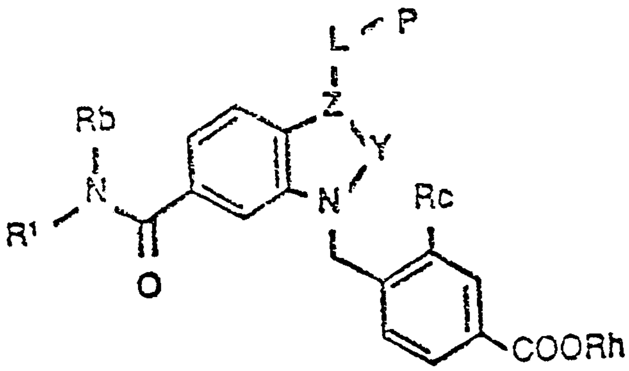

worin Rh eine der in Anspruch 1 angegebenen Bedeutungen besitzt und $R^1$, Rb, Rc, L, P und die Gruppe -Y-Z< eine der in Anspruch 1 angegebenen Bedeutungen besitzen.